(19) Europäisches Patentamt
European Patent Office
Office européen des brevets

(11) Veröffentlichungsnummer : **0 076 996**
**B1**

(12) # EUROPÄISCHE PATENTSCHRIFT

(45) Veröffentlichungstag der Patentschrift : **02.01.86**

(21) Anmeldenummer : **82109134.5**

(22) Anmeldetag : **04.10.82**

(51) Int. Cl.$^4$ : **C 07 D295/14**, C 07 D295/18, C 07 D295/22, A 61 K 31/495

(54) **Neue Carbonsäurederivate, Verfahren zu ihrer Herstellung sowie diese Verbindungen enthaltende Arzneimittel.**

(30) Priorität : **08.10.81 DE 3139970**

(43) Veröffentlichungstag der Anmeldung : **20.04.83 Patentblatt 83/16**

(45) Bekanntmachung des Hinweises auf die Patenterteilung : **02.01.86 Patentblatt 86/01**

(84) Benannte Vertragsstaaten : **AT BE CH DE FR GB IT LI LU NL SE**

(56) Entgegenhaltungen :
**EP-A- 0 023 569**
**GB-A- 1 299 172**
**US-A- 3 641 040**

(73) Patentinhaber : **Boehringer Mannheim GmbH**
**Sandhofer Strasse 116**
**D-6800 Mannheim 31 (DE)**

(72) Erfinder : **Witte, Ernst-Christian, Dr.rer.nat.**
**Beethovenstrasse 2**
**D-6800 Mannheim 1 (DE)**
Erfinder : **Wolff, Hans Peter, Dr.rer.nat.**
**Rebenweg 24**
**D-6945 Hirschberg-Grosssachsen (DE)**
Erfinder : **Hagenbruch, Bernd, Dr.rer.nat.**
**In den Gärten 5**
**D-6840 Lampertheim (DE)**
Erfinder : **Stegmeier, Karlheinz, Dr.rer.nat.**
**Kirchbergstrasse 17**
**D-6148 Heppenheim (DE)**
Erfinder : **Pill, Johannes, Dr.rer.nar., Dr.med.**
**In der Keitgasse 5**
**D-6906 Leimen 3 (DE)**

**EP 0 076 996 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Anmeldung sind neue Carbonsäurederivate der allgemeinen Formel I

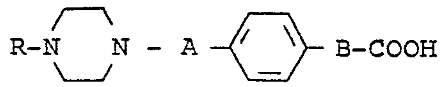

(I)

in der

A eine Valenzbindung oder eine geradkettige oder verzweigte Alkylenkette mit 1 bis 3 C-Atomen,

B eine Valenzbindung oder eine gesättigte oder ungesättigte, geradkettige oder verzweigte Alkylenkette mit 1 bis 3 C-Atomen und

R ein Wasserstoffatom, eine geradkettige oder verzweigte $C_1$-$C_{16}$-Alkylkette, die ein- oder mehrfach durch Hydroxyl, Carboxyl, eine Sulfonsäuregruppe oder eine gegebenenfalls ein- oder mehrfach durch $C_1$-$C_6$-Alkyl, $C_1$-$C_6$-Alkoxy, Halogen, Nitro, Cyano, Carboxyl oder $C_1$-$C_6$-Acylamino substituierte Phenoxygruppe substituiert ist, eine Phenylalkylkette, wobei die Phenylgruppe gegebenenfalls eine oder mehrere Halogenatome, Hydroxyl, $C_1$-$C_6$-Alkoxy-, Cyano-, Carboxyl-, Nitro-, $C_1$-$C_6$-Acylamino-, $C_1$-$C_6$-Alkyl oder Trifluormethyl-Gruppen tragen kann und die Alkylkette gegebenenfalls ungesättigt ist und bis zu 4 C-Atome aufweist, eine Phenacylgruppe, deren Phenylrest ein- oder mehrfach durch Halogen, Hydroxy oder $C_1$-$C_6$-Alkyl substituiert sein kann und deren Acyl-Teil 2 bis 4 C-Atome enthält, einen 2-Methyl-3-phenylpropionyl-, 3-Phenyl-propionyl-, Cinnamoyl-, Phenacetyl- oder Benzoyl-Rest, wobei die Phenylreste durch Halogen, Hydroxyl oder $C_1$-$C_6$-Alkyl substituiert sein können, einen Methansulfonyl-, Benzolsulfonyl- oder Phenacylsulfonyl-Rest, wobei der Phenylrest durch Halogen oder $C_1$-$C_6$-Alkyl substituiert sein kann, oder einen 4-Chlorphenyl-, 3-Trifluormethylphenyl-, einen 4-(4-Chlorbenzoyl)-phenyl-, einen 4-Biphenylyl-, einen 4-Phenoxyphenyl- oder 4-Benzyloxyphenyl-Rest

bedeuten, mit der Maßgabe, daß wenn A eine Valenzbindung ist, R nicht Wasserstoff, Methyl, Ethyl, Hydroxyethyl oder Benzyl sein darf, sowie deren physiologisch unbedenkliche Salze, deren Ester mit Methanol, Ethanol, n-Butanol, 4-Chlor-benzylalkohol oder 2-Diethylamino-ethanol und deren Amide mit Ammoniak, Diethylamin, Ethanolamin, p-Amino-benzoesäure, β-Alanin oder Morpholin.

In der EP-A-0 023 569 sind 2-Aminobenzoesäurederivate als Zwischenprodukte zur Herstellung von Carbonsäurederivaten mit blutzuckersenkender Wirkung beschrieben.

In der österreichischen Patentschrift 330 157 [C.A. *85*, 1976, 192 404 j] sind 3-N-Piperazinyl-alkylbenzoesäuren beschrieben, die als Muskelrelaxantien und zur Steigerung der Gedächtnisleistung verwendet werden können.

Ähnliche Substanzen, jedoch als Spasmolytica, sind im britischen Patent 1 382 606 [C.A. *83*, 1975, 27 946 p] und im südafrikanischen Patent 72, 06.378 [C.A. *80*, 1974, 82 431 e] beschrieben.

In dem US-Patent No. 3 641 040 sind Piperazinyl-phenyl-essigsäuren beschrieben, deren Piperazin-Substituent durch Methyl, Ethyl, 2-Hydroxyethyl, Benzyl oder Phenyl substituiert sein kann. Diese Verbindungen besitzen jedoch eine entzündungshemmende Wirkung.

Die neuen Verbindungen der allgemeinen Formel I und ihre physiologisch unbedenklichen Salze sowie ihre Ester und Amide zeigen sowohl eine ausgezeichnete lipidsenkende Wirkung als auch eine ausgeprägte Hemmwirkung auf die Thrombozytenaggregation.

Als Alkylgruppen des Substituenten R sind bevorzugt der Methyl-, der Ethyl- und der Hexadecylrest. Als substituierte Alkylgruppen sind bevorzugt: 2-Hydroxypropyl, 1-Carboxyethyl und der 3-Propyl-1-sulfonsäurerest. Bei den durch eine Phenoxygruppe substituierten Alkylgruppen handelt es sich bevorzugt um die 2-Phenoxyethylgruppe sowie um die 2-Phenoxypropylgruppe, welche gegebenenfalls substituiert sein kann durch $C_1$-$C_6$-Alkyl, insbesondere Methyl, $C_1$-$C_6$-Alkoxy, insbesondere Methoxy, Halogen, wobei hier und in allen anderen Fällen unter « Halogen » Fluor, Chlor, Brom und Jod zu verstehen sind, die Nitrogruppe, die Cyanogruppe sowie die Carboxygruppe.

Wenn R die Bedeutung Phenylalkylkette hat, sind die Gruppen Benzyl, Phenethyl, 3-Phenylpropyl oder 2-Benzylpropyl bevorzugt. Bevorzugte Substituenten des Phenylrestes sind Halogen, $C_1$-$C_6$-Alkoxy, insbesondere Methoxy oder $C_1$-$C_6$-Alkyl, insbesondere tert.-Butyl sowie Trifluormethyl und Hydroxy.

Wenn R die Bedeutung Phenylalkenyl hat, sollen bevorzugt der Cinnamylrest sowie der α- und der β-Methylcinnamylrest verstanden werden. Als Substituenten des Phenylringes sind insbesondere Methoxy und Acetylamino zu nennen.

Insbesondere sind unter Phenacyl die Reste 4-Chlorbenzoylmethyl- und 1-(4-Hydroxy-3.5-di-tert.-butylbenzoyl) ethyl zu verstehen.

In der Formel I bedeutet A entweder eine Bindung oder aber eine der Gruppen

$-CH_2-$, $-(CH_2)_2-$ und $-(CH_2)_3-$ (unverzweigt) sowie

$-\underset{\displaystyle CH_3}{\underset{|}{C}H}-CH_2-$ und $-CH_2\underset{\displaystyle CH_3}{\underset{|}{C}H}-$ (verzweigt)

B bedeutet entweder eine Bindung oder aber eine der Gruppen

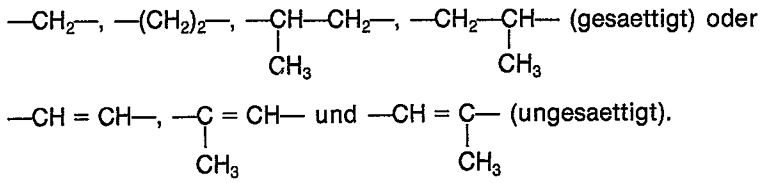
(gesaettigt) oder

und

(ungesaettigt).

Die obige Definition der erfindungsgemaeßen Verbindungen soll auch alle moeglichen Stereoisomeren sowie ihre Mischungen umfassen.

Gegenstand der vorliegenden Anmeldung sind weiterhin Verfahren zur Herstellung von Carbonsäuren der allgemeinen Formel I, indem man in an sich bekannter Weise

a) eine Verbindung der Formel II

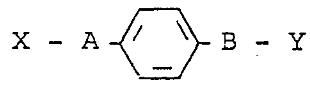
(II)

entweder mit einem $N_1$-geschützten Piperazin umsetzt und nach Abspaltung der Schutzgruppe den Substituenten R durch Alkylierung oder Acylierung einführt, oder mit einem bereits durch R monosubstituierten Piperazin umsetzt oder

b) eine Verbindung der Formel III

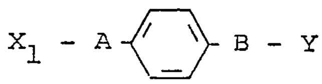
(III)

mit einer Verbindung der Formel IV

$$R—X_2 \quad \text{(IV)}$$

umsetzt, wobei einer der Reste $X_1$ oder $X_2$ eine $NH_2$-Gruppe bedeutet und der andere eine

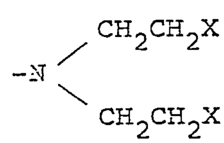

Gruppe darstellt, während in beiden obigen Verfahren
A und R die angegebene Bedeutung haben,
X einen reaktiven Rest,
B eine Valenzbindung, eine gesättigte oder ungesättigte niedere Alkylenkette oder einen in diese Gruppe überführbaren Rest und
Y eine Säureamid- oder $—COOR_2$-Gruppe, in der $R_2$ Wasserstoff oder eine niedere Alkylgruppe bedeutet oder einen in diese Gruppen überführbaren Rest darstellt,
bedeuten, anschließend gegebenenfalls die erhaltenen Verbindungen in die freie Carbonsäure, ihre Salze, Ester oder Amide überführt, gegebenenfalls Gruppen B in gesättigte oder ungesättigte niedere Alkylketten umwandelt oder den Rest R gegen einen anderen Rest R austauscht.

Das Verfahren a) wird bevorzugt zur Herstellung von Verbindungen der Formel I angewendet, in denen A eine Alkylenkette bedeutet, während Verfahren b) in der Regel zur Herstellung von Verbindungen mit A = Valenzbindung sowie einem Substituenten R, der einen gegebenenfalls substituierten Phenylrest darstellt, Verwendung findet.

Die nachträgliche Überführung der Gruppe -B-Y der Verfahrensprodukte gemäß Verfahren a) und b) kann auf verschiedenen Wegen durchgeführt werden. So kann z. B. eine Verbindung der allgemeinen Formel XI

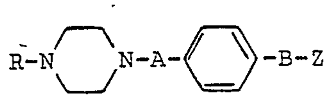
(XI)

in der R, A und B die angegebene Bedeutung haben und Z einen oxidativ in die Carboxylfunktion überführbaren Rest darstellt, oxidiert werden.

Weitere Verfahren bestehen darin, daß man eine Verbindung der allgemeinen Formel XII

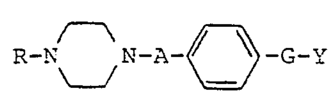
(XII)

in welcher G eine Kohlenstoff-Kette darstellt, die eine der Gruppen

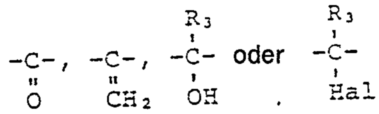

oder

oder gegebenenfalls funktionelle Derivate dieser Gruppen enthaelt, reduziert, oder, fuer den Fall, daß B eine unverzweigte Kette darstellt, eine Verbindung der allgemeinen Formel XIII

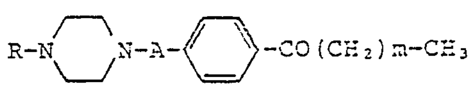 (XIII)

zu welcher m die Zahlen 0-2 darstellt, unter den Bedingungen einer gegebenenfalls modifizierten Willgerodt-Kindler-Reaktion umsetzt.

Zur Herstellung von Verbindungen der allgemeinen Formel XIV

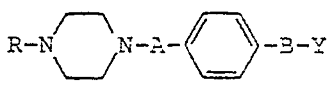 (XIV)

in welcher B die spezielle Bedeutung

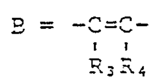 hat,

wobei $R_3$ und $R_4$ gleich oder verschieden und jeweils Wasserstoff oder eine niedere Alkylgruppe bedeuten, kommen alle gängigen Verfahren zur Darstellung von Zimtsäuren und ihren Derivate infrage :

1) Beim Vorliegen geeigneter Verbindungen XIV in denen B die Bedeutung

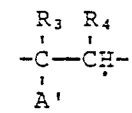 oder 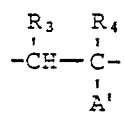 hat,

wobei A' ein Halogenatom, eine Hydroxygruppe oder eine funktionell abgewandelte Hydroxygruppe darstellt, gelangt man zu den gewuenschten Verbindungen XIV, in denen B die Gruppe

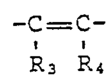

darstellt, durch Einwirkung eines HA abspaltenden Mittels.

2) Beim Vorliegen geeigneter Verbindungen XV

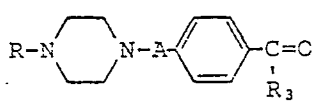 (XV)

kann man die gewuenschten Zimtsaeure-Derivate durch Reaktionen im Sinne einer Aldolkondensation, d. h. durch Umsetzen mit aktivierten CH-Gruppen, darstellen. Als solche Verbindungen kommen infrage Essigsaeure bzw. deren Derivate, insbesondere aber Malonsaeure-Derivate der allgemeinen Formel XVI

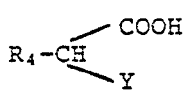 (XVI)

wobei in letzterem Falle nach erfolgter Kondensation eine Decarboxylierung eintritt.

Als weitere Moeglichkeit solcher aldolartiger Umsetzungen sei die Perkin-Reaktion genannt, die in einer Umsetzung von Verbindungen der allgemeinen Formel XV mit dem Anhydrid einer aliphatischen Carbonsaeure in Gegenwart eines Alkalisalzes ggf. der gleichen Carbonsaeure besteht.

3) Eine dritte Moeglichkeit besteht in der Umsetzung von Verbindungen der allgemeinen Formel XV mit geeigneten phosphororganischen Verbindungen im Sinne modifizierter Wittig-Reaktionen. Als solche phosphororganischen Reaktionspartner seien z. B. die Alkoxycarbonylmethylphosphonsaeure-alkylester der allgemeinen Formel XVII genannt :

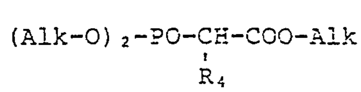 (XVII)

Durch Hydrieren der nach den Methoden 1 bis 3 oder nach anderen Methoden erhaltenen Zimtsaeure-Derivate lassen sich die analogen Verbindungen mit gesaettigter Kohlenstoff-Kette herstellen.

Es ist offensichtlich, daß sich die oben beschriebenen Methoden nicht anwenden lassen, wenn R gewisse, diese Methoden behindernde oder gegen diese Methoden empfindliche Gruppen enthaelt. In einem solchen Falle wird das Piperazin intermediaer geschuetzt. Man setzt dann die geschuetzte Verbindung (R' = Schutzgruppe)

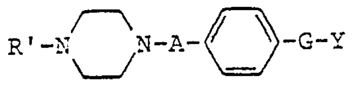 (XII') bzw.

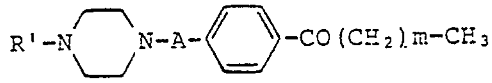 (XIII') bzw.

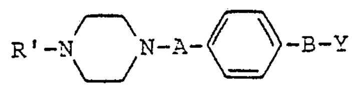 (XIV') bzw.

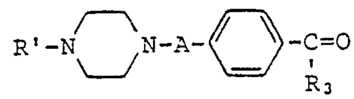 (XV')

um, entfernt durch geeignete Methoden die Schutzgruppe R' und fuehrt nun z. B. gemaeß Verfahren a) die gewuenschte Gruppe R durch Alkylierung oder Acylierung ein.

Bei Alkylierung gemäß Verfahren a) verwendet man für den Fall, daß R Alkyl, Phenylalkyl, Phenylalkenyl oder Phenacyl bedeutet, als reaktive Derivate entweder die Halogenide, insbesondere die Chloride und Bromide, oder aber geeignete Sulfonsäureester, z. B. Mesylate oder Tosylate.

Die Umsetzung der Halogenide bzw. Sulfonsäureester mit den Piperazinen erfolgen zweckmäßig unter Zusatz eines säurebindenden Mittels wie z. B. Natriumhydrogencarbonat, Natriumcarbonat, Kaliumcarbonat. Diese Funktion können aber auch organische Basen wie z. B. Pyridin oder Triethylamin übernehmen, oder man verwendet ein zweites Mol des eingesetzten Piperazins als Säureakzeptor. Als inertes Lösungsmittel dienen z. B. Ether, Benzol, Tetrahydrofuran, Dioxan, Methylenchlorid oder ein Überschuß an tertiärem Amin. Beim Einsatz anorganischer Säurebildner verwendet man als Reaktionsmedium z. B. Ethanol, Butanon-2, Dimethylformamid, Hexamethylphosphoräure-triamid oder Acetonitril.

Als reaktive Derivate der Carbonsaeuren finden bei Verfahren a) vor allem Saeurechloride Verwendung. Ebenso gut koennen aber auch deren Ester, Azide, Anhydride und gemischten Anhydride eingesetzt werden. Die Reaktion mit einem Piperazin kann im Falle aromatischer Saeurehalogenide auch nach « Schotten-Baumann » vorgenommen werden. Will man unter wasserfreien Bedingungen arbeiten, so wird vorzugsweise absolutes Pyridin angewandt. Man kann aber auch andere tert. Basen wie z. B. Dimethylanilin oder Triethylamin in einem inerten Loesungsmittel wie z. B. Methylenchlorid einsetzen. Anstelle der freien Piperazinoverbindung kann man auch deren Salze einsetzen.

Zur Acylierung der Piperazine lassen sich auch die freien Saeuren einsetzen, wenn man durch Azeotropdestillation dafuer sorgt, daß entstehendes Reaktionswasser aus dem Reaktionsgemisch entfernt wird.

Dem Reaktionsgemisch koennen gegebenenfalls katalytisch wirkende Mengen eines tertiaeren Amins (wie z. B. Triethylamin oder Dimethylanilin) und des Komplexes einer Lewis-Saeure (wie z. B. Bortrifluorid-diethylethakomplex) zugesetzt werden.

Als reaktive Derivate der Sulfonsaeuren kommen bei Verfahren a) insbesondere die Halogenide sowie die Ester infrage. Als saeurebindende Agenzien werden hier z. B. Alkaliacetat, Natriumhydrogencarbonat, Kaliumcarbonat, Natriumphosphat, Calciumoxid, Calciumcarbonat oder Magnesiumcarbonat eingesetzt, wobei man als Reaktionsmedium z. B. Wasser, waeßriges Ethanol oder waeßriges Dioxan verwendet. Muß man wegen Hydrolyseempfindlichkeit auf waeßrige Medien verzichten, so kann man auch hier tertiaere organische Amine wie Pyridin oder Triethylamin, ggf. in einem inerten Loesungsmittel wie z. B. Methylenchlorid einsetzen.

Zur Umsetzung von Verbindungen der allgemeinen Formel II mit Piperazinderivaten gemaeß Verfahren a) setzt man bevorzugt Verbindungen V ein, in denen X ein Halogenatom (z. B. Brom oder Chlor) oder eine Alkyl- oder Arylsulfonyloxygruppe, also z. B. die Mesyloxy- oder die Tosyloxygruppe darstellt.

Zur Umsetzung einer Verbindung III mit einer Verbindung IV gemaeß Verfahren b) eignen sich besonders solche Verbindungen IV, in denen R ein gegebenenfalls substituierter Arylrest ist und X entweder Chlor oder Brom oder einen mit einer Sulfonsaeure veresterten Alkoholrest darstellt, X also z. B. Mesyloxy oder Tosyloxy bedeutet. Die Reaktion wird bevorzugt in Alkoholen wie z. B. n-Butanol in Gegenwart eines saeurebindenden Mittels wie z. B. wasserfreiem Kaliumcarbonat durchgefuehrt. Es koennen aber auch andere Loesungsmittel wie z. B. Dioxan, Dimethylformamid u.a. eingesetzt werden.

Als oxidierbare Gruppen zur nachträglichen Überführung in eine Carboxylfunktion kommen vorzugsweise die Hydroxymethyl-, die Aminomethyl- und die Formylgruppen, gegebenenfalls auch die Acetylgruppe oder funktionelle Derivate dieser Gruppen infrage. Die Oxidation läßt sich mit den üblichen Oxidationsmitteln wie z. B. Mangan-IV-verbindungen, Permanganaten, Dichromaten, im Falle der Formylgruppe auch mit Luftsauerstoff oder Silberoxid, im Falle der Acetylgruppe dagegen mit Hypobromit durchführen.

Zur Reduktion der in der allgemeinen Formel XII enthaltenen Gruppen G eignen sich zahlreiche Verfahren. Die Reduktion der Gruppe —CO— kann z. B. nach Clemmensen mittels Zink/Salzsaeure erfolgen. Bevorzugt ist jedoch die Reduktion mit Wasserstoff bei Normaldruck oder bei erhoehtem Druck in Gegenwart von Metallkatalysatoren wie z. B. Palladium oder Platin in Loesungsmitteln wie z. B. Essigsaeure oder niederen Alkoholen.

Auch die Gruppen

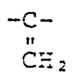 , 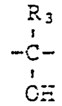 und 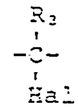

werden bevorzugt durch katalytisch angeregten Wasserstoff reduziert, wobei sich die eine Hydroxy-Gruppe enthaltende Gruppe G am besten in Gegenwart starker Saeuren reduzieren laesst. Bevorzugt ist hierbei die Anwesenheit von Schwefelsaeure oder Perchlorsaeure in katalytischen Mengen. Gegebenenfalls kann auch mit komplexen Metallhydriden reduziert werden. Bevorzugt wird Natriumborhydrid eingesetzt. In diesem Fall kann die Umsetzung in einem Alkohol, insbesondere in Methanol, oder in Dioxan oder in waessrig-alkalischem Milieu durchgefuehrt werden.

Die eingesetzten Ketone der allgemeinen Formel XIII lassen sich leicht durch Friedel-Crafts-Acylierung darstellen. Sie werden mit Schwefel und einem sekundaeren Amin, bevorzugt mit Morpholin, umgesetzt. Das bei dieser « Willgerodt-Kindler-Reaktion » entstehende Thiomorpholid wird in an sich bekannter Weise mit Hilfe von starker Alkalilauge oder mit starker Salzsaeure oder auch mit einem Gemisch aus Schwefelsaeure, Eisessig und Wasser zur Carbonsaeure verseift.

Zur Herstellung von Zimtsaeure-Derivaten gemaeß Verfahren 1 eignen sich alle Verfahren, die eine Abspaltung von HA gestatten. Stellt A eine Hydroxygruppe dar, so kann eine Dehydratisierung mit den ueblichen Agenzien wie z. B. Eisessig, Acetanhydrid, Schwefelsaeure, Hydrogensulfat, Polyphosphorsaeure, Phosphoroxichlorid, Thionylchlorid oder Phosphorpentoxid erfolgen, wobei zweckmaessigerweise in inerten Loesungsmitteln wie Benzol, Methylenchlorid, Tetrachlorkohlenstoff u. a. gearbeitet wird. Bevorzugt ist eine Dehydratisierung mit Phosphorpentoxid in siedendem Methylenchlorid. Die fuer die Dehydratisierung notwendigen Hydroxyverbindungen lassen sich z. B. durch eine Reformatzky-Reaktion aus den entsprechenden Aldehyden oder Ketonen darstellen, oder man gewinnt sie durch Reduktion der entsprechenden Ketoverbindungen entweder mit komplexen Hydriden wie z. B. Natriumborhydrid oder durch Hydrierung mit Raney-Nickel als Katalysator.

Zur Abspaltung von Halogenwasserstoff (wenn A ein Halogenatom bedeutet) kommen basische Agenzien infrage. Geeignete basische Agenzien sind z. B. anorganische oder organische Basen, wie NaOH, KOH, Natriumacetat, $Na_2CO_3$, $K_2CO_3$ sowie Alkoholate wie Natriummethylat oder Amine wie z. B. Triethylamin, Dimethylanilin und Pyridin. Man arbeitet zweckmaessig in inerten Loesungsmitteln wie Dioxan, DMSO, Benzol, Petrolether oder in Alkoholen wie Ethanol oder Isopropanol.

Die Kondensation von Verbindungen der allgemeinen Formel XV mit Malonsaeure-Derivaten erfolgt in bekannter Weise durch Reaktion der beiden Partner in geeigneten Loesungsmitteln wie z. B. Pyridin, bevorzugt in Gegenwart eines primaeren oder sekundaeren Amins, wobei als sekundaeres Amin Piperidin bevorzugt wird.

Die Umsetzungen zwischen Verbindungen XV mit Phosphonsaeureestern (PO-aktivierte Olefinbildung nach Horner) wird in inerten Loesungsmitteln in Gegenwart von Basen vorgenommen. Als inerte Loesungsmittel eignen sich z. B. Diglyme, Benzol, Toluol, THF, DMF, aber auch Ether und Petrolether. Geeignete Basen sind z. B. Natriumamid, Organolithiumverbindungen, Alkoholate (meist im entsprechenden Alkohol geloest), Natriumhydrid sowie Dimethylsulfoxylat in DMSO. Die Umsetzungen werden entweder bei Raumtemperatur oder bei erhoehten Temperaturen (Siedetemperatur des Loesungsmittels) durchgefuehrt.

Als intermediaer einzufuehrende Schutzgruppen fuer das Piperazin kommen im Prinzip alle moeglichen Schutzgruppen fuer sek. Amine infrage. Bevorzugt sind Acylgruppen, insbesondere Formyl, Acetyl und Benzoyl. Bei einigen Umsetzungen gemäß obigen Verfahren haben sich Alkoxycarbonylgruppen, insbesondere die Ethoxycarbonylgruppe, bewaehrt. Sofern keine hydrogenolytische Prozesse im Herstellungsverfahren eingeschlossen sind, ist als bevorzugte Schutzgruppe auch der Benzylrest anzufuehren.

Als Substituenten Y der allgemeinen Formel II, die in die —$COOR_2$-Gruppe ueberfuehrt werden koennen, kommen beispielsweise die Nitril-, Carbaldehyd-, Hydroxymethyl-, Aminomethyl- und Formylgruppe infrage.

Die gegebenenfalls im Anschluß an die Kondensation gemaeß Verfahren a) und b) bzw. im Anschluß an die Kettenumwandlung durchzufuehrende Umwandlung des Substituenten $R_2$ erfolgt beispielsweise

durch Verseifung der Carbonsaeureester ($R_2$ = Alkyl) zu den entsprechenden Carbonsaeuren ($R_2$ = Wasserstoff) mit Mineralsaeuren oder Alkalihydroxiden in einem polaren Loesungsmittel (wie Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei maessig erhoehten Temperaturen durchgefuehrt. Umgekehrt kann man aber auch die Carbonsaeuren in ueblicher Weise verestern oder Ester mit einem bestimmten Rest $R_2$ durch Umestern in einen Ester mit einem anderen Rest $R_2$ umwandeln. Die Veresterung der Carbonsaeuren wird zweckmaessig in Gegenwart eines sauren Katalysators, wie z. B. Chlorwasserstoff, Schwefelsaeure, p-Toluolsulfonsaeure oder eines stark sauren Ionenaustauschharzes, vorgenommen. Umesterungen hingegen erfordern den Zusatz einer geringen Menge einer basischen Substanz, z. B. eines Alkali- oder Erdalkalihydroxids oder eines Alkalialkoholats.

Die erfindungsgemaessen, von den Carbonsaeuren der allgemeinen Formel I abgeleiteten Amide werden bevorzugt nach an sich bekannten Methoden aus den Carbonsaeuren oder ihren reaktiven Derivaten (wie z. B. Carbonsaeurehalogeniden, -estern, -aziden, -anhydriden oder gemischten Anhydriden) durch Umsetzung mit Aminen hergestellt.

Zur Herstellung von Salzen mit pharmakologisch vertraeglichen organischen oder anorganischen Basen, wie z. B. Natriumhydroxid, Kaliumhydroxid, Calciumhydroxid, Ammoniumhydroxid, Methylglukamin, Morpholin oder Ethanolamin koennen die Carbonsaeuren mit den entsprechenden Basen umgesetzt werden. Auch Mischungen der Carbonsaeuren mit einem geeigneten Alkalicarbonat bzw. -hydrogencarbonat kommen in Betracht.

Sämtliche Verbindungen der Formel I, in denen R ein Wasserstoff bedeutet, können auch als Zwischenprodukte zur Herstellung von Verbindungen der Formel I mit angegebenem R betrachtet werden. Die Weiterverarbeitung zu den erfindungsgemäßen Verbindungen erfolgt gemäß Verfahren a) durch Acylierung oder Alkylierung.

Ferner sind sämtliche Verbindungen der allgemeinen Formel XI-XVI neu. Ihre Weiterverarbeitung zu den erfindungsgemäßen Verbindungen der Formel I geschieht in der oben aufgeführten Weise.

Zur Herstellung von Arzneimitteln werden die Verbindungen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z. B. Olivenoel, suspendiert oder geloest.

Die Substanzen der allgemeinen Formel I koennen in fluessiger oder fester Form oral und parenteral appliziert werden. Als Injektionsmedium kommt vorzugsweise Wasser zur Anwendung, welches die bei Injektionsloesungen ueblichen Stabilisierungsmittel, Loesungsvermittler und/oder Puffer enthaelt. Derartige Zusaetze sind z. B. Tartrat- oder Borat-Puffer, Ethanol, Dimethylsulfoxid, Komplexbildner (wie Ethylendiamintetraessigsaeure), hochmolekulare Polymere (wie fluessiges Polyethylenoxid) zur Viskositaetsregulierung oder Polyethylen-Derivate von Sorbitanhydriden.

Feste Traegerstoffe sind z. B. Staerke, Lactose, Mannit, Methylcellulose, Talkum, hochdisperse Kieselsaeure, hoehermolekulare Fettsaeuren (wie Stearinsaeure), Gelatine, Agar-Agar, Calciumphosphat, Magnesiumstearat, tierische und pflanzliche Fette oder feste hochmolekulare Polymere (wie Polyethylenglykole). Fuer die orale Applikation geeignete Zubereitungen koennen gewuenschtenfalls Geschmacks- und Sueßstoffe enthalten.

Die verabreichte Dosierung haengt vom Alter, der Gesundheit und dem Gewicht des Empfaengers, dem Ausmaß der Krankheit, der Art gleichzeitiger gegebenenfalls durchgefuehrter weiterer Behandlungen, der Hauefigkeit der Behandlungen und der Art der gewuenschten Wirkung ab. Ueblicherweise betraegt die taegliche Dosis der aktiven Verbindung 0,5 bis 500 mg/kg Koerpergewicht. Normalerweise sind 1,0 bis 400 und vorzugsweise 2 bis 200 mg/kg/Tag in einer oder mehreren Anwendungen pro Tag wirksam, um die gewuenschten Resultate zu erhalten.

## Beispiel 1

4-(Piperazin-1-yl) benzoesaeure-ethylester

Man erhitzt ein Gemisch aus 39.5 g (0.24 mol) 4-Aminobenzoesaeure-ethylester (freie Base), 140 ml n-Butanol und 47.1 g (0.265 mol) Bis-(2-chlorethyl) amin. HCl 36 h auf Rueckflusstemperatur, gibt dann 16.8 g (0.12 mol) pulv. $K_2CO_3$ sicc. zu und erhitzt weitere 80 h. Dann saugt man heiss ab, waescht den Filterkuchen mit etwas heißem Butanol und stellt die vereinigten Filtrate kalt. Nach Stehen ueber Nacht wird abgesaugt, mit Ether gewaschen (2 × 50 ml) und getrocknet. Ausbeute : 33.4 g (64 % d.Th.) gelbe Kristalle, die aus Ethanol + Ether (2 : 1 Vol.) umkristallisiert werden. 28.45 g (55 % d.Th.) fast farbloses Hydrochlorid mit dem Schmp. 176-178 °C. Hygroskopisch.

Die Base (Schmp. 76-77 °C) stellt man her, indem man 10 g Hydrochlorid in 75 ml Ether suspendiert, bis zur stark alkalischen Reaktion konz. $NH_4OH$ zutropft, den Ether abtrennt und die waessr. Phase 2 × mit Ether extrahiert. Nach Trocknen ($Na_2SO_4$) wird eingedampft. 8.4 g (97 % d.Th.), nicht haltbar.

In analoger Weise erhaelt man bei Einsatz von 4-Aminobenzoesaeure-n-butylester die Verbindung

1 a) : 4-(Piperazin-1-yl) benzoesaeure-n-butylester
Ausb. 43 % d.Th. Hydrochlorid mit dem Schmp. 178-180 °C (i-Propanol).

## Beispiel 2

4-(1-n-Hexadecyl-piperazin-4-yl) benzoesaeure

a) Man haelt ein Gemisch aus 300 ml Butanon-2, 27.0 g (0.1 mol) 4-(Piperazin-1-yl) benzoesaeure-ethylester-hydrochlorid, 28 g $K_2CO_3$ pulv. sicc. und 30.5 g (0.1 mol) 1-Brom-hexadecan 30 h auf Rueckflusstemperatur. Dann saugt man heiss ab, waescht den Filterkuchen mit heissem Butanon-2 und dampft die vereinigten Filtrate ein. Es bleiben beigefarbene Kristalle mit dem Schmp. 76-78 °C zurueck. Man loest in wenig Ether, versetzt mit einer ausreichenden Menge HCl-haltigem Ether, saugt ab und kristallisiert das Hydrochlorid (roh : 47.7 g = 96 % d.Th.) aus 150 ml Ethanol + 50 ml Ether um. 29.4 g sandfarbener 4-(1-n-Hexadecyl-piperazin-4-yl) benzoesaeure-ethylester als Hydrochlorid, Schmp. 193-196 °C.

b) Man haelt das Gemisch aus 29.4 g (59.4 mmol) des obigen Ethylesters, 148.5 ml 1 N-KOH und 148.5 ml Ethanol 8 h auf Rueckflusstemperatur, kuehlt ab, saugt den ausgefallenen Niederschlag ab ; loest ihn in kochendem Wasser und gibt 150 ml 1 H-HCl zu. Nach dem Abkuehlen wird abgesaugt, mit Wasser gewaschen und bei 50 °C getrocknet. 21.7 g (78 % d.Th.) 4-(1-n-Hexadecyl-piperazin-4-yl) benzoesaeurehydrochlorid, Schmp. 252-254 °C.

In Analogie zu a) werden aus 4-(Piperazin-1-yl) benzoesaeure-ethylester die folgenden Produkte dargestellt :

a 1) mit Benzylchlorid : 4-[1-(4-Chlorbenzyl) piperazin-4-yl] benzoesaeure-ethylester.
Ausb. 73 % Hydrochlorid, Schmp. 172-173 °C (Ethanol + Ether 5 : 1 Vol.)

a 2) mit 1-Brom-2-phenoxyethan : 4-[1-(2-Phenoxyethyl) piperazin-4-yl] benzoesaeure-ethylester.
Ausb. 68 % d.Th. Hydrochlorid, Schmp. 200-202 °C (Ethanol + Ether 1 : 3 Vol.)

a 3) mit 1-Brom-3-phenylpropan : 4-[1-(3-Phenylpropyl) piperazin-4-yl] benzoesaeure-ethylester.
Ausb. 58 % d.Th. Hydrochlorid, Schmp. 175-178 °C (Ethanol + Ligroin)

In Analogie zu b) lassen sich aus den entsprechenden Benzoesaeure-ethylestern die folgenden Saeuren herstellen :

b 1) : 4-[1-(4-Chlorbenzyl) piperazin-4-yl] benzoesaeure.
Ausb. 75 % d. Th., Hydrochlorid : Schmp. 282-284 °C

b 2) : 4-[1-(2-Phenoxyethyl) piperazin-4-yl] benzoesaeure,
Ausb. 92 % d.Th., Hydrochlorid : Schmp. 240-242 °C (waeßr. Aceton).

## Beispiel 3

4-[1-(4-Chlorphenacyl) piperazin-4-yl] benzoesaeure-ethylester

Ein Gemisch aus 11.7 g (50 mmol) 4-(Piperazin-1-yl) benzoesaeure-ethylester, 13.8 g (0.1 mol) pulv. trocknem $K_2CO_3$, 125 ml Acetonitril und 11.7 g (50 mmol) 2-Brom-4'-chlor-acetophenon (p-Chlorphenacylbromid) ruehrt man 20 h bei 20 °C. Dann wird abgesaugt, der Filterkuchen mit Aceton gewaschen. Die vereinten organischen Phasen dampft man i. Vak. zur Trockne ein : 15.4 g (80 % d.Th.) Rohausbeute. Nach Umkrist. aus Essigester 9.8 g (51 % d.Th.), Schmp. 152-153 °C, aus der Mutterlauge weitere 1.4 g (7 % d.Th.), Schmp. 150-152 °C.

Dieser Ester liess sich nicht unzersetzt zur Saeure hydrolysieren.

In Analogie dazu werden aus 4-(Piperazin-1-yl) benzoesaeure-n-butylester die folgenden Produkte hergestellt :

3 a) mit 4-Methoxybenzylchlorid : 4-[1-(4-Methoxybenzyl) piperazin-4-yl] benzoesaeure-n-butylester.
Ausb. 72 % d.Th., Schmp. 77-78 °C (Acetonitril).

3 b) mit Phenethylbromid : 4-[1-(Phenethyl) piperazin-4-yl] benzoesaeure-n-butylester.
Ausb. 74 % d.Th., Schmp. 67-68 °C (Acetonitril).

## Beispiel 4

4-{1-[2-(3.5-Di-tert. butyl-4-hydroxyphenyl) ethyl] piperazin-4-yl} benzoesaeure-ethylester.

Zu einem Gemisch aus 30.0 g (0.12 mol) 3.5-Di-tert. butyl-4-hydroxy-phenethylalkohol und 40 ml abs. Pyridin gibt man bei 0-5 °C im Laufe einer Std. portionsweise 25.4 g (132 mmol) Tosylchlorid. Anschliessend ruehrt man 4 h bei 0 °C nach, giesst auf ca. 500 ml Eiswasser und extrahiert mit Ether. Die Etherphase wird zweimal mit Wasser gewaschen, mit $Na_2SO_4$, getrocknet und i. Vak. eingedampft (Badtemp. max. 20 °C, sonst Zersetzung). Den Rueckstand kristallisiert man aus Ligroin um : 39.5 g (82 % d.Th.) p-Toluolsulfonsaeure-(3.5-di-tert. butyl-4-hydroxyphenethylester), Schmp. 104-105 °C.

Man haelt ein Gemisch aus 20.2 g (50 mmol) p-Toluolsulfonsaeure-(3.5-di-tert. butyl-4-hydroxyphenethylester), 11.7 g (50 mmol) 4-(Piperazin-1-yl) benzoesaeure-ethylester, 6.9 g (50 mmol) pulv. $K_2CO_3$ sicc. und 200 ml Butanon-2 18 h auf Rueckflusstemperatur. Dann wird abgesaugt, der Filterkuchen mit Aceton gewaschen und im Vak. eingedampft. Man kristallisiert aus Ligroin um und erhaelt insgesamt 15.7 g (68 % d.Th.) 4-{1-[2-(3.5-Di-tert. butyl-4-hydroxy-phenyl) ethyl] piperazin-4-yl} benzoesaeure-ethylester mit dem Schmp. 131-132 °C.

## Beispiel 5

4-[1-(2-Phenoxypropyl) piperazin-4-yl] benzoesaeureethylester

In 60 ml HMPT loest man 17.6 g (75 mmol) 4-(Piperazin-1-yl) benzoesaeure-ethylester und 17.28 g (75 mmol) p-Toluolsulfonsaeure-(2-phenoxy-propylester), spuelt mit $N_2$ und haelt unter $N_2$ 12 h auf Rueckflusstemperatur. Danach erfolgt nochmals Zugabe von 10 mmol Tosylat. Man haelt weitere 20 h auf Rueckflusstemperatur, gibt 1.7 g Tosylat zu und erhitzt nochmals 6 h. Dann wird abgekuehlt, in Wasser eingeruehrt und dreimal mit $CH_2Cl_2$ extrahiert. Die vereinigten organischen Phasen werden mit Wasser gewaschen, mit $MgSO_4$ getrocknet und mit HCl-haltigem Ether versetzt. Nach dem Eindampfen kristallisiert der Rueckstand durch. 11.4 g (34 % d.Th.), nach Umkristallisieren aus Ethanol/Ether (1 : 3 Vol.) 8.1 g (24 % d.Th.) 4-[1-(2-Phenoxypropyl) piperazin-4-yl] benzoesaeure-ethylester als Hydrochlorid mit dem Schmp. 200-202 °C.

Anstelle der Tosylate kann man hier auch Halogenide einsetzen :

4-[1-(4-Chlorcinnamyl) piperazin-4-yl] benzoesaeure-n-butylester.

Man erhitzt ein Gemisch aus 9.4 g (50 mmol) 4-Chlorcinnamylchlorid und 13.1 g (50 mmol) 4-(Piperazin-1-yl) benzoesaeure-n-butylester in 40 ml Hexamethylphosphorsaeuretriamid 10 h auf 60 °C, fuegt 6.9 g (50 mmol) Kaliumcarbonat zu und ruehrt weitere 5 h bei 60 °C. Nach dem Erkalten verduennt man mit der zehnfachen Menge Wasser, nimmt die organischen Bestandteile in Essigester auf und dampft die getrockneten Essigester-Loesungen ein. Der Rueckstand wird aus Methanol umkristallisiert. Man erhaelt 10.0 g (48 %) Produkt mit dem Schmp. 115-117 °C.

Das Hydrochlorid schmilzt bei 166-168 °C.

## Beispiel 6

4-[1-(4-Chlorbenzoyl) piperazin-4-yl] benzoesaeure-ethylester

Zu einem Gemisch aus 13.5 g (50 mmol) 4-(Piperazin-1-yl) benzoesaeure-ethylester-hydrochlorid und 130 ml abs. Pyridin gibt man bei 5-10 °C 50 mmol 4-Chlorbenzoylchlorid, laesst auf 20 °C kommen und ruehrt schliesslich 6 h bei 40 °C. Nach dem Abkuehlen wird in 400 ml Eiswasser eingeruehrt, zweimal mit je 250 ml $CH_2Cl_2$ extrahiert und die organische Phase mit $Na_2SO_4$ getrocknet. Man dampft i. Vak. ein, verreibt mit Ligroin und kristallisiert aus 150 ml Ethanol um. 15.6 g farblose Kristalle, Schmp. 147-150 °C.

In Analogie dazu laeßt sich aus 4-(Piperazin-1-yl) benzoesaeure-ethylester-hydrochlorid herstellen :

6 a) mit 4-Chlorcinnamoylchlorid : 4-[1-(4-Chlorcinnamoyl) piperazin-4-yl] benzoesaeure-ethyl-ester. Ausb. 82 % d.Th., Schmp. 148-150 °C (Ethanol).

## Beispiel 7

4-{1-[3-(3.5-Di-tert. butyl-4-hydroxyphenyl) propionyl] piperazin-4-yl} benzoesaeureethylester

Man haelt ein Gemisch aus 23.4 g (0.1 mol) 4-(Piperazin-1-yl) benzoesaeure-ethylester (Base) 600 ml Xylol und 27.8 g (0,1 mol) 3-(3.5-Di-tert. butyl-4-hydroxyphenyl) propionsaeure bei vorgelegtem Wasserabscheider 28 h am Sieden, dampft dann i. Vak. zur Trockne ein und nimmt den Rueckstand in Ether auf. Man extrahiert zur Entfernung von unumgesetztem 4-(Piperazin-1-yl) benzoesaeure-ethylester mit 1 N HCl, waescht mit Wasser und $NaHCO_3$-Loesung, trocknet ($Na_2SO_4$) und versetzt mit HCl-haltigem Ether, wobei das Hydrochlorid ausfaellt. Dieses wird mit Ether gewaschen und aus Ethanol umkristallisiert. 32.0 g (60 % d.Th.) farbl. Kristalle, Schmp. 196-198 °C.

In analoger Weise erhaelt man aus 4-(Piperazin-1-yl) benzoesaeure-n-butylester und 4-Chlor-phenylessigsaeure.

7 a) : 4-[1-(4-Chlor-phenacetyl) piperazin-4-yl] benzoesaeure-n-butylester :

Man erhitzt ein Gemisch aus 17.0 g (0.1 mol) p-Chlorphenylessigsaeure, 26.2 g (0.1 mol) 4-(Piperazin-1-yl) benzoesaeure-n-butylester (Base) und 1 Liter Xylol 70 h unter Ruehren am Wasserabscheider. Dann dampft man ein und kristallisiert den nach mehrtaegigem Stehen allmaehlich fest werdenden Rueckstand aus Ethanol um. Man erhaelt 17.2 g (42 %) Produkt mit dem Schmp. 85-86 °C.

Beispiel 8

4-(1-Methansulfonyl-piperazin-4-yl) benzoesaeure-n-butylester.

Zu einem Gemisch aus 27 g (0.1 mol) 4-(Piperazin-1-yl) benzoesaeure-n-butylester. HCl und 270 ml abs. Pyridin gibt man bei 5-10 °C 11.4 g (0.1 mol) Methansulfonylchlorid, laesst ueber Nacht bei 20 °C stehen und giesst dann in 250 ml Eiswasser. Man extrahiert zweimal mit Methylenchlorid, trocknet ($Na_2SO_4$) und dampft i. Vak. ein. Nach Verreiben mit Ligroin 17.1 g (55 % d.Th.) gelbe Kristalle, Schmp. 145-150 °C.

In Analogie dazu erhaelt man aus 4-(Piperazin-1-yl) benzoesaeure-ethylester. HCl die folgenden Produkte :

8 a) mit 4-Chlorbenzolsulfochlorid : 4-[1-(4-Chlorbenzolsulfonyl) piperazin-4-yl] benzoesaeure-ethylester.
Ausb. 65 % d.Th., Schmp. 158-159 °C (Essigester + Ligroin)

8 b) mit Benzolsulfochlorid : 4-(1-Benzolsulfonyl-piperazin-4-yl) benzoesaeure-ethylester.
Ausb. 95 % d.Th., Schmp. 102-104 °C.

8 c) mit p-Toluolsulfochlorid : 4-[1-(4-Methylbenzolsulfonyl) piperazin-4-yl) piperazin-4-yl] benzoesaeure-ethylester.
Ausb. 84 % d.Th., Schmp. 122-124 °C (Ethanol + Ligroin)

Beispiel 9

4-(1-Methyl-piperazin-4-yl) benzoesaeure-n-butylester

19.6 g (75 mmol) 4-(Piperazin-1-yl) benzoesaeure-butylester werden in einem Gemisch aus 112 ml 80 proz. Ameisensaeure und 37.5 mol 40 proz. Formaldehydloesung 12 h auf Rueckflusstemperatur erhitzt. Nach dem Erkalten giesst man in 2.5 Liter Wasser, stellt mit 10 N Natronlauge alkalisch und extrahiert mit Methylenchlorid. Die Methylenchloridextrakte werden getrocknet und eingedampft. Man erhaelt 17.8 g (86 % d.Th.).
Produkt mit dem Schmp. 118-120 °C.

Beispiel 10

3-[1-(4-Butoxycarbonyl-phenyl) piperazin-4-yl]-propansulfonsaeure

Man ruehrt eine Loesung aus 6.1 g (50 mmol) γ-Propansulton, 13.1 g (50 mmol) 4-(Piperazin-1-yl) benzoesaeure-butylester und 75 ml Ethanol 8 Tage lang bei 20 °C. Dann saugt man den ausgefallenen Niederschlag ab und waescht ihn mit Ethanol.
Ausbeute : 17.3 g (90 % d.Th.), Schmp. 232-234 °C (waessr. Ethanol).

Beispiel 11

4-{1-[4-(2-Hydroxypropyl) phenyl] piperazin-4-yl} benzoesaeure-n-butylester

Man ruehrt ein Gemisch aus 13.1 g (50 mmol) 4-(Piperazin-1-yl) benzoesaeure-n-butylester, 2.9 g (50 mmol) Propylenoxid und 6 ml Methanol 24 h bei 20 °C. Dann dampft man das Loesungsmittel ab und verreibt den Rueckstand mit Ligroin.
Ausb. 78 % d.Th., Schmp. 85-86 °C.

Beispiel 12

4-[1-(3.5-Di-tert. butyl-4-hydroxy-cinnamoyl) piperazin-4-yl] benzoesaeure-ethylester

Zu einer Loesung aus 18.75 g (80 mmol) 4-(Piperazin-1-yl) benzoesaeure-ethylester und 22.1 g (80 mmol) 3.5-Di-tert. butyl-4-hydroxyzimtsaeure in 160 ml abs. Pyridin wird unter Eiskuehlung eine Loesung aus 5.5 g (40 mmol) Phosphortrichlorid und 25 ml abs. Pyridin zugetropft und das Reaktionsgemisch 1 h unter Eiskuehlung nachgeruehrt. Dann giesst man auf Eis und saeuert mit conc. Salzsaeure an. Man extrahiert die waessr. Phase mit Essigester, waescht die Extrakte dreimal mit 0.5 N Salzsaeure, trocknet und engt ein. Der Rueckstand wird zweimal aus einem Gemisch aus 240 ml Ethanol und 80 ml Wasser umkristallisiert. Man erhaelt 29.2 g (74 %) Produkt mit dem Schmp. 147-150 °C.

Beispiel 13

4-[1-(2-Phenoxypropyl) piperazin-4-yl] benzoesaeure

Man ruehrt ein Gemisch aus 20.2 g (50 mmol) 4-[1-(2-Phenoxypropyl) piperazin-4-yl] benzoesaeure-ethylester-hydrochlorid, 150 ml 1 N KOH und 150 ml Ethanol 2.5 h bei 80 °C, destilliert anschliessend i. Vak. das Ethanol ab und bringt mittels verd. Salzsaeure die waessrige Phase auf pH 5.5. Die ausfallenden Kristalle werden abgesaugt und aus Essigester umkristallisiert.

Ausbeute : 8,6 g (50.5 % d.Th.) 4-[1-(2-Phenoxypropyl) piperazin-4-yl] benzoesaeure mit dem Schmp. 152-154 °C.

In manchen Faellen gelingt es nur schwer, die freie Saeure durch Einstellen des isoelektrischen Punktes auszufaellen. Dann saeuert man mit Salzsaeure an und faellt damit das Hydrochlorid aus.

In Analogie zu obigem Beispiel werden hergestellt :

13 a) : 4-[1-(4-Chlorcinnamoyl) piperazin-4-yl] benzoesaeure.
Ausb. 90 % d.Th., Schmp. ab 286 °C (Zers.)

13 b) : 4-{1-[3-(3.5-Di-tert. butyl-4-hydroxyphenyl) propionyl] piperazin-4-yl} benzoesaeure.
Ausb. 65 % d.Th., Schmp. 235-238 °C (Essigester + Ligroin 1 : 1 Vol.)

13 c) : 4-(1-Methansulfonyl-piperazin-4-yl) benzoesaeure.
Ausb. 84 % d. Th., Schmp. > 300 °C.

13 d) : 4-[1-(4-Chlorbenzolsulfonyl) piperazin-4-yl] benzoesaeure.
Ausb. 95 % d.Th., Schmp. 289-291 °C.

13 e) : 4-[1-(4-Methoxybenzyl) piperazin-4-yl] benzoesaeure.
Ausb. 79 % d.Th. Hydrochlorid, Schmp. 272-273 °C.

13 f) : 4-(1-Phenethyl-piperazin-4-yl) benzoesaeure.
Ausb. 93 % d.Th. Hydrochlorid, Schmp. 292-293 °C.

13 g) : 4-[1-(4-Chlorcinnamyl) piperazin-4-yl] benzoesaeure.
Ausb. 73 % d.Th., Schmp. 231-233 °C (Ethanol). Das Hydrochlorid schmilzt bei 261-264 °C (waessr. Ethanol).

13 h) : 4-[1-(4-Chlor-phenacetyl) piperazin-4-yl] benzoesaeure.
Ausb. 70 % d.Th., Schmp. 250-252 °C.

13 i) : 4-[1-(3-Phenylpropyl) piperazin-4-yl] benzoesaeure.
Ausb. 77 % d.Th. Hydrochlorid-monohydrat, Schmp. 281-284 °C.

13 j) : 4-(1-Methyl-piperazin-4-yl) benzoesaeure
Ausb. 71 % d.Th., Schmp. 288-292 °C.

13 k) : 3-[1-(4-Hydroxycarbonyl-phenyl) piperazin-4-yl] propansulfonsaeure
Ausb. 91 % d.Th., Schmp. > 340 °C.

13 l) : 4-{1-[4-(2-Hydroxypropyl) phenyl] piperazin-4-yl} benzoesaeure.
Ausb. 84 % d.Th. Dihydrochlorid-monohydrat,
Schmp. 276-278 °C.

13 m) : 4-[1-(3.5-Di-tert. butyl-4-hydroxy-cinnamoyl)-piperazin-4-yl] benzoesaeure.
Ausb. 70 % d.Th., Schmp. 272 °C (Ethanol).

13 n) : 4-(1-Benzolsulfonyl-piperazin-4-yl) benzoesaeure.
Ausb. 81 % d.Th., Schmp. 292-295 °C.

13 o) : 4-[1-(p-Toluolsulfonyl) piperazin-4-yl) benzoesaeure.
Ausb. 94 % d.Th., Schmp. 297-300 °C.

Beispiel 14

4-(Piperazin-1-yl-methyl) benzoesaeure-methylester

Man versetzt ein Gemisch aus 38 g (0.2 mol) Piperazinhexahydrat, 28 g (0.2 mol) Piperazindihydrochlorid und 150 ml Methanol unter Ruehren bei Zimmertemperatur mit 45 g (0.2 mol) 4-Brommethylbenzoesaeure-methylester. Das Reaktionsgemisch laesst man 2 Tage stehen und saugt vom Niederschlag ab. Die klare Loesung wird eingedampft und der Rueckstand aus Ethanol umkristallisiert. Man erhaelt 39 g (64 %) der Titelverbindung als Monohydrochlorid mit dem Schmp. 226-227 °C.
Die Titelverbindung ist auch auf folgendem Wege darstellbar :

a) Man haelt ein Gemisch aus 7.9 g (50 mmol) 1-Ethoxy-carbonyl-piperazin, 150 ml Butanon-2, 6.9 g (0.1 mol) pulv. Kaliumcarbonat und 12.0 g (52.5 mmol) 4-(Brommethyl) benzoesaeure-methylester 3 Tage lang auf Rueckflußtemperatur. Dann wird abgesaugt und der Filterkuchen mit Aceton gewaschen. Man dampft die vereinigten Filtrate i. Vak. ein, nimmt den Rueckstand in Ether auf, waescht die Etherphase mit Wasser und trocknet mit $Na_2SO_4$. Durch Zugabe von HCl-haltigem Ether wird das Hydrochlorid ausgefaellt. Nach Umkristallisieren aus Dioxan erhaelt man 15,5 g (86 % d.Th.) 4-[1-(Ethoxycarbonyl) piperazin-4-yl-methyl] benzoesaeure-methylester-hydrochlorid mit dem Schmp. 201-202 °C.

b) Ein Gemisch aus 10.2 g (33 mmol) 4-[1-(Ethoxycarbonyl) piperazin-4-yl-methyl] benzoesaeure-methylester-hydrochlorid und 100 ml conc. HCl wird 60 h auf 95 °C gehalten ; dann kuehlt man stark ab, versetzt mit Aceton und saugt den ausgefallenen Niederschlag ab. Ausbeute : 8.0 g (83 % d.Th.) 4-(Piperazin-1-yl-methyl) benzoesaeure-dihydrochlorid, Schmp. : ab 270 °C Zers...

c) Man begast ein Gemisch aus 43.7 g (0.15 mol) 4-(Piperazin-1-yl-methyl) benzoesaeure-dihydrochlorid und 300 ml abs. Methanol bis zur Saettigung mit Chlorwasserstoff, wobei man die Temperatur auf ca. 55 °C ansteigen laesst. Anschliessend haelt man noch 1 h bei 55 °C und laesst ueber Nacht bei 20 °C ausreagieren. Nach Einengen auf das halbe Volumen wird stark abgekuehlt, abgesaugt und getrocknet.
Ausbeute : 42.1 g (91 % d.Th.) 4-(Piperazin-1-yl-methyl) benzoesaeure-methylester-dihydrochlorid, Schmp. 252-253 °C.

## Beispiel 15

4-[1-(Benzolsulfonyl) piperazin-4-yl-methyl] benzoesaeuremethylester

Man haelt ein Gemisch aus 11.5 g (50 mmol) 4-Brommethyl-benzoesaeuremethylester, 11.3 g (50 mmol) 1-Benzolsulfonyl-piperazin, 6.91 g (50 mmol) pulv. Kaliumcarbonat und 100 ml Butanon-2 20 h auf Rueckflusstemperatur, dann saugt man heiss ab und engt das Filtrat ein. Nach Kuehlen wird abgesaugt und aus Methanol umkristallisiert. 11.6 g (62 % d.Th.) Produkt mit dem Schmp. 145-146 °C.
In analoger Weise werden aus 4-Brommethyl-benzoesaeuremethylester die folgenden Produkte dargestellt :

15 a) mit 1-(2-Phenoxypropyl) piperazin : 4-[1-(2-Phenoxypropyl) piperazin-4-yl-methyl] benzoesaeuremethylester.
Ausb. 65 % d.Th. Hydrochlorid, Schmp. 242-244 °C.

15 b) mit 1-(4-Chlorbenzyl) piperazin : 4-[1-(4-Chlorbenzyl) piperazin-4-yl-methyl] benzoesaeuremethylester.
Ausb. 76 % d.Th., Schmp. 98-99 °C (Methanol)

15 c) mit 1-(n-Hexadecyl) piperazin : 4-[1-(n-Hexadecyl) piperazin-4-yl-methyl]-benzoesaeuremethylester.
Ausb. 72 % d.Th., Schmp. 47-48 °C (Methanol) und unter Anwendung von Acetonitril als Reaktionsmedium :

15 d) mit 1-(4-Chlorphenyl) piperazin : 4-[1-(4-Chlorphenyl) piperazin-4-yl-methyl]-benzoesaeuremethylester.
Ausb. 91 % d.Th. Hydrochlorid, Schmp. 208 °C.
Die freie Base schmilzt bei 109 °C (Isopropanol).

15 e) mit 1-(3.5-Di-tert. butyl-4-hydroxyphenethyl) piperazin : 4-[1-(3.5-di-tert. butyl-4-hydroxyphenethyl) piperazin-4-yl-methyl] benzoesaeure-methylester.
Ausb. 61 % d.Th., Schmp. 103-104 °C (Isopropanol).

## Beispiel 16

4-[1-(2-Hydroxypropyl) piperazin-4-yl-methyl] benzoesaeure-methylester

in Analogie zu Beispiel 11 aus 4-(Piperazin-1-yl-methyl) benzoesaeure-methylester und Propylenoxid.
Ausbeute 63 % d.Th., Schmp. 63-64 °C.

Beispiel 17

4-[1-(4-Chlorbenzoyl) piperazin-4-yl-methyl] benzoesaeure-methylester

Zu einer Loesung aus 15.3 g (50 mmol) 4-(Piperazin-1-yl-methyl) benzoesaeure-methylester und 175 ml abs. Pyridin tropft man bei 0-5 °C 8.7 g (50 mmol) 4-Chlor-benzoylchlorid und haelt eine weitere Stunde bei 0-5 °C, dann ueber Nacht bei 20 °C. Der Ansatz wird in Eiswasser eingeruehrt, das ausgefallene Produkt abgesaugt, mit Wasser gewaschen und getrocknet. Ausbeute : 18.5 g (quantitativ), Schmp. 134-135 °C.

In analoger Weise erhaelt man aus 4-(Piperazin-1-yl-methyl) benzoesaeure-methylester.

17 a) mit 4-Chlor-cinnamoylchlorid : 4-[1-(4-Chlorcinnamoyl) piperazin-4-yl-methyl] benzoesaeure-methylester.
Ausbeute : 84 % d.Th., Schmp. 137-138 °C (Methanol).

Beispiel 18

4-[1-(n-Hexadecyl) piperazin-4-yl-methyl] benzoesaeure

Man haelt ein Gemisch aus 40 mmol 4-[1-(n-Hexadecyl) piperazin-4-yl-methyl] benzoesaeure-methylester, 100 ml 6 N HCl und 20 ml Dioxan 16 h auf Rueckflusstemperatur, engt dann ein, kuehlt ab und saugt ab. Nach Umkristallisieren aus einem DMF-Wasser-Gemisch 62 % d.Th. Hydrochlorid, Schmp. 245 °C.

Beispiel 19

Man erhitzt ein Gemisch aus dem der Saeure entsprechenden Ethylester, 1 N Kalilauge (dreifache molare Menge) und einem gleichen Volumen Methanol auf 40-60 °C, bis im Duennschichtchromatogramm kein Ausgangsmaterial mehr feststellbar ist. Dann wird das Methanol abdestilliert und mittels einer der Kalilauge aequivalenten Menge 1 N HCl neutralisiert. Die freie Piperazin-carbonsaeure wird nun abgesaugt oder, falls sie nicht ausfaellt, durch Ansaeuren mit HCl als Hydrochlorid abgeschieden. Man saugt ab, trocknet und kristallisiert um.

Nach diesem Verfahren werden folgende Saeuren dargestellt :

19 a) : 4-[1-(2-Hydroxypropyl) piperazin-4-yl-methyl] benzoesaeure.
Ausb. 69 % d.Th. Dihydrochlorid, Schmp. 281-283 °C.

19 b) : 4-[1-(3.5-Di-tert. butyl-4-hydroxyphenethyl) piperazin-4-yl-methyl] benzoesaeure.
Ausb. 76 % d.Th. Dihydrochlorid, Schmp. 285-288 °C (Z.).

19 c) : 4-[1-(4-Chlorphenyl) piperazin-4-yl-methyl] benzoesaeure.
Ausb. 60 % d.Th. Hydrochlorid, Schmp. 258-261 °C (Wasser).

19 d) : 4-[1-(4-Chlorbenzoyl) piperazin-4-yl-methyl] benzoesaeure.
Ausb. 69 % d.Th. Hydrochlorid, Schmp. 270-273 °C.

19 e) : 4-[1-(4-Chlor-cinnamoyl) piperazin-4-yl-methyl] benzoesaeure.
Ausb. 92 % d.Th. Hydrochlorid, Schmp. 289-290 °C.

19 f) : 4-[1-(Benzolsulfonyl) piperazin-4-yl-methyl] benzoesaeure.
Ausb. 72 % d.Th. Hydrochlorid, Schmp. 268-269 °C (waessr. Ethanol).

Beispiel 20

1) 4-[2-(Piperazin-1-yl) ethyl] benzoesaeure-ethylester

a) Man haelt ein Gemisch aus 51.4 g (0.2 mol) 4-(2-Bromethyl) benzoesaeureethylester, 500 ml Butanon-2, 35.2 g (0.2 mol) Benzylpiperazin und 55.3 g (0.4 mol) pulv. $K_2CO_3$ sicc. 48 h auf Rueckflusstemperatur, saugt dann ab und dampft i. Vak. ein. Den Rueckstand loest man in Ether. Die Etherphase wird mehrmals mit Wasser gewaschen, mit $Na_2SO_4$ getrocknet, dann mit HCl-haltigem Ether in ausreichender Menge versetzt. Nach Absaugen und Trocknen 15.9 g (75 % d.Th.) 4-[2-(1-Benzylpiperazin-4-yl) ethyl] benzoesaeureethylester-dihydrochlorid, Schmp. 277-278 °C (Zers.).

b) Man hydriert ein Gemisch aus 63.8 g (0.15 mol) 4-[2-(1-Benzylpiperazin-4-yl) ethyl] benzoesaeure-ethylester (als Di-hydrochlorid), 350 ml Wasser, 650 ml Ethanol und ca. 5 g 10 proz. Pd-Kohle bei 20 °C und Normaldruck. Anschliessend wird der Alkohol i. Vak. abdestilliert, die kalte Loesung mittels

halbkonz. $NH_4OH$ deutlich alkalisch gestellt und sofort mehrmals ausgeethert. Die vereinigten Etherextrakte trocknet man mit Kaliumcarbonat, gibt dann ausreichend HCl-haltigen Ether zu, saugt ab und trocknet. 38.5 g (77 % d.Th.) Dihydrochlorid, Schmp. 276 °C (Z.) (waessr. Ethanol).

2) In analoger Weise erhaelt man

4-[3-(Piperazin-1-yl) propyl] benzoesaeure-ethylester :

$a_1$) Aus 1-Benzyl-piperazin und 4-(3-Brompropyl) benzoesaeure-ethylester wird 4-[3-(1-Benzyl-piperazin-4-yl) propyl] = benzoesaeure-ethylester-dihydrochlorid hergestellt.
Ausbeute 76 % d.Th. Dihydrochlorid, Schmp. 253-254 °C (Ethanol).

$a_2$) Hydrieren der Benzylverbindung in einem Gemisch aus Ethanol und 2 N Salzsaeure (4 : 1 Vol.) in Gegenwart von 10 proz. Pd-Kohle liefert 4-[3-(Piperazin-1-yl) propyl] = benzoesaeure-ethylester-dihydrochlorid. Ausbeute 93 % d.Th., Schmp. 228-230 °C (Ethanol).

Beispiel 21

4-{2-[1-(4-Methoxybenzyl) piperazin-4-yl] ethyl} benzoesaeure-ethylester

Man haelt ein Gemisch aus 250 ml Butanon-2, 15.47 g (75 mmol) 1-(4-Methoxybenzyl) piperazin, 19.3 g (75 mmol) 4-(2-Bromethyl ; benzoesaeureethylester sowie 20.7 g (0.15 mol) pulv. $K_2CO_3$ 60 h auf Rueckflusstemperatur, saugt dann heiss ab und dampft i. Vak. ein. Nach Zugabe von Ether kristallisiert das Produkt : 17.5 g (61 % d.Th.), Schmp. 75-76 °C. Aus dem Filtrat wird mittels HCl-haltigem Ether das Hydrochlorid ausgefaellt. Nach Umkristallisieren aus waessrigem Ethanol 7.6 g (23 % d.Th.), Schmp. 280-281 °C.
In analoger Weise werden aus 4-(2-Bromethyl) benzoesaeure-ethylester dargestellt :

21 a) mit 1-Methylpiperazin : 4-[2-(1-Methylpiperazin-4-yl) ethyl] benzoesaeure-ethylester.
Ausb. 66 % d.Th. Hydrochlorid, Schmp. 273 °C (Ethanol)

21 b) mit 1-(2-Hydroxypropyl) piperazin : 4-{2-[1-(2-Hydroxypropyl) piperazin-4-yl] ethyl} benzoesaeure-ethylester.
Ausb. 75 % d.Th. Hydrochlorid, Schmp. 271-272 °C (Ethanol). Freie Base : Schmp. 128-129 °C.

21 c) mit 1-[1-(3.5-Di-tert. butyl-4-hydroxy-benzoyl) ethyl] piperazin, hier in Acetonitril : 4-{2-[1-(1-(3.5-Di-tert. butyl-4-hydroxy-benzoyl) ethyl) piperazin-4-yl] ethyl} benzoesaeure-ethylester.
Ausb. 70 % d.Th. Dihydrochlorid, Schmp. 236-240 °C.

21 d) mit 1-(Phenethyl) piperazin : 4-{2-[1-(Phenethyl) piperazin-4-yl] ethyl} benzoesaeure-ethylester.
Ausb. 67 % d.Th. Dihydrochlorid, Schmp. 289 °C (Z.).

21 e) mit 1-(4-Chlorphenyl) piperazin : 4-{2-[1-(4-Chlorphenyl) piperazin-4-yl] ethyl} benzoesaeure-ethylester.
Ausb. 56 % d.Th. Hydrochlorid, Schmp. 210 °C (waessr. Ethanol).

21 f) mit 1-[2-(4-Tolyloxy) propyl] piperazin : 4-{2-[1-(2-(4-Methylphenoxy) propyl) piperazin-4-yl] ethyl} benzoesaeure-ethylester.
Ausb. 69 % d.Th. Dihydrochlorid, Schmp. 247-248 °C.

21 g) mit 1-(4-Phenoxyphenyl) piperazin : 4-{2-[1-(4-Phenoxyphenyl) piperazin-4-yl] ethyl} = benzoesaeure-ethylester.
Ausbeute 78 % d.Th. Dihydrochlorid, Schmp. 193-194 °C (Ethanol), Schmp. der freien Base : 96-97 °C (Ether).

21 h) mit 1-(4-Benzyloxyphenyl) piperazin : 4-{2-[1-(4-Benzyloxyphenyl) piperazin-4-yl] ethyl} = benzoesaeure-ethylester
Ausbeute 55 % d.Th., Schmp. 145-146 °C (Essigester/Ether).

21 i) mit 1-(3-Trifluormethyl-phenyl) piperazin : 4-{2-[1-(3-Trifluormethylphenyl) piperazin-4-yl] = ethyl} benzoesaeure-ethylester.
Ausbeute 49 % d.Th. Hydrochlorid, Schmp. 183-185 °C (Ethanol).
Aus 4-(2-Bromethyl) benzoesaeure-methylester erhaelt man.

21 j) mit 1-(2-Chlorbenzyl) piperazin : 4-{2-[1-(2-Chlorbenzyl) piperazin-4-yl] ethyl} benzoesaeure-methylester.

Ausbeute 60 % d.Th. Dihydrochlorid, Schmp. 228-230 °C.
Die freie Base schmilzt bei 68-70 °C (Ligroin).

21 k) und mit 1-(3.5-Di-tert. butyl-4-hydroxy-phenethyl) = piperazin : 4-{2-[1-(3.5-Di-tert. butyl-4-hydroxy-phenethyl) = piperazin-4-yl] ethyl} benzoesaeure-methylester.
Ausbeute 76 % d.Th., Schmp. 109-110 °C.
Dihydrochlorid : Schmp. 274 °C (Ethanol).
Aus 4-(2-Bromethyl) benzoesaeure-n-butylester erhaelt man analog.

21 l) mit 1-(2-Chlorbenzyl) piperazin : 4-{2-[1-(2-Chlorbenzyl) piperazin-4-yl] ethyl} = benzoesaeure-n-butylester.
Ausbeute 53 % d.Th. Dihydrochlorid, Schmp. 228-230 °C (n-Butanol).

## Beispiel 22

4-{2-[1-(2-Phenoxypropyl) piperazin-4-yl] ethyl} benzoesaeure-ethylester.

Man erhitzt ein Gemisch aus 8.2 g (37 mmol) 1-(2-Phenoxypropyl)-piperazin, 9.57 g (37 mmol) 4-(2-Bromethyl) benzoesaeureethylester, 3.77 g (37 mmol) Triethylamin und 100 ml abs. Tetrahydrofuran 36 h auf Rueckflusstemperatur. Dann dampft man ein, nimmt den Rueckstand in Ether auf und faellt aus dieser Loesung mit Chlorwasserstoff enthaltendem Ether das Hydrochlorid. Man erhaelt 14.7 g (84 %) der Titelverbindung als Hydrochlorid mit dem Schmp. 232 °C.

In analoger Weise stellt man unter Verwendung von 4-(2-Bromethyl) benzoesaeure-ethylester folgende Produkte her :

22 a) mit 1-(2-Methyl-3-phenyl-propyl) piperazin · 2HCl : 4-{2-[1-(2-Methyl-3-phenyl-propyl) piperazin-4-yl] = ethyl} benzoesaeure-ethylester.
Ausbeute 53 % d.Th. Dihydrochlorid, Schmp. 262 °C.

22 b) mit 1-[3-(4-Chlorphenyl)-2-methyl-propyl] piperazin · 2HCl : 4-{2-[1-(3-(4-Chlorphenyl)-2-methyl-propyl) piperazin-4-yl] ethyl} benzoesaeure-ethylester.
Ausbeute 56 % d.Th. Dihydrochlorid, Schmp. 278-279 °C (Ethanol).

## Beispiel 23

4-{2-[1-(4-Chlorcinnamyl) piperazin-4-yl] ethyl} benzoesaeure-ethylester.

Man haelt ein Gemisch aus 150 ml Butanon-2, 9.45 g (36 mmol) 4-[2-(Piperazin-1-yl) ethyl] benzoesaeure-ethylester, 7.5 g (40 mmol) 4-Chlorcinnamylchlorid und 5.55 g (40 mmol) pulv. $K_2CO_3$ sicc. 40 h auf Rueckflusstemperatur. Dann wird heiss abgesaugt und eingedampft. Das Rohprodukt loest man in Essigester. Durch Zugabe von HCl-haltigem Ether faellt man das Dihydrochlorid. 78 % d.Th., Schmp. 282 °C (Z.). Die freie Base schmilzt bei 44-45 °C.

In analoger Weise lassen sich aus 4-[2-(Piperazin-1-yl) ethyl] benzoesaeure-ethylester die folgenden Produkte darstellen :

23 a) mit α-Brom-propionsaeure-ethylester : 4-{2-[1-(1-Ethoxycarbonyl-ethyl) piperazin-4-yl] ethyl} benzoesaeure-ethylester.
Ausb. 94 % d.Th., viskoses Oel.

23 b) mit 1-n-Hexadecylbromid : 4-[2-(1-n-Hexadecyl-piperazin-4-yl) ethyl] benzoesaeure-ethylester.
Ausb. 61 % d.Th., Schmp. 54-56 °C (Aceton).

23 c) mit 4-Chlor-benzylchlorid : 4-{2-[1-(4-Chlorbenzyl) piperazin-4-yl] ethyl} benzoesaeure-ethylester.
Ausb. 93 % d.Th., Schmp. 44-45 °C.

23 d) mit 2-Phenoxy-ethylbromid : 4-{2-[1-(2-Phenoxyethyl) piperazin-4-yl] ethyl} benzoesaeure-ethylester.
Ausb. 96 % d.Th. Hydrochlorid, Schmp. 256 °C.

23 e) mit 2-(4-(Chlorphenoxy) propylbromid : 4-{2-[1-(2-(4-Chlorphenoxy) propyl) piperazin-4-yl] ethyl} benzoesaeure-ethylester.
Ausb. 72 % d.Th. Hydrochlorid, Schmp. 250-253 °C (Methanol).

23 f) mit 2-(4-Methoxyphenoxy) propylbromid : 4-{2-[1-(2-(4-Methoxyphenoxy) propyl) piperazin-4-yl] ethyl} benzoesaeure-ethylester.
Ausb. 79 % d.Th. Hydrochlorid, Schmp. 243-246 °C (Methanol).

23 g) mit 2-(4-Nitrophenoxy) propylbromid : 1-(4-Ethoxycarbonyl-phenethyl)-4-[2-(4-nitrophenoxy) = propyl] piperazin.
Ausbeute 73 % d.Th. Dihydrochlorid, Schmp. 249-250 °C (waessr. Ethanol).

23 h) mit 2-(4-Cyanphenoxy) propylbromid : 1-(4-Ethoxycarbonyl-phenethyl)-4-[2-(4-cyanophenoxy) = propyl] piperazin.
Ausbeute 66 % d.Th. Dihydrochlorid, Schmp. 243-244 °C (waessr. Ethanol).

23 i) mit 3-Chlor-benzylchlorid : 4-{2-[1-(3-Chlorbenzyl) piperazin-4-yl] ethyl} benzoesaeure-ethylester.
Ausbeute 84 % d.Th. Dihydrochlorid, Schmp. 259-260 °C (Ethanol + einige Tropfen verd. HCl).

23 j) mit 3-Trifluormethyl-benzylchlorid : 4{2-[1-(3-Trifluormethyl-benzyl) piperazin-4-yl] ethyl} = benzoesaeure-ethylester.
Ausbeute 75 % d.Th. Dihydrochlorid, Schmp. 249-251 °C (Ethanol + 2 N Salzsaeure).

23 k) mit 4-Fluor-benzylchlorid : 4-{2-[1-(4-Fluorbenzyl) piperazin-4-yl] ethyl} benzoesaeure-ethylester.
Ausbeute 73 % d.Th. Dihydrochlorid, Schmp. 293 °C (Zers.) (Ethanol + 2 N Salzsaeure).

23 l) mit 3-Trifluormethyl-phenethylchlorid : 4-{2-[1-(3-Trifluormethyl-phenethyl) piperazin-4-yl] ethyl}-benzoesaeure-ethylester.
Ausbeute 77 % d.Th. Sulfat, Schmp. 256-258 °C (Wasser).

23 m) mit 3.4-Dichlorcinnamylchlorid : 4-{2-[1-(3.4-Dichlor-cinnamyl) piperazin-4-yl] ethyl} benzoesaeure-ethylester.
Ausbeute 62 % d.Th. Dihydrochlorid, Schmp. 245 °C (Zers.) (Ethanol).

## Beispiel 24

In Analogie zu Beispiel 23, jedoch mit Acetonitril anstelle von Butanon-2, erhaelt man aus 4-[2-(Piperazin-1-yl) ethyl] benzoesaeure-ethylester folgendes Produkt : mit 4-Chlor-phenacylbromid bei 20 °C : 4-{2-[1-(4-Chlorphenacyl) piperazin-4-yl] ethyl} benzoesaeure-ethylester.
Ausb. 67 % d.Th. Hydrochlorid, Schmp. 251-253 °C.

## Beispiel 25

4-{2-[1-(4-Chlorcinnamyl) piperazin-4-yl] ethyl} benzoesaeure-ethylester.

Man haelt ein Gemisch aus 33.5 g (0.1 mol) 4-[2-(Piperazin-1-yl) ethyl] benzoesaeure-ethylester-dihydrochlorid, 19.6 g (105 mmol) 4-Chlor-cinnamyl-chlorid, 40.8 g (0.4 mol) abs. Triethylamin und 400 ml abs. Tetrahydrofuran 24 h auf Rueckflusstemperatur und dampft anschliessend i. Vak. ein. Der Rueckstand wird mit Methylenchlorid und Wasser versetzt. Nach kraeftigem Durchmischen trennt man die Phasen. Die Methylenchlorid-Phase wird mehrfach mit Wasser gewaschen, dann getrocknet ($Na_2SO_4$). Man dampft dann ein, loest in wenig Methylenchlorid und fuegt HCl-haltigen Ether zu, worauf das Dihydrochlorid ausfaellt. Dieses wird abgesaugt, mit Ether gewaschen, mit heissem Ethanol digeriert und schließlich getrocknet. 39.5 g Dihydrochlorid mit dem Schmp. 283-285 °C.

In analoger Weise laeßt sich unter Verwendung des Ausgangsmaterials 4-[(Piperazin-1-yl) methyl]-benzoesaeure-ethylester-dihydrochlorid das folgende Produkt darstellen :

25 a) mit 4-Chlorcinnamylchlorid : 4-[1-(4-Chlorcinnamyl) piperazin-4-yl-methyl]-benzoesaeure-ethylester.
Ausbeute 84 % d.Th. Dihydrochlorid, Schmp. 241-241.5 °C und wiederum unter Verwendung des Ausgangsmaterials 4-[2-(Piperazin-1-yl) ethyl] benzoesaeure-ethylester-dihydrochlorid die folgenden Produkte :

25 b) mit 2-(4-Cyanophenoxy) propylbromid : 1-(4-Ethoxycarbonyl-phenethyl)[2-(4-cyanophenoxy) = propyl] piperazin.
Ausbeute 69 % d.Th. Dihydrochlorid, Schmp. 243-244 °C (waessr. Ethanol).

25 c) mit 2-Chlor-cinnamylchlorid : 4-{2-[1-(2-Chlorcinnamyl) piperazin-4-yl] ethyl} benzoesaeure-ethylester.
Ausbeute 89 % d.Th. Dihydrochlorid, Schmp. 262-264 °C (Ethanol).

25 d) mit 3-Brom-cinnamylchlorid : 4-{2-[1-(3-Bromcinnamyl) piperazin-4-yl] ethyl} benzoesaeure-ethylester.
Ausbeute 89 % d.Th. Dihydrochlorid, Schmp. 277-278 °C.

25 e) mit 4-Nitro-cinnamylchlorid : 4-{2-[1-(4-Nitrocinnamyl) piperazin-4-yl] ethyl} benzoesaeure-ethylester.
Ausbeute 91 % d.Th. Dihydrochlorid, Schmp. 256-259 °C.

25 f) mit 4-Cyano-cinnamylbromid : 4-{2-[1-(4-Cyanocinnamyl) piperazin-4-yl] ethyl} = benzoesaeure-ethylester.
Ausbeute 65 % d.Th., Schmp. 99-100 °C (Isooctan).

25 g) mit 3-Methoxycinnamylchlorid : 4-{2-[1-(3-Methoxycinnamyl) piperazin-4-yl] ethyl} = benzoesaeure-ethylester.
Ausbeute 96 % d.Th. Dihydrochlorid, Schmp. 265-267 °C.

25 h) mit 3-Trifluormethyl-cinnamylchlorid : 4-{2-[1-(3-Trifluormethyl-cinnamyl) piperazin-4-yl] = ethyl} benzoesaeure-ethylester.
Ausbeute 87 % d.Th. Dihydrochlorid, Schmp. 272-275 °C.
Aus 4-[3-(Piperazin-1-yl) propyl] benzoesaeure-ethylester-dihydrochlorid laesst sich analog herstellen :

25 i) mit 4-Chlor-cinnamylchlorid : 4-{3-[1-(4-Chlorcinnamyl) piperazin-4-yl] propyl} = benzoesaeure-ethylester.
Ausbeute 76 % d.Th. Dihydrochlorid, Schmp. 245-247 °C (waessr. Ethanol).
und aus 4-(Piperazin-1-yl) phenylessigsaeure-ethylester.

25 j) mit 4-Chlor-cinnamylchlorid : 4-[1-(4-Chlorcinnamyl) piperazin-4-yl] phenylessigsaeure-ethylester.
Ausbeute 67 % d.Th. Dihydrochlorid, Schmp. 200-201 °C (Eisessig).

## Beispiel 26

4-{2-[1-(3-Phenylpropyl) piperazin-4-yl] ethyl} benzoesaeure-ethylester.

Man haelt ein Gemisch aus 40 ml HMPT, 23.6 g (90 mmol) 4-[2-(Piperazin-1-yl) ethyl] benzoesaeure-ethylester und 8.96 g (45 mmol) 3-Brompropyl-benzol 22 h auf 110 °C, wobei gleich nach dem Zusammengeben eine Temperaturerhoehung (auf ca. 55 °C) und Bildung eines hellen Niederschlages zu beobachten sind. Bei 110 °C liegt dann eine klare, rotbraune Loesung vor, aus der beim Erkalten Produkt auskristallisiert. Man giesst in wenig Wasser, extrahiert mit Ether, waescht die Etherphase mit Wasser, trocknet ($Na_2SO_4$) und dampft ein. Das Rohprodukt wird in Ether geloest und mittels HCl-haltigem Ether in das Dihydrochlorid umgewandelt. Umkristallisieren aus Ethanol + 2 Tr. conc. HCl : Schmp. 273 °C (Z.).
Ausb. 76 % d.Th.

## Beispiel 27

4-{2-[1-(4-Chlorphenylacetyl) piperazin-4-yl] ethyl} benzoesaeure-ethylester.

Zu einem Gemisch aus 9.7 g (37 mmol) 4-[2-(Piperazin-1-yl) ethyl] benzoesaeure-ethylester, 40 ml $CH_2Cl_2$ und 6.3 g (80 mmol) abs. Pyridin tropft man bei 0 °C waehrend 40 min eine Loesung aus 7.0 g (37 mmol) 4-Chlor-phenylacetyl-chlorid und 20 ml $CH_2Cl_2$, wobei ein kaesiger Niederschlag entsteht. Nach 4 h Ruehren bei 20 °C giesst man in 250 ml Eiswasser, macht mit Ammoniak alkalisch und extrahiert mehrmals mit $CH_2Cl_2$. Die $CH_2Cl_2$-Phase wird zweimal mit Wasser gewaschen, mit $Na_2SO_4$ getrocknet und eingedampft. Nach Zusatz von Ligroin tritt Kristallisation ein. Man kristallisiert aus Ether um und erhaelt 51 % d.Th. Produkt mit dem Schmp. 88-89 °C.
In Analogie dazu erhaelt man aus 4-[2-(Piperazin-1-yl) ethyl] benzoesaeure-ethylester (Dihydrochlorid) folgende Produkte :

27 a) mit 4-Chlor-benzoylchlorid : 4-{2-[1-(4-Chlorbenzoyl) piperazin-4-yl] ethyl} benzoesaeure-ethylester.
Ausb. 71 % d.Th., Schmp. 80-82 °C (Essigester + Ligroin).

27 b) mit 4-Chlor-cinnamoylchlorid : 4-{2-[1-(4-Chlor-cinnamoyl) piperazin-4-yl] ethyl} benzoesaeure-ethylester.
Ausb. 68 % d.Th., Schmp. 114-116 °C (Ethanol + Ligroin).

Beispiel 28

4-{2-[1-(3.5-Di-tert. butyl-4-hydroxy-cinnamoyl) piperazin-4-yl] ethyl} benzoesaeure-ethylester.

Zu einem Gemisch aus 16.8 g (50 mmol) 4-[2-(Piperazin-1-yl) ethyl] benzoesaeure-ethylester-dihydrochlorid und 200 ml abs. Pyridin tropft man bei 5-10 °C waehrend 40 min eine Loesung aus 14.8 g 3.5-Di-tert. butyl-4-hydroxy-cinnamoylchlorid und 50 ml abs. Benzol. Man ruehrt 4 h bei 20 °C, destilliert i. Vak. weitgehend Benzol und Pyridin ab und ruehrt den Rueckstand in ca. 1 Liter Eiswasser ein. Der ausfallende, schmierige Niederschlag wird in $CH_2Cl_2$ aufgenommen. Die $CH_2Cl_2$-Phase waescht man mit Sodaloesung und Wasser, trocknet ($Na_2SO_4$) und dampft i. Vak. zur Trockne. Die Rohbase wird in Ethanol geloest, mit HCl begast und schliesslich mittels Ether als Hydrochlorid gefaellt. 17.3 g (62 % d.Th.), nach Ausruehren mit Ether Schmp. 193-195 °C (Z.).

Beispiel 29

4-{2-[1-(3-(3.5-Di-tert. butyl-4-hydroxyphenyl) propionyl) piperazin-4-yl] ethyl} benzoesaeure-ethylester.

wird in Analogie zu Beispiel 12 aus 4-[2-(Piperazin-1-yl) ethyl] benzoesaeure-ethylester, 3-(3.5-Di-tert. butyl-4-hydroxyphenyl) propionsaeure, Phosphortrichlorid und Pyridin hergestellt.
Ausb. 62 % d.Th., Schmp. 105-107 °C (Essigester + Ligroin).

Beispiel 30

4-[2-(1-Benzolsulfonyl-piperazin-4-yl) ethyl] benzoesaeure-ethylester.

Zu einer Loesung aus 16.75 g (50 mmol) 4-[2-(Piperazin-1-yl) ethyl] benzoesaeure-ethylester-dihydrochlorid und 250 ml abs. Pyridin tropft man bei 10-15 °C unter heftigem Ruehren 8.83 g (50 mmol) Benzolsulfochlorid. Nach 24 h Stehen bei 20 °C zieht man i. Vak. 2/3 des Pyridins ab, ruehrt in Eiswasser ein und saugt den ausgefallenen Niederschlag ab. Nach Waschen mit Wasser und Trocknen wird aus Essigester umkristallisiert : 17.6 g (88 % d.Th.), Schmp. 156-157 °C.
In analoger Weise erhaelt man aus 4-[2-(Piperazin-1-yl) ethyl] benzoesaeure-ethylester.

30 a) mit Tosylchlorid : 4-{2-[1-(p-Toluolsulfonyl) piperazin-4-yl] ethyl} benzoesaeure-ethylester.
Ausb. 80 % d.Th., Schmp. 141 °C (Essigester).

30 b) mit Methansulfonylchlorid : 4-[2-(1-Methansulfonyl-piperazin-4-yl) ethyl] benzoesaeure-ethylester.
Ausb. 87 % d.Th., Schmp. 132-134 °C (Essigester + Ligroin)

30 c) mit 4-Chlor-benzolsulfonylchlorid : 4-{2-[1-(4-Chlor-benzolsulfonyl) piperazin-4-yl] ethyl} benzoesaeure-ethylester.
Ausb. 70 % d.Th., Schmp. 148-150 °C (Essigester + Ligroin).

Beispiel 31

4-{2-[1-(Acetophenon-2-sulfonyl) piperazin-4-yl] ethyl} benzoesaeure-ethylester.

Zu einer Loesung aus 24.2 g (92 mmol) 4-[2-(Piperazin-1-yl) ethyl] benzoesaeure-ethylester in 65 ml abs. Ether wird unter Ruehren und Eiskuehlung eine Loesung aus 10.1 g (46 mmol) ω-Acetophenonsulfonylchlorid in 260 ml abs. Ether zugetropft. Man ruehrt noch 2 h bei 20 °C nach und laesst 3 Tage stehen. Das Reaktionsgemisch wird dann mit Wasser versetzt und kraeftig geschuettelt. Die Etherphase wird abgetrennt und die waessrige Phase dreimal mit Essigester rueckextrahiert. Die vereinigten Essigesterextrakte werden gemeinsam mit der Etherphase getrocknet und eingedampft. Den Rueckstand kristallisiert man zweimal aus Ethanol um. Man erhaelt 8.0 g (39 %) der Titelverbindung mit dem Schmp. 115-117 °C.

Beispiel 32

3-[1-(4-Ethoxycarbonyl-phenethyl) piperazin-4-yl] propansulfonsaeure.

Man gibt zu einer Loesung aus 10.25 g (39 mmol) 4-[2-(Piperazin-1-yl) ethyl] benzoesaeure-ethylester und 70 ml abs. Ethanol 4.76 g (39 mmol) 1.3-Propansulton und ruehrt bei 20 °C nach. Nach Stehen ueber

Nacht zieht man Ethanol ab und behandelt den Rueckstand mit Ether und Ligroin. Nach dem Absaugen wird aus Ethanol umkristallisiert : 11.2 g (75 % d.Th.), Schmp. 235-236 °C.

Beispiel 33

4-{2-[1-(3-Phenyl-propyl) piperazin-4-yl] ethyl} benzoesaeure.

Man haelt ein Gemisch aus 14.1 g (31 mmol) 4-{2-[1-(3-Phenylpropyl) piperazin-4-yl] ethyl} benzoesaeure-ethylester-dihydrochlorid, 100 ml Ethanol und 100 ml 1 N KOH 2 h auf 50 °C, dann destilliert man i. Vak. das Ethanol ab. Nach Ansaeuern mit HCl faellt das Dihydrochlorid aus. Man saugt ab, waescht mit 1 N HCl und trocknet. Ausbeute : 10.5 g (96 % d.Th.) Dihydrochlorid mit dem Schmp. 303 °C (Z.).
In analoger Weise werden hergestellt :

33 a) : 4-{2-[1-(1-Carboxy-ethyl) piperazin-4-yl] ethyl} benzoesauere.
Ausb. 63 % d.Th., Schmp. 273-276 °C.
Hydrochlorid : Schmp. 252-255 °C.

33 b) : 4-{2-[1-(4-Chlorbenzyl) piperazin-4-yl] ethyl} benzoesaeure.
Ausb. 76 % d.Th., Schmp. 195-197 °C (Methanol).
Dihydrochlorid : Schmp. > 260 °C.

33 c) : 4-{2-[1-(2-Phenoxyethyl) piperazin-4-yl] ethyl} benzoesaeure.
Ausb. 86 % d.Th., Schmp. 208-210 °C.
Dihydrochlorid : Schmp. 265-268 °C.

33 d) : 4-{2-[1-(2-Phenoxypropyl) piperazin-4-yl] ethyl} benzoesaeure.
Ausb. 62 % d.Th. Hydrochlorid, Schmp. 245-248 °C (Ethanol).

33 e) : 4-{2-[1-(2-(4-Chlorphenoxy) propyl) piperazin-4-yl] ethyl} benzoesaeure.
Ausb. 84 % d.Th., Schmp. 183 °C.
Dihydrochlorid : Schmp. 246-249 °C.

33 f) : 4-{2-[1-(2-(4-Methoxyphenoxy) propyl) piperazin-4-yl] ethyl} benzoesaeure.
Ausb. 69 % d.Th., Schmp. 128-130 °C.
Dihydrochlorid : Schmp. 246-249 °C.

33 g) : 4-{2-[1-(2-(4-Methylphenoxy) propyl) piperazin-4-yl] ethyl} benzoesaeure.
Ausb. 71 % d.Th. Hydrochlorid, Schmp. 147-148 °C (waessr. Ethanol).

33 h) : 4-{2-[1-(4-Chlorphenyl) piperazin-4-yl] ethyl} benzoesaeure.
Ausb. 87 % d.Th. Hydrochlorid-monohydrat.
Schmp. 275 °C (waessr. Ethanol).

33 i) : 4-{2-[1-(4-Chlorbenzoyl) piperazin-4-yl] ethyl} benzoesaeure.
Ausb. 80 % d.Th., Schmp. 85-87 °C.
Hydrochlorid : Schmp. 294-296 °C.

33 j) : 4-{2-[1-(4-Chlorcinnamoyl) piperazin-4-yl] ethyl} benzoesaeure.
Ausb. 84 % d.Th., Schmp. 243-244 °C.

33 k) : 4-{2-[1-(3.5-Di-tert. butyl-4-hydroxy-cinnamoyl) piperazin-4-yl] ethyl} benzoesaeure.
Ausb. 88 % d.Th., Schmp. 233 °C.

33 l) : 4-{2-[1-(3-(3.5-Di-tert. butyl-4-hydroxy-phenyl) propionyl) piperazin-4-yl] ethyl} benzoesaeure.
Ausb. 77 % d.Th., Schmp. 93-95 °C.
Hydrochlorid : Schmp. 230-231 °C.

33 m) : 4-{2-[1-(Methansulfonyl) piperazin-4-yl] ethyl} benzoesaeure.
Ausb. 71 % d.Th. Hydrochlorid, Schmp. 274-276 °C (waessr. Aceton).

33 n) : 4-{2-[1-(Benzolsulfonyl) piperazin-4-yl] ethyl} benzoesaeure.
Ausb. 96 % d.Th., Schmp. 266-267 °C.

33 o) : 4-{2-[1-(4-Chlor-benzolsulfonyl) piperazin-4-yl] ethyl} benzoesaeure.
Ausb. 71 % d.Th. Hydrochlorid, Schmp. 273-275 °C (Ethanol).

33 p) : 4-{2-[1-(4-Methyl-benzolsulfonyl) piperazin-4-yl] ethyl} benzoesaeure.
Ausb. 97 % d.Th. Hydrochlorid, Schmp. 298 °C.

33 q) : 4-{2-[1-(Acetophenon-2-sulfonyl) piperazin-4-yl] ethyl} benzoesaeure.
Ausb. 77 % d.Th. Hydrochlorid, Schmp. 232 °C (Ethanol).

33 r) : 4-{2-[1-(4-Phenoxyphenyl) piperazin-4-yl] ethyl} = benzoesaeure.
Ausbeute 88 % d.Th. Hydrochlorid, Schmp. 296 °C.

33 s) : 4-{2-[1-(4-Benzyloxyphenyl) piperazin-4-yl] ethyl} = benzoesaeure.
Ausbeute 87 % d.Th., Schmp. 234-235 °C.

33 t) : 4-{2-[1-(2-Methyl-3-phenyl-propionyl) piperazin-4-yl] = ethyl} benzoesaeure.
Ausbeute 94 % d.Th. Hydrochlorid, Schmp. 222.5-223 °C.

33 u) : 4-{2-[1-(3-(4-Chlorphenyl)-2-methyl-propyl) piperazin-4-yl] ethyl} benzoesaeure.
Ausbeute 88 % d.Th. Dihydrochlorid, Schmp. 277-278 °C (Wasser).

33 v) : 4-{2-[1-(3-Trifluormethyl-phenethyl) piperazin-4-yl] = ethyl} benzoesaeure.
Ausbeute 95 % d.Th. Sulfat, Schmp. 283-285 °C (60 proz. Essigsaeure).

33 w) : 4-{2-[1-(3.4-Dichlor-cinnamyl) piperazin-4-yl] ethyl} = benzoesaeure.
Ausbeute 76 % d.Th. Dihydrochlorid, Schmp. 295 °C (waessr. Ethanol).

## Beispiel 34

4-{2-[1-Phenethyl-piperazin-4-yl] ethyl} benzoesaeure.

Man haelt ein Gemisch aus 9.0 g (20.5 mmol) 4-{2-[1-Phenethyl-piperazin-4-yl] ethyl} benzoesaeureethylester-dihydrochlorid und 100 ml 6 N HCl 20 h unter Ruehren auf Rueckflusstemperatur. Dann wird abgekuehlt, abgesaugt und der Niederschlag mit kalter 6 N HCl gewaschen und getrocknet. Ausbeute 6.6 g Dihydrochlorid, Schmp. 317 °C (Z.).

Falls die Ausgangsester sehr schwer loeslich sind, empfiehlt sich die Zugabe von etwas Ethanol zum Hydrolysegemisch.

In Analogie zu diesem Beispiel wurden hergestellt :

34 a) : 4-{2-[1-(2-Hydroxypropyl) piperazin-4-yl] ethyl} benzoesaeure.
Ausbeute 87 % d.Th. Dihydrochlorid, Schmp. 289 °C (Ethanol + conc. HCl).

34 b) : 4-{2-[1-(4-Methoxybenzyl) piperazin-4-yl] ethyl} benzoesaeure.
Ausbeute 79 % d.Th. Dihydrochlorid, Schmp. 286 °C (waessr. Ethanol).

34 c) : 4-{2-[1-(4-Chlorcinnamyl) piperazin-4-yl] ethyl} benzoesaeure.
Ausbeute 71 % d.Th. Dihydrochlorid, Schmp. 288 °C (Wasser). Das Sulfat schmilzt bei 299 °C.

34 d) : 4-{2-[1-Methyl-piperazin-4-yl] ethyl} benzoesaeure.
Ausbeute 70 % d.Th. Dihydrochlorid, Schmp. 298 °C.

34 e) : 3-[1-(4-Carboxy-phenethyl) piperazin-4-yl] propansulfonsaeure.
Ausbeute 96 % d.Th. Hydrochlorid, Schmp. 298 °C.

34 f) : 4-[1-(4-Chlorcinnamyl) piperazin-4-yl-methyl] benzoesaeure.
Ausbeute 73 % d.Th. Dihydrochlorid, Schmp. 249-250 °C (waessr. Ethanol).

34 g) : 4-[2-(Piperazin-1-yl) ethyl] benzoesaeure.
Ausbeute 87 % d.Th. Dihydrochlorid, Schmp. 285-287 °C (2 N Salzsaeure).

34 h) : 4-[2-(1-Benzyl-piperazin-4-yl) ethyl] benzoesaeure.
Ausbeute 84 % d.Th. Dihydrochlorid, Schmp. 272-274 °C (2 N Salzsaeure).

34 i) : 4-{2-[1-(2-Chlorbenzyl) piperazin-4-yl] ethyl} = benzoesaeure.
Ausbeute 87 % d.Th. Dihydrochlorid, Schmp. 287-290 °C (waessr. Ethanol).

34 j) : 4-{2-[1-(4-Fluorbenzyl) piperazin-4-yl] ethyl} = benzoesaeure.
Ausbeute 82 % d.Th. Dihydrochlorid, Schmp. 311 °C (2 N Salzsaeure).

34 k) : 4-{2-[1-(3-Chlorbenzyl) piperazin-4-yl] ethyl} = benzoesaeure.
Ausbeute 87 % d.Th. Dihydrochlorid, Schmp. 294 °C (waessr. Aceton).

34 l) : 4-{2-[1-(3-Trifluormethyl-phenyl) piperazin-4-yl] = ethyl} benzoesaeure.
Ausbeute 73 % d.Th. Dihydrochlorid, Schmp. 242-243 °C (waessr. Ethanol).

34 m) : 4-{2-[1-(2-Chlorcinnamyl) piperazin-4-yl] ethyl} = benzoesaeure.
Ausbeute 85 % d.Th. Dihydrochlorid, Schmp. 280-281 °C (verd. Salzsaeure).

34 n) : 4-{2-[1-(3-Bromcinnamyl) piperazin-4-yl] ethyl} = benzoesaeure.
Ausbeute 78 % d.Th. Dihydrochlorid, Schmp. 282-283 °C (verd. Salzsaeure).

34 o) : 4-{2-[1-(4-Nitrocinnamyl) piperazin-4-yl] ethyl} = benzoesaeure.
Ausbeute 85 % d.Th. Dihydrochlorid, Schmp. 273-274 °C (verd. Salzsaeure). Lichtempfindlich.

34 p) : 4-{2-[1-(3-Methoxycinnamyl) piperazin-4-yl] ethyl} = benzoesaeure.
Ausbeute 72 % d.Th. Dihydrochlorid, Schmp. > 254 °C (verd. Salzsaeure).

34 q) : 4-{2-[1-(3-Trifluormethyl-cinnamyl) piperazin-4-yl] = ethyl} benzoesaeure.
Ausbeute 79 % d.Th. Dihydrochlorid, Schmp. 281-282 °C (verd. Salzsaeure).

34 r) : 4-{2-[1-(3-Trifluormethyl-benzyl) piperazin-4-yl] = ethyl} benzoesaeure.
Ausbeute 96 % d.Th. Dihydrochlorid, Schmp. 262-264 °C (waessr. Aceton).

34 s) : 1-(4-Carboxyphenethyl)-4-[2-(4-nitrophenoxy) propyl] = piperazin.
Ausbeute 66 % d.Th. Dihydrochlorid, Schmp. 274-276 °C (waessr. Ethanol).

34 t) : 1-(4-Carboxyphenethyl)-4-[2-(4-aminophenoxy) propyl] = piperazin.
Ausbeute 82 % d.Th. Dihydrochlorid, Schmp. 254-255 °C (waessr. Ethanol).

34 u) : 4-{2-[1-(2-Methyl-3-phenyl-propyl) piperazin-4-yl] = ethyl} benzoesaeure.
Ausbeute 81 % d.Th. Dihydrochlorid, Schmp. 261-263 °C (verd. Salzsaeure).

34 v) : 4-{3-[1-(4-Chlorcinnamyl) piperazin-4-yl] propyl} = benzoesaeure.
Ausbeute 78 % d.Th. Dihydrochlorid, Schmp. 276-277 °C (verd. Salzsaeure).

34 w) : 4-[3-(Piperazin-1-yl) propyl] benzoesaeure.
Ausbeute 86 % d.Th. Dihydrochlorid, Schmp. 264-265 °C (verd. Salzsaeure).

34 x) : 4-[3-(1-Benzyl-piperazin-4-yl) propyl] benzoesaeure.
Ausbeute 83 % d.Th. Dihydrochlorid, Schmp. 275-276 °C (verd. Salzsaeure).

34 y) : 4-[1-(4-Chlorcinnamyl) piperazin-4-yl] phenylessigsaeure.
Ausbeute 76 % d.Th. Hydrochlorid, Schmp. 219-220 °C (Essigsaeure).

34 z) : 4-(Piperazin-1-yl) benzoesaeure.
Ausbeute 82 % d.Th. Hydrochlorid, Schmp. 322 °C (Wasser).

## Beispiel 35

4-(Piperazin-1-yl) phenylessigsaeure-ethylester

Man erhitzt ein Gemisch aus 100 g (0.568 mol) p-Amino-phenylessigsaeure, 99.6 g (0.568 mol) Bis-(2-chlorethyl) aminhydrochlorid und 350 ml n-Butanol unter Ruehren 120 h auf Rueckflusstemperatur. Dann gibt man 39.2 g (0.284 mol) Kaliumcarbonat zu und erhitzt weitere 145 h auf Rueckflusstemperatur. Anschliessend wird heiss vom Unloeslichen abgesaugt und das Filtrat eingedampft. Den Rueckstand nimmt man in Wasser auf, stellt mit 10 N Natronlauge stark alkalisch und extrahiert die organischen Bestandteile mit Ether. Die Etherextrakte werden eingeengt und der Rueckstand in Ethanol geloest. Aus dieser Loesung wird mit etwa 40 ml conc. Schwefelsaeure 4-(Piperazin-1-yl) phenylessigsaeure-ethylester als Sulfat gefaellt. Ausbeute 120 g (61 % d.Th.), Fp. 194-197 °C.

In analoger Weise stellt man unter Einsatz von 4-Aminophenyl-essigsaeure-n-butylester her :
4-(Piperazin-1-yl) phenylessigsaeure-n-butylester.
Ausbeute 52 % d.Th. Sulfat, Schmp. 197-199 °C.

Beispiel 36

In Analogie zu Beispiel 23 stellt man durch Umsetzen von 4-(Piperazin-1-yl) phenylessigsaeure-ethylester mit den entsprechenden Alkylhalogeniden in Butanon-2 in Gegenwart von wasserfreiem Kaliumcarbonat folgende Verbindungen her :

36 a) mit 1-n-Hexadecylbromid : 4-[1-(1-n-Hexadecyl) piperazin-4-yl] phenylessigsaeure-ethylester. Ausbeute 79 % d.Th., aus Isopropanol-Wasser-Gemisch amorphes Pulver.

36 b) mit 4-Chlor-benzylchlorid : 4-[1-(4-Chlorbenzyl) piperazin-4-yl] phenylessigsaeure-ethylester. Ausbeute 92 % d.Th. viskoses Oel.

36 c) mit 2-Phenoxypropylbromid : 4-[1-(2-Phenoxypropyl) piperazin-4-yl] phenylessigsaeure-ethylester.
Ausbeute 70 % d.Th. Dihydrochlorid, Schmp. 153-154 °C.

Beispiel 37

4-[1-(3.5-Di-tert. butyl-4-hydroxy-phenethyl) piperazin-4-yl]-phenylessigsaeure-n-butylester.
Man haelt ein Gemisch aus 20.2 g (50 mmol) Tosylat des 3.5-Di-tert. butyl-4-hydroxy-phenethylalkohols, 13.8 g (50 mmol) 4-(Piperazin-1-yl) phenylessigsaeure-n-butylester, 6,9 g (50 mmol) pulv. $K_2CO_3$ sicc. und 200 ml Butanon-2 24 h auf Rueckflusstemperatur, saugt dann heiss ab und dampft das Filtrat i. Vak. ein. Den Rueckstand nimmt man in Ether auf, die Etherphase wird zweimal mit Wasser extrahiert, dann getrocknet ($Na_2SO_4$) und eingedampft. Man bringt mittels Ligroin zur Kristallisation und kristallisiert aus n-Butanol um.
Ausbeute 14.3 g (59 % d.Th.), Schmp. 103-105 °C.

Beispiel 38

Durch Umsetzen von 4-(Piperazin-1-yl) phenylessigsaeure-ethylester bzw. -n-butylester in Pyridin in Analogie zu Beispiel 17 erhaelt man folgende Verbindungen :

38 a) mit 4-Chlor-benzoylchlorid : 4-[1-(4-Chlorbenzoyl) piperazin-4-yl] phenylessigsaeure-n-butylester.
Ausbeute 79 % d.Th. Hydrochlorid, Schmp. 202-204 °C (n-Butanol).

38 b) mit 4-Chlor-cinnamoylchlorid : 4-[1-(4-Chlor-cinnamoyl) piperazin-4-yl] phenylessigsaeure-ethylester.
Ausbeute 69 % d.Th., Schmp. 137-139 °C.
Hydrochlorid : Schmp. 203-206 °C.

Beispiel 39

4-[1-(4-Chlorphenyl) piperazin-4-yl] phenylessigsaeure-n-butylester

erhaelt man in Analogie zu Beispiel 35 durch Umsetzen von 4-Chlor-N.N-bis-(2-chlorethyl) amin mit 4-Aminophenylessigsaeure-n-butylester in n-Butanol in Gegenwart von Kaliumcarbonat. Nach dem Absaugen von $K_2CO_3$ und KCl in der Hitze faellt aus dem Filtrat das Produkt aus. Ausbeute 66 % d.Th., Schmp. 99-100 °C (n-Butanol).

Beispiel 40

4-[1-(Benzolsulfonyl) piperazin-4-yl] phenylessigsaeure-ethylester

erhaelt man durch Umsetzen von 4-(Piperazin-1-yl) phenyl-essigsaeure-ethylester mit Benzolsulfochlorid in Analogie zu Beispiel 30. Ausbeute 85 % d.Th., Schmp. 160-163 °C.

Beispiel 41

4-[2-(Piperazin-1-yl) ethyl] phenylessigsaeure

a) Ein Gemisch aus 17.6 g (0.1 mol) 1-Benzylpiperazin, 100 ml abs. THF, 22.8 g (0.1 mol) 4'-(2-Bromethyl) acetophenon und 21 g (0.21 mol) Triethylamin wird 8 h auf Rueckfluss gehalten, dann kuehlt man ab und saugt ausgefallenes Hydrobromid ab. Man gibt abs. Ether zu, saugt nachgefaelltes Hydrobromid ab und versetzt nun das Filtrat unter Kuehlen mit einer ausreichenden Menge HCl-haltigem

Ether. Das ausfallende Hydrochlorid wird abgesaugt, mit Ether gewaschen und getrocknet. Ausbeute 32.5 g (82 % d.Th.) 1-(4-Acetylphenethyl)-4-benzyl-piperazin-dihydrochlorid, Schmp. 253-255 °C.

Dieses Ausgangsmaterial laesst sich auch in folgender Weise darstellen :

Man erhitzt ein Gemisch aus 22.7 g (0.1 mol) 4'-(2-Bromethyl) acetophenon, 50 ml Ethanol und 17.6 g (0.1 mol) 1-Benzylpiperazin 16 h auf Rueckflusstemperatur, gibt 10.0 g pulv. $Na_2CO_3$ zu und erhitzt weitere 8 h. Dann wird eingedampft und der Rueckstand in verd. HCl geloest. Man ethert aus und macht die waessr. Phase mit 10 proz. NaOH alkalisch. Nun wird wieder ausgeethert, die Etherphase mit KOH getrocknet, und schliesslich faellt man durch Zugabe von HCl-haltigem Ether das Dihydrochlorid des 1-(4-Acetyl-phenethyl)-4-benzyl-piperazins aus. Nach Waschen mit Ether und Trocknen 32.4 g (82 % d.Th.), Schmp. 249-253 °C.

b) Man haelt ein Gemisch aus 20 g (50 mmol) 1-(4-Acetyl-phenethyl)-4-benzyl-piperazin, 3.2 g (0.1 mol) Schwefel und 30 ml (0.35 mol) Morpholin 20 h bei 135 °C, kuehlt dann ab, giesst in Wasser und ethert aus. Die Etherphase wird dreimal mit Wasser gewaschen, mit $MgSO_4$ getrocknet, mit Aktivkohle behandelt und abgesaugt. Das Filtrat versetzt man mit einer ausreichenden Menge HCl-haltigem Ether. Dann wird abgesaugt, mit Ether gewaschen und getrocknet. Ausbeute 13.9 g (44 % d.Th.) 4-[2-(1-Benzylpiperazin-4-yl) ethyl]-phenylessigsaeurethiomorpholid als Dihydrochlorid, Schmp. 235-237 °C.

c) Ein Gemisch aus 10.0 g (20 mmol) Thiomorpholid. 2 HCl und 100 ml 6 N HCl wird 5 h gekocht, dann auf ein halbes Volumen eingeengt und abgekuehlt. Man saugt ab, waescht mit wenig kalter 2 N HCl und kristallisiert aus Wasser um.

Ausbeute 5.1 g (61 % d.Th.) Dihydrochlorid der 4-[2-(1-Benzyl-piperazin-4-yl) ethyl] phenylessigsaeure mit dem Schmp. 244-245 °C (Z.).

d) Man loest 140 g (0.34 mol) Dihydrochlorid der 4-[2-(1-Benzyl-piperazin-4-yl) ethyl] phenylessigsaeure in einem Gemisch aus Ethanol und 6 N HCl, gibt 10 proz. Palladiumkohle zu und hydriert in der Schuettelente. Wenn die Reaktion zu traege verlaeuft, ist der Katalysator durch neuen zu ersetzen. Man saugt dann ab, dampft ein und bringt mit etwas Essigester zur Kristallisation. Ausbeute 90 g (82 % d.Th.) Dihydrochlorid der 4-[2-(Piperazin-1-yl) ethyl]-phenylessigsaeure mit dem Schmp. 238-239 °C (Aceton + Wasser).

## Beispiel 42

4-[2-(Piperazin-1-yl) ethyl] phenylessigsaeureethylester

Man begast eine Loesung aus 83.5 g (0.26 mol) 4-[2-(Piperazin-1-yl) ethyl] phenylessigsaeure und 850 ml abs. Ethanol von der Oberflaeche her mit Chlorwasserstoff bis zur Saettigung, laesst ueber Nacht stehen, dampft dann auf das halbe Volumen ein und kuehlt im Eisbad. Man saugt das ausgefallene Dihydrochlorid ab, waescht mit etwas Ether und trocknet. Ausbeute 85.1 g (94 % d.Th.), Schmp. 258-260 °C (Z.).

In analoger Weise erhaelt man

42 a) : 4-{2-[1-(2-Chlorbenzyl) piperazin-4-yl] ethyl} = benzoesaeure-n-butylester
aus 4-{2-[1-(2-Chlorbenzyl) piperazin-4-yl] ethyl} = benzoesaeure und n-Butanol. Ausbeute 87 % d.Th. Dihydrochlorid, Schmp. 227-230 °C (n-Butanol) und

42 b) : 4-{2-[1-(4-Chlorcinnamyl) piperazin-4-yl] ethyl} = benzoesaeure-methylester
aus 4-{2-[1-(4-Chlorcinnamyl) piperazin-4-yl] ethyl} = benzoesaeure und Methanol. Ausbeute 82 % d.Th. Di-hydrogensulfat, Schmp. 194-195 °C (Butanon-2-Methanol-Gemisch).

## Beispiel 43

4-{2-[1-(n-Hexadecyl) piperazin-4-yl] ethyl} phenylessigsaeure-ethylester

Man haelt ein Gemisch aus 0.1 mol 4-[2-(Piperazin-1-yl) ethyl]-phenylessigsaeureethylester. 2 HCl, 250 ml Butanon-2, 0.2 mol pulv. $K_2CO_3$ sicc. und 0.11 mol 1-n-Hexadecylbromid 16 h lang auf Rueckflusstemperatur, saugt dann ab und waescht das Kaliumcarbonat/Kaliumhalogenid-Gemisch mit warmen Aceton. Die vereinigten organischen Phasen werden eingedampft. Der Rueckstand wird umkristallisiert. Zur Herstellung des Dihydrochlorids loest man den Eindampfrueckstand in Ether, filtriert (wenn noetig) und gibt nun unter Kuehlen eine ausreichende Menge HCl-haltigen Ether zu. Das Hydrochlorid wird abgesaugt, mit etwas Ether gewaschen und getrocknet, dann aus Ethanol, dem 2 Tropfen conc. HCl beigefuegt wurden, umkristallisiert.

Ausbeute 60 % d.Th. Dihydrochlorid, Schmp. 282 °C.

In analoger Weise werden aus 4-[2-(Piperazin-1-yl) ethyl]-phenylessigsaeure-ethylester die folgenden Produkte dargestellt :

43 a) mit 4-Chlor-benzylchlorid : 4-{2-[1-(4-Chlorbenzyl) piperazin-4-yl] ethyl} phenylessigsaeure-ethylester
Reaktionszeit : 1 Woche ; Ausbeute 91 % d.Th. Dihydrochlorid, Schmp. 272-275 °C

43 b) mit Phenethylbromid : 4-[2-(1-Phenethyl-piperazin-4-yl) ethyl] phenylessigsaeure-ethylester.
Reaktionszeit : 4 Tage ; Ausbeute 80 % d.Th. Dihydrochlorid, Schmp. 264-266 °C.

43 c) mit 3-Phenyl-propylbromid : 4-{2-[1-(3-Phenyl-propyl) piperazin-4-yl] ethyl} phenylessigsaeure-ethylester.
Reaktionszeit : 40 Stunden ; Ausbeute 66 % d.Th. Base (viskoses Oel).

43 d) mit 4-Chlor-cinnamylchlorid : 4-{2-[1-(4-Chlorcinnamyl) piperazin-4-yl] ethyl} phenylessigsaeure-ethylester
Reaktionszeit : 40 Stunden ; Ausbeute 58 % d. Th.
Dihydrochlorid, Schmp. 282 °C. Die freie Base schmilzt bei 69 °C (Ligroin)

43 e) mit 4-Methoxy-benzylchlorid : 4-{2-[1-(4-Methoxybenzyl) piperazin-4-yl] ethyl} phenyl-essigsaeure-ethylester
Reaktionszeit : 72 Stunden ; Ausbeute 76 % d. Th.
Hydrochlorid, Schmp. 249-251 °C (Ethanol + Ligroin)

43 f) mit 2-Phenoxy-ethylbromid : 4-{2-[1-(2-Phenoxy-ethyl) piperazin-4-yl] ethyl} phenylessigsauere-ethylester
Reaktionszeit : 240 Stunden ; Ausbeute 87 % d. Th.
Hydrochlorid, Schmp. 238-240 °C

## Beispiel 44

4-{2-[1-(3.5-Di-tert. butyl-4-hydroxyphenethyl) piperazin-4-yl]-ethyl} phenylessigsaeure-ethylester

Man erhitzt ein Gemisch aus 13.9 g (50.2 mmol) p-Toluol-sulfonsaeure-(3.5-di-tert. butyl-4-hydroxyphenethyl) ester, 18.4 g (45.6 mmol) 4-[2-(Piperazin-1-yl)ethyl] phenylessigsauere-ethylester, 6.3 g Kaliumcarbonat und 300 ml Butanon-2 8 h unter Ruehren auf Rueckflusstemperatur. Zum Schutz gegen Oxidation wird unter Stickstoff gearbeitet. Nach dem Erkalten wird der anorganische Niederschlag abgesaugt, das Filtrat eingeengt und der Eindampfrueckstand in Ether aufgenommen. Aus der Etherloesung wird mit HCl-haltigem Ether das Hydrochlorid gefaellt. Man erhaelt nach Umkristallisieren aus Ethanol 16.4 g (62 %)
Hydrochlorid, Schmp. 238-240 °C.

## Beispiel 45

4-{2-[1-(2-Hydroxypropyl) piperazin-4-yl] ethyl} phenylessigsaeure-ethylester

stellt man in Analogie zu Beispiel 11 durch Umsetzen von 4-[2-(Piperazin-1-yl ) ethyl] phenylessigsaeure-ethylester mit Propylenoxid dar. Ausbeute 92 % d. Th., farbloses Oel.

## Beispiel 46

4-{2-[1-(4-Chlorphenyl) piperazin-4-yl] ethyl} phenylessigsaeure

a) Durch Umsetzen von 1-(4-Chlorphenyl) piperazin mit 4'-(2-Bromethyl) acetophenon in Analogie zu Beispiel 41, Abschnitt a) erhaelt man 4'-{2-[1-(4-Chlorphenyl)-piperazin-4-yl] ethyl} acetophenon. Ausbeute 64 % d. Th., Schmp. 141-143 °C (Essigester).

b) Durch Umsetzen von 4'-{2-[1-(4-Chlorphenyl) piperazin-4-yl] ethyl} acetophenon mit Morpholin und Schwefel in Analogie zu Beispiel 41, Abschnitt b) erhaelt man 4-{2-[1-(4-Chlorphenyl) piperazin-4-yl] ethyl} phenylessigsaeurethiomorpholid als Hydrochlorid. Ausbeute 81 % d. Th., Schmp. 144-147 °C.

c) Durch Verseifen von 4-{2-[1-(4-Chlorphenyl) piperazin-4-yl] ethyl} phenylessigsauere-thiomorpholid mittels 6 N HCl (16 h Rueckflusstemperatur) erhaelt man in Analogie zu Beispiel 41, Abschnitt c) in 64 % Ausbeute das Hydrochlorid der 4-{2-[1-(4-Chlorphenyl) piperazin-4-yl] ethyl}-phenylessigsauere, Schmp. 241-243 °C.

Beispiel 47

Durch Acylierung mittels Saeurechlorid bzw. durch Sulfonierung mit Sulfonsaeurechlorid gemaess nachfolgender Vorschrift erhaelt man die aufgefuehrten Verbindungen :

Zu einer Loesung aus 10.47 g (30 mmol) 4-[2-(Piperazin-1-yl)-ethyl] phenylessigsaeure-ethylester. 2 HCl und 75 ml abs. Pyridin gibt man bei 5-10 °C unter Ruehren 30 mmol Acylchlorid bzw. Sulfonylchlorid und laesst ueber Nach bei 20 °C ruehren. Dann giesst man in Eiswasser, saugt den ausgefallenen Niederschlag ab und waescht mit Bicarbonatloesung und Wasser. Nach dem Trocknen wird umkristallisiert.

47 a) mit 4-Chlorcinnamoylchlorid : 4-{2-[1-(4-Chlorcinnamoyl) piperazin-4-yl] ethyl} phenylessigsaeure-ethylester
Ausbeute 68 % d. Th., Schmp. 123-124 °C (Essigester)

47 b) mit 4-Chlor-benzolsulfochlorid : 4-{2-[1-(4-Chlor-benzolsulfonyl) piperazin-4-yl] ethyl}-phenylessigsaeure-ethylester
Ausbeute 71 % d. Th., Schmp. 231-233 °C (Ethanol)

47 c) mit 4-Chlor-benzoylchlorid : 4-{2-[1-(4-Chlor-benzoyl) piperazin-4-yl] ethyl} phenyl-essigsaeure-ethylester
Ausbeute 81 % d. Th., Schmp. 126-128 °C

47 d) mit Benzolsulfochlorid : 4-[2-(1-Benzolsulfonyl-piperazin-4-yl) ethyl] phenylessigsaeure-ethylester
Ausbeute 70 % d. Th., Schmp. 125-127 °C.

Beispiel 48

4-{2-[1-(3.5-Di-tert. butyl-4-hydroxy-cinnamoyl) piperazin-4-yl]-ethyl} phenylessigsaeure-ethylester

Eine Loesung aus 14.0 g (51 mmol) 3.5-Di-tert. butyl-4-hydroxyzimtsaeure und 17.7 g (51 mmol) 4-[2-(Piperazin-1-yl) ethyl]-phenylessigsaeure-ethylester-dihydrochlorid in 150 ml abs. Pyridin wird unter Eiskuehlung tropfenweise mit einer Loesung aus 3.57 g (26 mmol) Phosphortribromid in 20 ml Pyridin versetzt. Man ruehrt noch 2 h unter Eiskuehlung nach und laesst 12 h bei Zimmertemperatur stehen. Dann giesst man auf Eis und extrahiert mit Essigester. Die Extrakte werden getrocknet und eingedampft. Der Rueckstand wird in Ether aufgenommen und mit HCl-haltigem Ether das Hydrochlorid gefaellt. Man erhaelt nach Umkristallisieren aus einem Gemisch von Ethanol und Wasser, dem man etwas conc. Salzsaeure zugesetzt hat, 14.5 g (47 %) Hydrochlorid mit dem Schmp. 111-113 °C.

In analoger Weise erhaelt man aus 3-(3.5-Di-tert. butyl-4-hydroxyphenyl) propionsaeure und 4-[2-(Piperazin-1-yl) ethyl] phenylessigsaeure-ethylester die Verbindung

48 a) : 4-{2-[1-(3-(3.5-Di-tert. butyl-4-hydroxyphenyl) propionyl) piperazin-4-yl] ethyl} phenylessigsaeure-ethylester
Ausbeute 61 % d. Th., Schmp. 116-117 °C (Essigester) und aus 3.5-Di-tert. butyl-4-hydroxy-zimtsaeure und 4-(Piperazin-1-yl) phenylessigsaeure-methylester die Verbindung

48 b) : 4-[1-(3.5-Di-tert. butyl-4-hydroxycinnamoyl) piperazin-4-yl] phenylessigsaeure-methylester
Ausbeute 54 % d. Th. Hydrochlorid, Schmp. 177-178 °C (Methanol).

Beispiel 49

4-{2-[1-(n-Hexadecyl) piperazin-4-yl] ethyl} phenylessigsaeure

Ein Gemisch aus 0.1 mol des Ethylesters (als Dihydrochlorid), 200 ml 6 N HCl und 100 ml Dioxan wird 3 h bei 80 °C gehalten, dann stark abgekuehlt. Man saugt die ausgefallenen Kristalle ab und kristallisiert sie aus waessrigem Ethanol um. Ausbeute 91 % d. Th. Dihydrochlorid mit dem Schmp. 251 °C.

In analoger Weise erhaelt man aus den entsprechenden Phenylessigsaeure-ethylestern (bzw. deren Hydrochloriden) die folgenden Carbonsaeuren :

49 a) : 4-{2-[1-(4-Chlorbenzyl) piperazin-4-yl] ethyl} phenylessigsaeure
Reaktionszeit : 10 Stunden (ohne Dioxanzusatz).
Ausbeute 65 % d. Th. Dihydrochlorid, Schmp. 280-282 °C (Eisessig)

49 b) : 4-[2-(1-Phenethyl-piperazin-4-yl) ethyl] phenylessigsaeure
Reaktionszeit : 10 Stunden (ohne Dioxanzusatz)
Ausbeute 54 % d. Th. Dihydrochlorid, Schmp. 285-286 °C (Eisessig)

49 c) : 4-{2-[1-(3-Phenyl-propyl) piperazin-4-yl] ethyl} phenyl-essigsaeure
Reaktionszeit : 6 Stunden (ohne Dioxanzusatz)
Ausbeute 62 % d. Th. Dihydrochlorid, Schmp. 248-251 °C (Eisessig)

49 d) : 4-{2-[1-(4-Chlor-cinnamyl) piperazin-4-yl] ethyl} phenylessigsaeure
Reaktionszeit : 5 Stunden (mit Dioxanzusatz)
Ausbeute 69 % d. Th. Dihydrochlorid, Schmp. 273-274 °C (Aceton + 2 N HCl)

49 e) : 4-{2-[1-(4-Chlor-benzolsulfonyl) piperazin-4-yl] ethyl}-phenylessigsaeure
Reaktionszeit : 35 Stunden (ohne Dioxanzusatz)
Ausbeute 68 % d. Th. Hydrochlorid, Schmp. 247-249 °C (Eisessig).

Beispiel 50

Als Alternative zu Beispiel 49 kann die Verseifung der Carbonester auch z. B. in alkalihaltigem Methanol vorgenommen werden :

Man loest 50 mmol Carbonsaeureester in 200 ml Methanol und versetzt die Loesung unter Ruehren mit 100 ml 1 N Kalilauge. Bei Hydrochloriden setzt man zusaetzlich aequivalente Mengen Kalilauge zu. Dann erhitzt man solange auf 40 °C, bis im DC kein Ausgangsmaterial mehr zu erkennen ist. Man dampft nun das Methanol ab und neutralisiert mit einer aequivalenten Menge Salzsaeure. Wenn die Aminosaeuren nicht ausfallen, wird mit HCl sauer gestellt, zur Trockne eingedampft und das Hydrochlorid durch Ausruehren mit Ethanol vom anorganischen Rueckstand abgetrennt. Oxidationsempfindliche Ester werden unter Stickstoff verseift.

Analog werden dargestellt :

50 a) : 4-{2-[1-(2-Hydroxypropyl) piperazin-4-yl] ethyl} phenylessigsaeure
Ausbeute 53 % d. Th., Schmp. 176-178 °C,
Dihydrochlorid-monohydrat : Schmp. 224-227 °C

50 b) : 4-{2-[1-(4-Methoxybenzyl) piperazin-4-yl] ethyl} phenylessigsaeure
Ausbeute 64 % d. Th., Schmp. 175-177 °C,
Dihydrochlorid : Schmp. 280-282 °C

50 c) : 4-{2-[1-(3.5-Di-tert. butyl-4-hydroxy-phenethyl) piperazin-4-yl] ethyl} phenylessigsaeure
Ausbeute 60 % d. Th., Schmp. 210-213 °C,
Dihydrochlorid-monohydrat : Schmp. 257-260 °C

50 d) : 4-{2-[1-(2-Phenoxy-ethyl) piperazin-4-yl] ethyl} phenylessigsaeure
Ausbeute 81 % d. Th., Schmp. 143-145 °C,
Dihydrochlorid : Schmp. 214-217 °C

50 e) : 4-{2-[1-(4-Chlor-benzoyl) piperazin-4-yl] ethyl} phenylessigsaeure
Ausbeute 69 % d. Th. Hydrochlorid, Schmp. 228-230 °C

50 f) : 4-{2-[1-(3.5-Di-tert. butyl-4-hydroxy-cinnamoyl) piperazin-4-yl] ethyl} phenylessigsaeure
Ausbeute 59 % d. Th. Hydrochlorid-monohydrat, Schmp. 164-167 °C

50 g) : 4-{2-[1-(Benzolsulfonyl) piperazin-4-yl] ethyl} phenylessigsaeure
Ausbeute 85 % d. Th., Schmp. 184-186 °C,
Hydrochlorid : Schmp. 282-284 °C und folgende 4-(Piperazin-1-yl) phenylessigsaeuren :

50 h) : 4-[1-(n-Hexadecyl) piperazin-4-yl] phenylessigsaeure
Ausbeute 93 % d. Th., Schmp. 128-130 °C

50 i) : 4-[1-(4-Chlorbenzyl) piperazin-4-yl] phenylessigsaeure
Ausbeute 63 % d. Th., Schmp. 183 °C (i-Propanol)

50 j) : 4-[1-(3.5-Di-tert. butyl-4-hydroxyphenethyl) piperazin-4-yl] phenylessigsaeure
Ausbeute 71 % d. Th. Hydrochlorid, Schmp. 244-245 °C (66 proz. Ethanol)

50 k) : 4-[1-(2-Phenoxypropyl) piperazin-4-yl] phenylessigsaeure
Ausbeute 64 % d. Th., Schmp. 156-158 °C

50 l) : 4-[1-(4-Chlorbenzoyl) piperazin-4-yl] phenylessigsaeure
Ausbeute 77 % d. Th., Schmp. 146-147 °C (66 proz. Ethanol)

50 m) : 4-[1-(4-Chlorcinnamoyl) piperazin-4-yl] phenylessigsaeure
Ausbeute 69 % d.Th., Schmp. 228-230 °C (waessr. Ethanol)

50 n) : 4-[1-(4-Chlorphenyl) piperazin-4-yl] phenylessigsaeure
Ausbeute 81 % d. Th., Schmp. 210-212 °C

50 o) : 4-[1-(Benzolsulfonyl) piperazin-4-yl] phenylessigsaeure
Ausbeute 70 % d. Th., Schmp. 128-130 °C.

50 p) : 4-[2-(1-Benzyl-piperazin-4-yl) propyl] benzoesaeure
Ausbeute 75 % d. Th. Dihydrochlorid, Schmp. 277 °C (Ethanol + 4 N HCl).

Beispiel 51

3-[4-(Piperazin-1-yl) phenyl] propionsaeure-n-butylester

Man haelt ein Gemisch aus 175 g (0.79 mol) 3-(4-Aminophenyl)-propionsaeure-n-butylester (durch Hydrieren von 4-Nitrozimtsaeure-n-butylester in Gegenwart von Palladiumkohle in Butanol hergestellt, Sdp. 137-138 °C/0.013 mbar), 141.3 g (0.79 mol) Bis-(2-chlorethyl) amin-hydrochlorid und 600 ml n-Butanol 48 h auf Rueckflusstemperatur, gibt dann 54.5 g (0.39 mol) pulv. Kaliumcarbonat zu und laesst weitere 120 h in der Siedehitze reagieren. Danach wird heiss abgesaugt, das Filtrat i. Vak. eingedampft und das zurueckbleibende Oel in n-Butanol aufgenommen. In der Kaelte wird nun mittels conc. $H_2SO_4$ das Sulfat ausgefaellt. Man saugt ab, verreibt mit Ether und trocknet. 286.8 g (69 % d. Th.), Schmp. 183-185 °C.

Beispiel 52

3-{4-[1-(2-Phenoxypropyl) piperazin-4-yl] phenyl} propionsaeuren-butylester

Man ruehrt ein Gemisch aus 21.4 g (74 mmol) 3-[4-(Piperazin-1-yl) phenyl] propionsaeure-n-butylester, 150 ml HMPT und 15.8 g (74 mmol) 2-Phenoxy-propylbromid 5 h bei 120 °C, giesst dann auf Eiswasser und extrahiert mit Essigester. Der Extrakt wird eingedampft und der Eindampfrueckstand in Ether geloest. Man trocknet ($MgSO_4$) und faellt mit HCl-haltigem Ether das Hydrochlorid aus. Nach Absaugen, Waschen mit Ether und Trocknen 15.1 g (44 % d. Th.), Schmp. 180-183 °C.

In analoger Weise stellt man aus 3-[4-(Piperazin-1-yl) phenyl]-propionsaeure-n-butylester her :

52 a) mit n-Hexadecylbromid : 3-[4-(1-n-Hexadecyl-piperazin-4-yl) phenyl] propionsaeure-n-butylester
Ausbeute 63 % d. Th. Hydrochlorid, Schmp. 168-170 °C.

Beispiel 53

3-{4-[1-(4-Chlorbenzyl) piperazin-4-yl] phenyl} propionsaeure-n-butylester

Man erhitzt ein Gemisch aus 20.0 g (68.8 mmol) 3-[4-(Piperazin-1-yl) phenyl] propionsaeure-n-butylester, 200 ml Acetonitril, 10.4 g (75.6 mmol) pulv. Kaliumcarbonat und 12.2 g (75.6 mmol) 4-Chlorbenzylchlorid 36 h auf Rueckflusstemperatur, saugt ab und dampft das Filtrat ein. Der Rueckstand wird in Ether aufgenommen und mittels HCl-haltigem Ether das Hydrochlorid ausgefaellt. Man saugt ab, kristallisiert aus Isopropanol um und trocknet. Ausbeute 29.0 g (87 % d. Th.) Hydrochlorid, Schmp. 222-225 °C.

Beispiel 54

3-{4-[1-(4-Chlorphenyl) piperazin-4-yl] phenyl} propionsaeure-n-butylester

Man haelt ein Gemisch aus 12.3 g (55 mmol) 3-(4-Aminophenyl)-propionsaeure-n-butylester, 16.0 g (55 mmol) N.N-Bis-(2-chlorethyl)-4-chloranilin und 50 ml n-Butanol 48 h auf Rueckflusstemperatur, gibt dann 3.8 g (27 mmol) pulv. $K_2CO_3$ sicc. zu und ruehrt nun 1 Woche lang bei Rueckflusstemperatur. Danach wird heiss abgesaugt, das Filtrat i. Vak. eingedampft, der Rueckstand mit Ligroin verruehrt und abgesaugt. Man nimmt nun in Ether auf, filtriert und versetzt mit HCl-haltigem Ether. Da das Hydrochlorid schlecht absaugbar ist, dampft man i. Vak. ein und kristallisiert aus n-Butanol um. 9.5 g (39 % d. Th.) Hydrochlorid, Schmp. 179-183 °C (Z.).

Beispiel 55

3-{4-[1-(4-Chlorcinnamoyl) piperazin-4-yl] phenyl} propionsaeure-n-butylester

Zu einer Loesung aus 15.8 g (54 mmol) 3-[4-(Piperazin-1-yl)-phenyl] propionsaeure-n-butylester, 110 ml $CH_2Cl_2$ und 8.2 g (82 mmol) Triethylamin tropft man bei 0 bis 5 °C eine Loesung aus 11.0 g (54 mmol) 4-Chlor-cinnamoylchlorid und 50 ml $CH_2Cl_2$. Anschliessend wird 3 h bei 20 °C geruehrt, dann schuettelt man zweimal mit Wasser aus, trocknet mittels $MgSO_4$ und dampft ein. Nach Umkristallisieren aus Butanol 14.8 g (60 % d. Th.), Schmp. 132-133 °C, Schmp. des Hydrochlorids 204-207 °C. Die Substanz ist lichtempfindlich.

In analoger Weise stellt man dar :

55 a) : 4-{2-[1-(2-Methyl-3-phenyl-propionyl) piperazin-4-yl] ethyl} benzoesaeure-ethylester aus 4-[2-(Piperazin-1-yl) ethyl] benzoesaeure-ethylester. 2 HCl und 2-Methyl-3-phenyl-propionylchlorid.
Ausbeute 96 % d. Th. oelige Base. Das Hydrochlorid schmilzt bei 188-189 °C (Essigester).

55 b) : 4-{3-[1-(4-Chlorbenzoyl) piperazin-4-yl] propyl} benzoesaeure-ethylester aus 4-[3-(Piperazin-1-yl) propyl] benzoesaeure-ethylester. 2 HCl und 4-Chlor-benzoylchlorid.
Ausbeute 70 % d. Th. Hydrochlorid-monohydrat, Schmp. 186-187 °C.

55 c) : 4-{1-[1-(4-Chlorbenzoyl) piperazin-4-yl] prop-2-yl} benzoesaeure-ethylester aus 4-[1-(Piperazin-1-yl) prop-2-yl] benzoesaeure ethylester-dihydrochlorid und 4-Chlor-benzoylchlorid.
Ausbeute 78 % d. Th. Hydrochlorid, Schmp. 234-235 °C (Ethanol).

## Beispiel 56

Durch Umsetzen mit Saeurechloriden bzw. Sulfonylchloriden in Analogie zu Beispiel 47 erhaelt man aus 3-[4-(Piperazin-1-yl)-phenyl] propionsaeure-n-butylester die folgenden Produkte :

56 a) : mit 4-Chlor-benzoylchlorid : 3-{4-[1-(4-Chlorbenzoyl) piperazin-4-yl] phenyl} propionsaeure-n-butylester
Ausbeute 65 % d. Th. Hydrochlorid, Schmp. 186-188 °C (i-Propanol)

56 b) : mit Benzolsulfochlorid : 3-{4-[1-(Benzolsulfonyl) piperazin-4-yl] phenyl} propionsaeure-n-butylester
Ausbeute 69 % d. Th. Hydrochlorid, Schmp. 197-199 °C. Freie Base : Schmp. 60-62 °C.

## Beispiel 57

3-{4-[1-(2-Phenoxypropyl) piperazin-4-yl] phenyl} propionsaeure

Man ruehrt ein Gemisch aus 15 g (33 mmol) n-Butylester, 100 ml 1 N NaOH und 100 ml Methanol 6 h bei 60 °C. Dann wird das Methanol abdestilliert, mit HCl auf den isoelektrischen Punkt eingestellt und das ausfallende schmierige Produkt vom Wasser befreit. Man loest es in 6 N HCl, verduennt mit Wasser und saugt die ausfallenden Kristalle ab. Nach Umkristallisieren aus Methanol 6.5 g (43 % d. Th.), Schmp. 202-204 °C (Hydrochlorid).

In analoger Weise erhaelt man aus den entsprechenden n-Butylestern :

57 a) : 3-{4-[1-(4-Chlorbenzyl) piperazin-4-yl] phenyl} propionsaeure
Ausbeute 87 % d. Th. Hydrochlorid, Schmp. 238-240 °C

57 b) : 3-{4-[1-(4-Chlorbenzoyl) piperazin-4-yl] phenyl} propionsaeure
Ausbeute 63 % d. Th., Schmp. 162-164 °C (Methanol)

57 c) : 3-{4-[1-(Benzolsulfonyl) piperazin-4-yl] phenyl} propionsaeure
Ausbeute 95 % d. Th. Hydrochlorid, Schmp. 238-239 °C.

## Beispiel 58

3-[4-(1-n-Hexadecyl-piperazin-4-yl) phenyl] propionsaeure

Man ruehrt ein Gemisch aus 17.0 g (30 mmol) 3-[4-(1-n-Hexadecyl-piperazin-4-yl) phenyl] propionsaeure-n-butylester, 125 ml 1 N NaOH und 125 ml Methanol 6 h bei 60 °C und destilliert anschliessend das Methanol ab. Aus der kalten waessrigen Phase fallen Kristalle aus, welche abgesaugt und mit Wasser gewaschen werden. Man loest sie in heissem Wasser und faellt mittels 2 N HCl das Hydrochlorid aus. Nach Absaugen, Waschen mit Wasser und Trocknen 13.0 g (85 % d. Th.), Schmp. 218-220 °C. Aus Methanol unter Zusatz von wenig Wasser umkristallisiert schmilzt das Hydrochlorid bei 219-221 °C.

Beispiel 59

3-{4-[1-(4-Chlorphenyl) piperazin-4-yl] phenyl} propionsaeure

Man haelt ein Gemisch aus 12.0 g (27 mmol) n-Butylester der Titelverbindung und 120 ml 6 N HCl 8 h auf Rueckflusstemperatur, kuehlt dann und saugt das ausgefallene Produkt ab. Nach Waschen mit verd. HCl 9.1 g Hydrochlorid mit dem Schmp. 240-242 °C (Eisessig).
In Analogie dazu erhaelt man aus dem entsprechenden Methylester

59 a) : 4-[2-(Piperazin-1-yl) propyl] benzoesaeure
Ausbeute 88 % d. Th. Dihydrochlorid, Schmp. 300 °C (Methanol + 4 N HCl).

Beispiel 60

3-{4-[2-(Piperazin-1-yl) ethyl] phenyl} propionsaeure-ethylester

a) Man haelt ein Gemisch aus 4.8 g (20 mmol) 4'-(2-Bromethyl)-propiophenon, 3.5 g (20 mmol) 1-Benzylpiperazin, 5.5 g (40 mmol) pulv. $K_2CO_3$ sicc. und 100 ml Butanon-2 120 h auf Rueckflusstemperatur, saugt dann heiss ab, waescht den Filterkuchen mit Aceton und dampft die vereinten Filtrate ein. Der Rueckstand wird durch Loesen in Methanol und Filtrieren von Unloeslichem befreit, dann destilliert man das Methanol ab und nimmt in Ether auf. Nach mehrmaligem Waschen mit Wasser und Trocknen ($Na_2SO_4$) wird mit HCl-haltigem Ether das Hydrochlorid ausgefaellt. Man saugt ab, waescht mit Ether und trocknet. Ausbeute 7.1 g (87 % d. Th.) 4'-[2-(1-Benzyl-piperazin-4-yl) ethyl] propiophenon als Hydrochlorid mit dem Schmp. 280-282 °C. Die freie Base schmilzt bei 51-53 °C.

b) Man haelt ein Gemisch aus 57.8 g (172 mmol) 4'-[2-(1-Benzyl-piperazin-4-yl) ethyl] propiophenon, 14.35 g (447 mmol) Schwefel und 90.9 ml Morpholin 120 h bei 140 °C, kuehlt dann ab und ruehrt in Eiswasser ein, wobei eine plastische Masse entsteht. Man nimmt in $CH_2Cl_2$ auf, waescht dreimal mit Wasser und trocknet ($Na_2SO_4$). Dann wird eingedampft ; wobei in quant. Ausbeute 3-{4-[2-(1-Benzyl-piperazin-4-yl) ethyl] phenyl}-propionsaeure-thiomorpholid als dunkelbraunes, nicht kristallisierbares Oel zurueckbleibt, das roh weiterverarbeitet wird.

c) 5.3 g des rohen Thiomorpholids werden mit 15 ml konz. HCl 15 h gekocht, dann behandelt man mit Aktivkohle und filtriert heiss. Nach dem Eindampfen verreibt man mit etwas Wasser, saugt ab und kristallisiert aus Aceton um. Ausbeute 3.4 g 3-{4-[2-(1-Benzyl-piperazin-4-yl) ethyl] phenyl} propionsaeure als Di-Hydrochlorid mit dem Schmp. 246-247 °C.

d) Ein siedendes Gemisch aus 117 g (275 mmol) 3-{4-[2-(1-Benzyl-piperazin-4-yl) ethyl] phenyl} propionsaeure-dihydrochlorid und 1.2 Liter abs. Ethanol wird 18 h langsam mit HCl begast. Dann kuehlt man und saugt ab. 85.3 g (69 % d. Th.) 3-{4-[2-(1-Benzyl-piperazin-4-yl) ethyl] phenyl} propionsaeure-ethylester als Dihydrochlorid. Schmp. 262-264 °C (Ethanol).

e) Man hydriert ein Gemisch aus 57 g (0.126 mol) 3-{4-[2-(1-Benzyl-piperazin-4-yl) ethyl] phenyl} propionsaeure-ethylester und 200 ml Ethanol unter Zusatz von 10 proz. Palladiumkohle bei 50 °C und 50 bar Wasserstoffdruck im Schuettelautoklav, laesst abkuehlen und filtriert. Nach dem Eindampfen wird der Rueckstand in Ether geloest. Man faellt nun das Dihydrochlorid durch Zugabe von HCl-haltigem Ether. Das Dihydrochlorid wird mit Ethanol verrieben und abgesaugt. Ausbeute 31.0 g (68 % d. Th.) 3-{4-[2-(Piperazin-1-yl) ethyl] phenyl}-propionsaeure-ethylester-dihydrochlorid, Schmp. 243-244 °C.

Beispiel 61

3-{4-[2-(1-(4-Chlorbenzyl) piperazin-4-yl) ethyl] phenyl} propionsaeure-chlorbenzylester

erhaelt man durch Umsetzen von 3-{4-[2-(Piperazin-1-yl) ethyl]-phenyl} propionsaeure (Schmp. 264-266 °C) mit 2 mol 4-Chlorbenzylchlorid in Analogie zu den in Beispiel 60, Abschnitt a) angegebenen Bedingungen ($K_2CO_3$/Butanon-2, 36 h Rueckflusstemperatur). Ausbeute 62 % d. Th. Dihydrochlorid, Schmp. 248-250 °C.

Beispiel 62

3-{4-[2-(1-(4-Methoxybenzyl) piperazin-4-yl) ethyl] phenyl} propionsaeure-ethylester

erhaelt man durch Umsetzen von 3-{4-[2-(Piperazin-1-yl) ethyl]-phenyl} propionsaeure-ethylester mit 4-Methoxybenzylchlorid in Analogie zu Beispiel 60, Abschnitt a) in Butanon-2 in Gegenwart von $K_2CO_3$. Reaktionsdauer : 36 h. Ausbeute 71 % d. Th.
Dihydrochlorid, Schmp. 262-264 °C (waessr. Ethanol).

Beispiel 63

3-{4-[2-(1-(4-Chlorbenzoyl) piperazin-4-yl) ethyl] phenyl}-propionsaeure-ethylester

erhaelt man in Analogie zu Beispiel 47 aus 4-Chlor-benzoyl-chlorid und 3-{4-[2-(Piperazin-1-yl) ethyl] phenyl} propionsaeureethylester. Ausbeute 80 % d. Th. Hydrochlorid, Schmp. 222-223 °C (Ethanol).
In Analogie dazu erhaelt man

63 a) : mit Benzolsulfochlorid : 3-{4-[2-(1-Benzolsulfonyl-piperazin-4-yl) ethyl] phenyl}-propionsaeure-ethylester
Ausbeute 91 % d. Th., Schmp. 114-116 °C (Ethanol).

Beispiel 64

3-{4-[2-(1-(4-Chlorbenzyl) piperazin-4-yl) ethyl] phenyl} propionsaeure

Man haelt ein Gemisch aus 0.1 mol des 4-Chlorbenzylesters der Titelverbindung, 0.3 mol 1 N KOH und einem gleichen Volumen Methanol 6 h bei 50 °C, dann wird das Methanol abdestilliert. Die waessrigalkalische Phase wird mit Ether extrahiert, dann mit 2 N HCl angesaeuert. Man saugt ab und behandelt das getrocknete Produkt mit HCl-haltigem Ether. Ausbeute 83 % d. Th. Dihydrochlorid, Schmp. 289-290 °C.
In analoger Weise erhaelt man aus den entsprechenden Ethylestern folgende Saeuren :

64 a) : 3-{4-[2-(1-(4-Methoxybenzyl) piperazin-4-yl) ethyl] phenyl}-propionsaeure
Ausbeute 52 % Dihydrochlorid, Schmp. 277-278 °C (2 N HCl)

64 b) : 3-{4-[2-(1-(4-Chlorbenzoyl) piperazin-4-yl) ethyl] phenyl}-propionsaeure
Ausbeute 76 % d. Th. Hydrochlorid, Schmp. 249-250 °C

64 c) : 3-{4-[2-(1-Benzoylsulfonyl-piperazin-4-yl) ethyl] phenyl}-propionsaeure
Ausbeute 95 % d. Th., Schmp. 177-178 °C,
Hydrochlorid : Schmp. 248-249 °C.

Beispiel 65

4-[1-(n-Hexadecyl) piperazin-4-yl] zimtsaeure

a) Aus n-Hexadecylbromid und 4-(Piperazin-1-yl) benzoesaeureethylester erhaelt man in Analogie zu Beispiel 60, Abschnitt a) in 73 % Ausbeute 4-[1-(n-Hexadecyl) piperazin-4-yl] benzoesaeure-ethylester mit dem Schmp. 73-75 °C

b) Durch Lithiumalanat-Reduktion von 4-[1-(n-Hexadecyl)-piperazin-4-yl] benzoesaeure-ethylester in Ether erhaelt man in 72 % d. Th. 4-[1-(n-Hexadecyl) piperazin-4-yl] benzyl-alkohol mit dem Schmp. 101-102 °C

c) Man ruehrt ein Gemisch aus 16.0 g (38 mmol) des Benzylalkohols, 250 ml Methylenchlorid und 15 g Mangandioxid 6 h bei 20 °C, gibt nochmals 8.5 g Mangandioxid zu, ruehrt weitere 5 h und saugt dann ab. Nach Eindampfen i. Vak. wird einmal aus Ligroin und einmal aus Toluol umkristallisiert. Ausbeute 7.3 g (46 % d. Th.) 4-[1-(n-Hexadecyl) piperazin-4-yl] benzaldehyd. Schmp. 72-73 °C.

d) Man haelt ein Gemisch aus 10.0 g (24 mmol) 4-[1-(n-Hexadecyl)-piperazin-4-yl] benzaldehyd, 2.51 g (24 mmol) Malonsaeure, 50 ml Pyridin und 1.4 ml Piperidin 3 h auf 90 °C, kuehlt dann, gibt etwas Ether zu und saugt ab. Nach Waschen mit Ligroin wird mittels HCl-haltigem Ether das Hydrochlorid hergestellt. Zur Beseitigung von Pyridin-hydrochlorid wird mit Wasser verruehrt, abgesaugt und getrocknet. Nach Umkristallisieren aus Methanol 5.9 g (47 % d. Th.) 4-[1-(n-Hexadecyl) piperazin-4-yl] zimtsaeure-hydrochlorid mit dem Schmp. 253-254 °C.

In analoger Weise erhaelt man 4-[1-(2-Phenoxypropyl) piperazin-4-yl]zimtsaeure ueber folgende Stufen :

65 a1) : 4-[1-(2-Phenoxypropyl) piperazin-4-yl] benzoesaeure-ethylester aus 2-Phenoxypropylbromid und 4-(Piperazin-1-yl)-benzoesaeure-ethylester
Ausbeute 54 % d. Th., Schmp. des Hydrochlorids : 199-201 °C (waessr. Ethanol)

65 b1) : 4-[1-(2-Phenoxypropyl) piperazin-4-yl] benzylalkohol durch Lithium-alanat-Reduktion des Aldehyds.
Ausbeute 80 % d. Th., Schmp. 82-83 °C

65 c1) : 4-[1-(2-Phenoxypropyl) piperazin-4-yl] benzaldehyd durch Oxidation des Benzylalkohols mittels $MnO_2$.

Ausbeute 65 % farbloses Oel.

65 d1) : Man ruehrt ein Gemisch aus 9.0 g (28 mmol) 4-[1-(2-Phenoxypropyl) piperazin-4-yl] benzaldehyd, 2.9 g (28 mmol) Malonsaeure, 40 ml Pyridin und 1.5 ml Piperidin 3 h bei 90 °C. Dann wird abgekuehlt, auf Eiswasser gegossen und das oelige Produkt mit Essigester aufgenommen. Man dampft i. Vak. ein, loest in verd. Natronlauge und schuettelt mit Essigester aus. Die waessrige Phase wird mit HCl angesaeuert, das schwerloesliche Hydrochlorid abgesaugt und mit Wasser gewaschen. Nach Umkristallisieren aus Methanol 7.0 g (63 % d. Th.) 4-[1-(2-Phenoxypropyl) piperazin-4-yl]-zimtsaeure als Hydrochlorid mit dem Schmp. 208-211 °C.

Beispiel 66

4-[1-(n-Hexadecyl) piperazin-4-yl] zimtsaeure

Man suspendiert 6.81 g (48 mmol) Phosphorpentoxid in 100 ml Methylenchlorid, gibt 15.2 g (32 mmol) 3-{4-[1-(n-Hexadecyl)-piperazin-4-yl] phenyl}-3-hydroxy-propionsaeure-methylester (farbloses Oel, hergestellt durch Reduktion von 3-{4-[1-(n-Hexadecyl) piperazin-4-yl] phenyl}-3-oxo-propionsaeuremethylester mittels Natriumborhydrid in Methanol) zu und erhitzt unter kraeftigem Ruehren 12 h auf Rueckflusstemperatur. Anschliessend wird von der schmierigen Polyphosphorsaeure abdekantiert, die fluessige Phase eingedampft, der Eindampfrueckstand in Ethanol geloest und nach Zusatz von 50 ml 2 N Kalilauge 6 h auf 85 °C erhitzt. Man destilliert anschliessend das Ethanol ab, ethert die waessrige Phase aus und saeuert sie danach mit 6 N HCl an. Das ausgefallene Produkt wird abgesaugt, mit kalter 2 N Salzsaeure gewaschen und getrocknet. Ausbeute 8.8 g (56 % d. Th.) Hydrochlorid mit dem Schmp. 251-253 °C.

Beispiel 67

4-{2-[1-(4-Chlorcinnamyl) piperazin-4-yl] ethyl}-α-methyl-zimtsaeure

a) Durch Umsetzen aequimolarer Mengen 1-(4-Chlorcinnamyl) = piperazin mit dem Glykolacetal des 4-(2-Bromethyl)-benzaldehyds in Analogie zu Beispiel 21 erhaelt man in 71 % Ausbeute das Glykolacetal des 4-{2-[1-(4-Chlorcinnamyl) = piperazin-4-yl] ethyl} benzaldehyds mit dem Schmp. 75-77 °C.

b) Man haelt ein Gemisch aus 25 g (59.5 mmol) des obigen Glykolacetals, 500 ml Aceton, 10 ml Wasser und 59.5 ml 2 N Salzsaeure 2 Stunden auf Rueckflusstemperatur und dampft dann ein. Der kristalline Rueckstand wird in Methylenchlorid aufgenommen und die Loesung mit 2 N Sodaloesung ausgeschuettelt. Man waescht mit Wasser, trocknet ($Na_2SO_4$) und dampft ein, wobei in fast quantitativer Ausbeute oeliger 4-{2-[1-(4-Chlorcinnamyl) piperazin-4-yl] ethyl} benzaldehyd zurueckbleibt. Schmp. des Dihydrochlorids : 255-258 °C.

c) Zu 2.92 g (60 mmol) Natriumhydrid (als 50 proz. Oelsuspension) tropft man bei 20 °C eine Loesung aus 14.5 g (60 mmol) 2-Phosphonopropansaeure-triethylester und 50 ml abs. 1.2-Dimethoxyethan. Dann wird 10 Minuten geruehrt und anschliessend eine Loesung aus 22.5 g (60 mmol) 4-{2-[1-(4-Chlorcinnamyl) piperazin-4-yl] ethyl} benzaldehyd und 50 ml abs. 1.2-Dimethoxyethan zugetropft, wobei die Temperatur 30 °C nicht ueberschreiten darf. Man ruehrt dann 20 Minuten bei Raumtemperatur, gibt langsam ca. 50 ml Wasser zu und extrahiert mit Ether. Die Etherphase wird mit Wasser gewaschen, mit $Na_2SO_4$ getrocknet und eingedampft. Es bleiben 13.5 g (49 % d. Th.) oeliger 4-{2-[1-(4-Chlorcinnamyl) piperazin-4-yl] ethyl}-α-methyl-zimtsaeure-ethylester zurueck. Dessen Dihydrochlorid schmilzt bei 276-280 °C.

d) Eine Loesung aus 12.0 g (26.5 mmol) Ethylester, 120 ml Ethanol und 55 ml 1 N Kalilauge wird 12 Stunden bei Raumtemperatur geruehrt, dann engt man auf ein Drittel ein, gibt 50 ml Wasser zu und extrahiert mehrmals mit Ether. Die waessrige Phase wird nun mit HCl angesaeuert. Man saugt den ausgefallenen Niederschlag ab und digeriert ihn mit heissem Ether. Ausbeute 6.8 g (52 % d. Th.) Dihydrochlorid der 4-{2-[1-(4-Chlorcinnamyl) piperazin-4-yl] ethyl}-α-methyl-zimtsaeure mit dem Schmp. 285-287 °C.

In analoger Weise erhaelt man

67 a) : 4-{2-[1-(4-Chlorbenzoyl) piperazin-4-yl] ethyl}-α-methyl-zimtsaeure ueber folgende Zwischenstufen :

a1) : 4-{2-[1-(4-Chlorbenzoyl) piperazin-4-yl] ethyl} = benzaldehyd-glykolacetal aus 1-(4-Chlorbenzoyl) piperazin und 4-(2-Bromethyl) = benzaldehyd-glykolacetal

Ausbeute 70 % d. Th., Schmp. 103-104 °C.

b1) : 4-{2-[1-(4-Chlorbenzoyl) piperazin-4-yl] ethyl} = benzaldehyd aus dem Glykolacetal.
Ausbeute 91 % d. Th., Schmp. 71-73 °C.

c1) : 4-{2-[1-(4-Chlorbenzoyl) piperazin-4-yl] ethyl}-α-methyl-zimtsaeure-ethylester aus 4-{2-[1-(4-Chlorbenzoyl) piperazin-4-yl] ethyl} = benzaldehyd und 2-Phosphonopropansaeure-triethylester.
Rohausbeute 95 % d. Th. als farbloses Oel. Schmp. des Hydrochlorids : 235-239 °C.

d1) : Durch Hydrolyse des Esters erhaelt man die Saeure.
Ausbeute 50 % d. Th. Hydrochlorid mit dem Schmp. 275-276 °C (Methanol).

Beispiel 68

4-{2-[1-(4-Chlorcinnamyl) piperazin-4-yl] ethyl}-β-methyl-zimtsaeure

a) Durch Umsetzen von 1-(4-Chlorcinnamyl) piperazin mit 4-(2-Bromethyl) acetophenon in Analogie zu Beispiel 22 erhaelt man 4-{2-[1-(4-Chlorcinnamyl) piperazin-4-yl] = ethyl} acetophenon. Ausbeute 66 % d. Th. Dihydrochlorid, Schmp. 266-267 °C (waessr. Ethanol). Die freie Base schmilzt bei 64-65 °C.

b) Zu einer Loesung aus 1.0 g (43 mg-Atom) Natrium und 75 ml abs. Ethanol gibt man 6.3 g (28 mmol) Phosphonoessigsaeuretriethylester, laesst 15 Minuten reagieren und tropft dann eine Loesung aus 10.8 g (28 mmol) 4-{2-[1-(4-Chlorcinnamyl) = piperazin-4-yl] ethyl} acetophenon und 50 ml Ethanol zu. Nach 24 Stunden Stehen bei Raumtemperatur dampft man ein, loest den Rueckstand in Ether und extrahiert die Etherphase zweimal mit Wasser. Nach dem Trocknen mit $Na_2SO_4$ wird mit HCl-haltigem Ether das Dihydrochlorid ausgefaellt. Man saugt ab, verreibt mit Ether und kristallisiert aus HCl-haltigem Ethanol um. Ausbeute 10.3 g (70 % d. Th.) Dihydrochlorid des 4-{2-[1-(4-Chlorcinnamyl) piperazin-4-yl] ethyl}-β-methyl-zimtsaeure-ethylesters mit dem Schmp. 269-271 °C.

c) Ein Gemisch aus 9.3 g (17.6 mmol) Ethylester-dihydrochlorid, 150 ml Ethanol und 50 ml 2 N Natronlauge wird bis zur vollstaendigen Verseifung bei 45 °C geruehrt, dann destilliert man den Alkohol i. Vak. ab. Die Loesung wird mit etwas Wasser verduennt, mit Essigester extrahiert und schliesslich mit HCl angesaeuert. Man saugt ab, waescht mit Wasser und Aceton und kristallisiert aus HCl-haltigem Wasser um. Ausbeute 6.2 g (71 % d. Th.) Dihydrochlorid der 4-{2-[1-(4-Chlorcinnamyl) piperazin-4-yl] ethyl}-β-methyl-zimtsaeure mit dem Schmp. 271-272 °C.
In analoger Weise erhaelt man 4-{2-[1-(4-Chlorbenzoyl) piperazin-4-yl] ethyl}-β-methyl-zimtsaeure-ethylester auf folgendem Wege :

a1) : Durch Umsetzen von 1-(4-Chlorbenzoyl) piperazin-hydrochlorid mit 4-(2-Bromethyl) acetophenon in Analogie zu Beispiel 22 erhaelt man 62 % d. Th. Hydrochlorid des 4-{2-[1-(4-Chlorbenzoyl) piperazin-4-yl] ethyl} acetophenons mit dem Schmp. 212-214 °C. Die freie Base ist ein farbloses Oel.

b1) : Die Titelverbindung erhaelt man in Analogie zu b) aus 4-{2-[1-(4-Chlorbenzoyl) piperazin-4-yl] ethyl} acetophenon und Phosphonoessigsaeure-triethylester. Das oelig ausfallende Hydrochlorid wird mit wenig Aceton in Loesung gebracht, dann gibt man Ether zu, wobei Kristallisation einsetzt. Ausbeute 67 % d. Th. Hydrochlorid mit dem Schmp. 212-215 °C (Ethanol).

Beispiel 69

3-{4-{2-[1-(4-Chlorbenzoyl) piperazin-4-yl] ethyl} phenyl} buttersaeure

a) Durch Umsetzen von 1-Ethoxycarbonyl-piperazin mit 4-(2-Bromethyl) acetophenon in Analogie zu Beispiel 22 erhaelt man 69 % d. Th. 4-[2-(1-Ethoxycarbonyl-piperazin-4-yl) ethyl] acetophenon-hydrochlorid mit dem Schmp. 182-185 °C (Ethanol).

b) Aus 4-[2-(1-Ethoxycarbonyl-piperazin-4-yl) ethyl] acetophenon und Phosphonoessigsaeure-triethylester erhaelt man in Analogie zu Beispiel 68 b 63 % d. Th. Hydrochlorid des 4-[2-(1-Ethoxycarbonyl-piperazin-4-yl) ethyl]-β-methyl-zimtsaeure-ethylesters. Schmp. 217-218 °C (Ethanol).

c) Man hydriert bei Normaldruck und Raumtemperatur ein Gemisch aus 25.0 g (60 mmol) 4-[2-(1-Ethoxycarbonyl-piperazin-4-yl) ethyl]-β-methyl-zimtsaeure-ethylester-hydrochlorid, 500 ml Methanol, 75 ml 2 N Salzsaeure und einer Spatelspitze 10 proz. Pd-Kohle, saugt ab, dampft ein und kristallisiert den Rueckstand aus Ethanol um. Ausbeute 14.3 g (57 % d. Th.) 3-{4-[2-(1-Ethoxycarbonyl-piperazin-4-yl) ethyl] phenyl} buttersaeure-ethylester-hydrochlorid mit dem Schmp. 220-222 °C.

d) Man haelt ein Gemisch aus 14.0 g (34 mmol) 3-{4-[2-(1-Ethoxycarbonyl-piperazin-4-yl) ethyl] phenyl} buttersaeure-ethylesterhydrochlorid und 150 ml conc. Salzsaeure 12 Stunden auf Rueckflusstemperatur, dampft dann ein und kristallisiert aus Aceton, das etwas 2 N HCl enthaelt, um. Ausbeute 11.6 g (98 % d. Th.) Dihydrochlorid der 3-{4-[2-(Piperazin-1-yl) ethyl] phenyl} buttersaeure mit dem Schmp. 231-232 °C.

e) Man loest 11.4 g Dihydrochlorid der 3-{4-[2-(Piperazin-1-yl) ethyl] phenyl} buttersaeure in 120 ml Ethanol und begast 2 Stunden lang bei Rueckflusstemperatur mit Chlorwasserstoff. Dann wird auf die Haelfte eingedampft, abgekuehlt, mit Ether versetzt und abgesaugt. Ausbeute 10.8 g (88 % d. Th.) Dihydrochlorid des 3-{4-[2-(Piperazin-1-yl) ethyl] phenyl} buttersaeure-ethylesters mit dem Schmp. 249-251 °C (Ethanol).

f) Durch Umsetzen des obigen Buttersaeure-ethylesters mit 4-Chlor-benzoylchlorid in Analogie zu Beispiel 55 erhaelt man in 94 % Ausbeute das Hydrochlorid des 3-{4-{2-[1-(4-Chlor-benzoyl) piperazin-4-yl] ethyl} phenyl} buttersaeure-ethylesters. Schmp. 189-191 °C (Ethanol).

g) Die Titelverbindung erhaelt man durch Hydrolyse von 3-{4-{2-[1-(4-Chlor-benzoyl) piperazin-4-yl] ethyl} phenyl} buttersaeure-ethylester mit einem Gemisch aus 2 N Natronlauge und Ethanol in Analogie zu Beispiel 64. Nach Zugabe von Salzsaeure enthaelt das Produkt 4-Chlor-benzoesaeure. Diese wird durch Anruehren mit Ether entfernt. Ausbeute 60 % d. Th. Hydrochlorid, Schmp. 249-251 °C (Ethanol).

Beispiel 70

4-{2-[1-(4-Carboxycinnamyl) piperazin-4-yl] ethyl} benzoesaeure

Man erhitzt ein Gemisch aus 4-{2-[1-(4-Cyanocinnamyl) piperazin-4-yl] ethyl} benzoesaeure-ethylester und einer Loesung aus gleichen Volumina 10 N Natronlauge und Ethanol 3 Stunden im Autoklav auf 130 °C und dampft anschliessend das Ethanol ab. Dann wird mit conc. Salzsaeure angesaeuert, stark gekuehlt und der ausgefallene Niederschlag abgesaugt. Nach Umkristallisieren aus Wasser 47 % d. Th. Dihydrochlorid mit dem Schmp. 274-276 °C.

Beispiel 71

1-(4-Carboxy-phenethyl)-4-[2-(4-carboxy-phenoxy) propyl] piperazin

Man haelt ein Gemisch aus 7.5 g (15 mmol) 1-(4-Ethoxycarbonyl-phenethyl)-4-[2-(4-cyanophenoxy) propyl] piperazin-dihydrochlorid und 75 ml conc. Salzsaeure 12 Stunden auf Rueckflusstemperatur. Dann wird abgekuehlt, abgesaugt und aus 1 N Salzsaeure umkristallisiert. Ausbeute 5.2 g (70 % d. Th.) Dihydrochlorid, Schmp. 270-271 °C.

Beispiel 72

1-(4-Carboxy-phenethyl)-4-[2-(4-acetamino-phenoxy) propyl] piperazin

Ein Gemisch aus 9.3 g (18.9 mmol) 1-(4-Carboxy-phenethyl)-4-[2-(4-aminophenoxy) propyl] piperazintrihydrochlorid und 50 ml Acetanhydrid wird 2 Stunden auf 100 °C gehalten und dann abgekuehlt. Man saugt den ausgefallenen Niederschlag ab und kristallisiert aus Essigsaeure um. Ausbeute 8.2 g (87 % d. Th.) Dihydrochlorid, Schmp. 234-235 °C.

Beispiel 73

1-(4-Ethoxycarbonyl-phenethyl)-4-[2-(4-aminophenoxy) propyl] piperazin

Durch Hydrieren von 1-(4-Ethoxycarbonyl-phenethyl)-4-[2-(4-nitrophenoxy) propyl] piperazin-dihydrochlorid in Ethanol in Gegenwart von 10 proz. Palladiumkohle unter Zusatz von 10 Vol.-% 2 N Salzsaeure bei Normaldruck. Man filtriert den Katalysator ab, dampft i. Vak. ein und verreibt den Rueckstand mit kaltem Ethanol. Ausbeute 80 % d. Th. Dihydrochlorid, Schmp. 260-261 °C.

Beispiel 74

4-{2-[1-(3-Chlorbenzyl) piperazin-4-yl] ethyl} benzoesaeure-diethylamid

Ein Gemisch aus 5.0 g (11 mmol) 4-{2-[1-(3-Chlorbenzyl) piperazin-4-yl] ethyl} benzoesaeure. 2 HCl, 50 ml Thionylchlorid und 2 Tropfen DMF wird 4 Stunden auf Rueckflusstemperatur gehalten, dann kuehlt man und saugt ab. Man waescht das Saeurechlorid mit abs. Ether, loest es in THF und gibt 8.1 g (0.11 g) Diethylamin zu. Nach 30 Minuten Ruehren wird Diethylaminhydrochlorid abgesaugt, das Filtrat eingedampft, der Rueckstand in Ether aufgenommen und die Loesung mit HCl-haltigem Ether versetzt. Das

ausgefallene Dihydrochlorid saugt man ab und kristallisiert aus einem Ethanol-2 N HCl-Gemisch um. Ausbeute 3.6 g (66 % d. Th.) Dihydrochlorid, Schmp. 251-252 °C.

Beispiel 75

4-{2-[1-(2-Chlorbenzyl) piperazin-4-yl] ethyl} benzoesaeure-(2-hydroxyethylamid)

Zu einer Loesung aus 11.0 g (24.4 mmol) 4-{2-[1-(2-Chlorbenzyl) piperazin-4-yl] ethyl} benzoylchloriddihydrochlorid (darstellbar aus dem Dihydrochlorid der Saeure durch 3-stuendiges Kochen mit ueberschuessigem Thionylchlorid, Eindampfen und Digerieren mit Ligroin ; Ausbeute 98 % d. Th., Schmp. 260 °C) und 10 ml abs. Pyridin gibt man unter Ruehren bei 20 °C 10 ml Ethanolamin, laesst ueber Nacht stehen und giesst dann in ca. 100 ml Wasser. Der ausgefallene Niederschlag wird mit Natriumhydrogencarbonat-Loesung und Wasser gewaschen, getrocknet und aus waessrigem Ethanol umkristallisiert. Ausbeute 5.0 g (51 % d. Th.), Schmp. 121-122 °C.

Beispiel 76

4-{2-[1-(2-Chlorbenzyl) piperazin-4-yl} benzoesaeureamid

Zu einer Loesung aus 14.2 g (31.5 mmol) 4-{2-[1-(2-Chlorbenzyl) piperazin-4-yl] ethyl} benzoylchloriddihydrochlorid und 150 ml Dioxan tropft man unter Ruehren bei Raumtemperatur innerhalb 30 Minuten eine Loesung aus 100 ml conc. Ammoniak in 50 ml Dioxan. Man ruehrt weitere 30 Minuten und fuegt dann so viel Wasser zu, dass das Amid ausfaellt. Nach Absaugen, Trocknen und Umkristallisieren aus Toluol 8.5 g (75 % d. Th.) Amid, Schmp. 170-173 °C.

Beispiel 77

4-{2-[1-(2-Chlorbenzyl) piperazin-4-yl] ethyl} benzoesaeure-(2-diethylamino-ethylester)

Ein Gemisch aus 9.45 g (21 mmol) 4-{2-[1-(2-Chlorbenzyl) piperazin-4-yl] ethyl} benzoylchloriddihydrochlorid, 50 ml Methylenchlorid und 3.3 g (28 mmol) 2-Diethylamino-ethanol wird 3 Stunden auf Rueckflusstemperatur gehalten, dann kuehlt man ab und versetzt bis zur Truebung mit Ether. Es faellt langsam ein dicker Niederschlag aus. Der wird abgesaugt, mit sec.-Butanol gewaschen, in einem Gemisch aus sec.-Butanol und Ethanol heiss geloest. Beim Abkuehlen und partiellen Einengen erhaelt man 5.0 g (35 % d. Th.) Dihydrochlorid mit dem Schmp. 220-221 °C.

Beispiel 78

4-{1-[1-(4-Chlorcinnamyl) piperazin-4-yl] prop-2-yl} benzoesaeure

a) Durch Umsetzen von 1-Benzyl-piperazin mit 4-(1-Brom-prop-2-yl) benzoesaeure-ethylester in Analogie zu Beispiel 23 erhaelt man in 80 % Ausbeute 4-[1-(1-Benzyl-piperazin-4-yl) prop-2-yl] benzoesaeure-ethylester als Dihydrochlorid mit dem Schmp. 235-236 °C (Ethanol).

b) Hydrieren der unter a) erhaltenen Verbindung unter den in Beispiel 20 b angegebenen Bedingungen ergibt in 62 % Ausbeute das Dihydrochlorid des 4-[1-(Piperazin-1-yl) prop-2-yl] benzoesaeure-ethylesters. Schmp. 245-246 °C) waessriges Ethanol).

c) Durch Umsetzen von 4-Chlor-cinnamylchlorid mit dem Dihydrochlorid des 4-[1-(Piperazin-1-yl) prop-2-yl] benzoesaeureesters in Methylenchlorid in Gegenwart von Triethylamin (Analogie zu Beispiel 25) erhaelt man in 81 % Ausbeute das Dihydrochlorid des 4-{1-[1-(4-Chlorcinnamyl) piperazin-4-yl] prop-2-yl} benzoesaeure-ethylesters mit dem Schmp. 249-250 °C (Ethanol).

d) Die Titelverbindung erhaelt man durch 16 Stunden Verseifen des unter c) beschriebenen Ethylesters mit waessriger Salzsaeure in der Siedehitze (Analogie zu Beispiel 34) als Dihydrochlorid. Schmp. 290-291 °C (Eisessig). Ausbeute 64 % d. Th.

Beispiel 79

4-{2-[1-(4-Chlorcinnamyl) piperazin-4-yl] propyl} benzoesaeure

a) Zu einem Gemisch aus 15.5 g (88 mmol) 1-Benzylpiperazin und 200 ml abs. Methanol gibt man so viel chlorwasserstoffhaltiges Methanol, dass pH 5 erreicht wird. Dann fuegt man 15.3 g (80 mmol) 4-(2-Oxo-propyl) benzoesaeure-methylester und 3.5 g (55 mmol) Natrium-cyan-borhydrid zu, ruehrt 4 Tage lang bei Raumtemperatur und versetzt mit conc. Salzsaeure. Durch fraktioniertes Faellen mit Ether erhaelt man 19.2 g (57 % d. Th.) Dihydrochlorid des 4-[2-(1-Benzyl-piperazin-4-yl) propyl] benzoesaeure-methylesters mit dem Schmp. 256 °C.

b) Man hydriert die nach a) erhaltene Benzylverbindung in methanolischer Loesung in Gegenwart von Palladium-Kohle bei 50 °C und Normaldruck und kristallisiert aus Methanol um. Ausbeute 76 % d. Th. Dihydrochlorid des 4-[2-(Piperazin-1-yl) propyl] benzoesaeure-methylesters, Schmp. 238 °C.

c) Durch Umsetzen des freien 4-[2-(Piperazin-1-yl) propyl) benzoesaeure-methylesters mit 4-Chlorcinnamylchlorid in Butanon-2 in Gegenwart von Kaliumcarbonat (Analogie zu Beispiel 23) erhaelt man 4-{2-[1-(4-Chlorcinnamyl) piperazin-4-yl] propyl} benzoesaeure-methylester. Ausbeute 71 % Dihydrochlorid, Schmp. 263 °C (Methanol + 2 N HCl).

d) Durch Hydrolyse des Methylesters in Analogie zu Beispiel 64 erhaelt man die Titelverbindung in 84 % Ausbeute als Dihydrochlorid mit dem Schmp. 286 °C (Ethanol + 2 N HCl).

Beispiel 80

Es wurden Tabletten mit jeweils 20 mg
4-{2-[1-(2-Phenoxypropyl) piperazin-4-yl] ethyl} benzoesaeure gemäß folgender Rezeptur hergestellt :

| | |
|---|---|
| 4-{2-[1-(2-Phenoxypropyl) piperazin-4-yl] ethyl} benzoesaeure | 20 g |
| Lactose | 160 g |
| Stärke | 158 g |
| Magnesiumstearat | 2 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1 000 Tabletten mit einem Einzelgewicht von 0,24 g verpreßt.

Versuchsprotokoll

Zur Bestimmung der lipidsenkenden und aggregationshemmenden Wirkung der erfindungsgemäßen Verbindungen wurden folgende Versuche durchgeführt :

1. Lipidsenkung

Jeweils 10 männlichen, stoffwechselgesunden Ratten wurde die zu testende Substanz 7 Tage lang in einer Dosis von 100 mg/kg/d in Methylcellulose-Suspension verabreicht. Am Versuchsende, 3 Stunden nach der letzten Sondierung wurden die Cholesterin- und Triglyceridwerte im Serum bestimmt. In Tabelle I sind die Veränderungen in % im Vergleich zu Kontrollkollektiven für eine repräsentative Auswahl von Verbindungen angegeben

| Beispiel Nr. | Lipidsenkung | |
|---|---|---|
| | % Senkung Chol. | % Senkung Triglyc. |
| 33 r | 33 | 15 |
| 33 u | 28 | 32 |
| 34 i | 21 | 1o |
| 34 s | 47 | 21 |
| 55 b | 33 | 3 |
| 67 $d_1$ | 37 | 9 |
| 5o g | 28 | 9 |
| 13 i | 38 | 15 |
| 33 f | 37 | 42 |
| 33 l | 38 | 22 |
| 33 c | 13 | 11 |
| 33 i | 41 | 24 |
| 33 o | 35 | 13 |
| 34 c | 3o | 4o |
| 33 d | 43 | 41 |
| 49 e | 41 | 8 |
| 5o f | 4o | 16 |
| 33 m | 2o | 3o |

Fortsetzung

| Beispiel Nr. | Lipidsenkung | |
|---|---|---|
| | % Senkung Chol. | % Senkung Triglyc. |
| 5o o | 26 | 14 |
| 58 | 17 | 6 |
| 33 b | 4o | 3o |
| 5o j | 4o | 0 |
| 65 $d_1$ | 5 | 2o |

2. Aggregationshemmung

Die Beeinflussung der Thrombozyten-Aggregation wurde nach dem Born-Test (J. Physiol. *168*, 178, 1963) mit Human-Blutproben bestimmt.

a) Methodik

Venöses Blut von stoffwechselgesunden Probanden wird mit Natriumcitrat (9 : 1) gemischt. Durch Zentrifugation bei 150 g werden die Erythrozyten sedimentiert. Im Überstand sind die Thrombozyten angereichert. Dieser Überstand wird als plättchenreiches Plasma (PRP) bezeichnet.

Ein Aliquot des PRP wird in die Küvette eines Aggregometers (Universal Aggregometer der Firma Braun Melsungen) gebracht und dort mittels eines kleinen Magneten gerührt. Die zu testende Substanz wird in wässriger Lösung (pH ca. 7) zugesetzt. Veränderungen der Lichttransmission in der Suspension werden laufend durch einen Schreiber ausgezeichnet.

Nach Ablauf der spontanen Aggregation wird durch die Zugabe von Adrenalin (Endkonzentration $5 \times 10^{-6}$ mol/l) die Aggregation ausgelöst. Es bilden sich größere Thrombozyten-Aggregate und dadurch nimmt die Lichttransmission durch die Suspension zu.

b) Auswertung

Die Adrenalin-induzierte Aggregation verläuft in 2 Phasen d. h., die Lichttransmission nimmt zunächst zu, stagniert dann kurzfristig und nimmt nochmals zu. Durch Aggregationshemmer kann nur die 2. Phase der Aggregation beeinflußt werden.

Für die Dokumentation der Ergebnisse wird der Winkel der 2. Aggregationsphase gegen die Horizontale für die durch Adrenalin-induzierte Aggregation bestimmt und diese wird als 0 % Hemmung angesetzt. (Kontrollversuch).

Mit dem gleichen PRP wird nach Zugabe der Testsubstanz die Aggregation mit Adrenalin induziert und der Verlauf der Aggregation über den Schreiber registriert. Der Winkel der 2. Phase gegenüber der Horizontalen wird wiederum bestimmt und das Verhältnis der beiden Winkel gibt die % Hemmung der 2. Phase der Thrombozyten-Aggregation an.

(Für das gut aggregationshemmende Vergleichspräparat Acetylsalicylsäure, beträgt die Hemmung bei einer Konzentration von $10^{-4}$ mol/l 100 %. Bei einer Konzentration von $5 \times 10^{-5}$ mol/l 0 %).

Alle Substanzen wurden in einer Konzentration von $10^{-4}$ mol/l auf ihre aggregationshemmende Wirkung geprüft.

Die Ergebnisse sind in Tabelle II zusammengefaßt :

| Beispiel | % Hemmung der Thrombozyten-aggregation |
|---|---|
| 34 i | 100 |
| 33 c | 40 |
| 34 c | 7 |
| 33 d | 6 |
| 33 b | 19 |

**Patentansprüche**

1. Carbonsäuren der allgemeinen Formel I

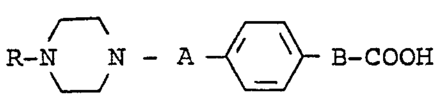

(I)

in der

A eine Valenzbindung oder eine geradkettige oder verzweigte Alkylenkette mit 1 bis 3 C-Atomen,

B eine Valenzbindung oder eine gesättigte oder ungesättigte, geradkettige oder verzweigte Alkylenkette mit 1 bis 3 C-Atomen und

R ein Wasserstoffatom, eine geradkettige oder verzweigte $C_1$-$C_{16}$-Alkylkette, die ein- oder mehrfach durch Hydroxyl, Carboxyl, eine Sulfonsäuregruppe oder eine gegebenenfalls ein- oder mehrfach durch $C_1$-$C_6$-Alkyl, $C_1$-$C_6$-Alkoxy, Halogen, Nitro, Cyano, Carboxyl oder $C_1$-$C_6$-Acylamino substituierte Phenoxygruppe substituiert ist, eine Phenylalkylkette, wobei die Phenylgruppe gegebenenfalls eine oder mehrere Halogenatome, Hydroxyl, $C_1$-$C_6$-Alkoxy, Cyano, Carboxyl-, Nitro-, $C_1$-$C_6$-Acylamino-, $C_1$-$C_6$-Alkyl- oder Trifluormethyl-Gruppen tragen kann und die Alkylkette gegebenenfalls ungesättigt ist und bis zu 4 C-Atome aufweist, eine Phenacylgruppe, deren Phenylrest ein- oder mehrfach durch Halogen, Hydroxy oder $C_1$-$C_6$-Alkyl substituiert sein kann und deren Acyl-Teil 2 bis 4 C-Atome enthält, einen 2-Methyl-3-phenylpropionyl-, 3-Phenyl-propionyl-, Cinnamoyl-, Phenacetyl- oder Benzoyl-Rest, wobei die Phenylreste durch Halogen, Hydroxyl oder $C_1$-$C_6$-Alkyl substituiert sein können, einen Methansulfonyl-, Benzolsulfonyl- oder Phenacylsulfonyl-Rest, wobei der Phenylrest durch Halogen oder $C_1$-$C_6$-Alkyl substituiert sein kann, oder einen 4-Chlorphenyl-, 3-Trifluormethylphenyl-, einen 4-(4-Chlorbenzoyl)-phenyl-, einen 4-Biphenylyl-, einen 4-Phenoxyphenyl- oder 4-Benzyloxyphenyl-Rest

bedeuten, mit der Maßgabe, daß wenn A eine Valenzbindung ist, R nicht Wasserstoff, Methyl, Ethyl, Hydroxyethyl oder Benzyl sein darf, sowie deren physiologisch unbedenkliche Salze, deren Ester mit Methanol, Ethanol, n-Butanol, 4-Chlor-benzylalkohol oder 2-Diethylamino-ethanol und deren Amide mit Ammoniak, Diethylamin, Ethanolamin, p-Amino-benzoesäure, β-Alanin oder Morpholin.

2. Verbindung gemäß Formel I des Anspruches 1 mit B = Valenzbindung und den angegebenen Bedeutungen für A and R.

3. Verbindungen gemäß Formel I des Anspruches 1 mit B = $CH_2$-Gruppe und A eine niedere Alkylenkette und den angegebenen Bedeutungen für R.

4. Verbindungen gemäß Formel I des Anspruches 1 mit A = $CH_2$-$CH_2$ und B = Valenzbindung und den angegebenen Bedeutungen für R.

5. Verfahren zur Herstellung von Carbonsäuren der allgemeinen Formel I

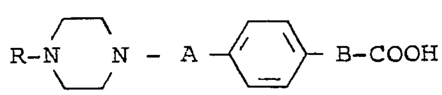

(I)

in der

A eine Valenzbindung oder eine geradkettige oder verzeigte Alkylenkette mit 1 bis 3 C-Atomen,

B eine Valenzbindung oder eine gesättigte oder ungesättigte, geradkettige oder verzweigte Alkylenkette mit 1 bis 3 C-Atomen und

R ein Wasserstoffatom, eine geradkettige oder verzweigte $C_1$-$C_{16}$-Alkylkette, die ein- oder mehrfach durch Hydroxyl, Carboxyl, eine Sulfonsäuregruppe oder eine gegebenenfalls ein- oder mehrfach durch $C_1$-$C_6$-Alkyl, $C_1$-$C_6$-Alkoxy, Halogen, Nitro, Cyano, Carboxyl oder $C_1$-$C_6$-Acylamino substituierte Phenoxygruppe substituiert ist, eine Phenylalkylkette, wobei die Phenylgruppe gegebenenfalls eine oder mehrere Halogenatome, Hydroxyl, $C_1$-$C_6$-Alkoxy-, Cyano-, Carboxyl-, Nitro-, $C_1$-$C_6$-Acylamino-, $C_1$-$C_6$-Alkyl- oder Trifluormethyl-Gruppen tragen kann und die Alkylkette gegebenenfalls ungesättigt ist und bis zu 4 C-Atome aufweist, eine Phenacylgruppe, deren Phenylrest ein- oder mehrfach durch Halogen, Hydroxy oder $C_1$-$C_6$-Alkyl substituiert sein kann und deren Acyl-Teil 2 bis 4 C-Atome enthält, einen 2-Methyl-3-phenylpropionyl-, 3-Phenyl-propionyl-, Cinnamoyl-, Phenacetyl- oder Benzoyl-Rest, wobei die Phenylreste durch Halogen, Hydroxyl oder $C_1$-$C_6$-Alkyl substituiert sein können, einen Methansulfonyl-, Benzolsulfonyl- oder Phenacylsulfonyl-Rest, wobei der Phenylrest durch Halogen oder $C_1$-$C_6$-Alkyl substituiert sein kann, oder einen 4-Chlorphenyl-, 3-Trifluormethylphenyl-, einen 4-(4-Chlorbenzoyl)-phenyl-, einen 4-Biphenylyl-, einen 4-Phenoxyphenyl- oder 4-Benzyloxyphenyl-Rest

bedeuten, mit der Maßgabe, daß wenn A eine Valenzbindung ist, R nicht Wasserstoff, Methyl, Ethyl, Hydroxyethyl oder Benzyl sein darf, sowie deren physiologisch unbedenkliche Salze, deren Ester mit Methanol, Ethanol, n-Butanol, 4-Chlor-benzylalkohol oder 2-Diethylamino-ethanol und deren Amide mit Ammoniak, Diethylamin, Ethanolamin, p-Amino-benzoesäure, β-Alanin oder Morpholin, dadurch gekennzeichnet, daß man in an sich bekannter Weise

a) eine Verbindung der Formel II

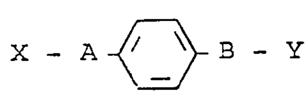

(II)

entweder mit einem $N_1$-geschützten Piperazin umsetzt und nach Abspaltung der Schutzgruppe den Substituenten R durch Alkylierung oder Acylierung einführt, oder mit einem bereits durch R monosubstituierten Piperazin umsetzt oder

b) eine Verbindung der Formel III

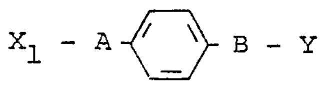

(III)

mit einer Verbindung der Formel IV

$$R—X_2 \qquad (IV)$$

umsetzt, wobei einer der Reste $X_1$ oder $X_2$ eine $NH_2$-Gruppe bedeutet und der andere eine

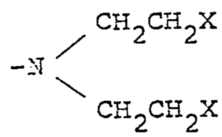

-Gruppe darstellt, während in beiden obigen Verfahren

A und R die angegebene Bedeutung haben,

X einen reaktiven Rest,

B eine Valenzbindung, eine gesättigte oder ungesättigte niedere Alkylenkette oder einen in diese Gruppe überführbaren Rest und

Y eine Säureamid- oder $—COOR_2$-Gruppe, in der $R_2$ Wasserstoff oder eine niedere Alkylgruppe bedeutet oder einen in diese Gruppen überführbaren Rest darstellt,

bedeuten, anschließend gegebenenfalls die erhaltenen Verbindungen in die freie Carbonsäure, ihre Salze, Ester oder Amide überführt, gegebenenfalls Gruppen B in gesättigte oder ungesättigte niedere Alkylketten umwandelt oder den Rest R gegen einen anderen Rest R austauscht.

6. Arzneimittel, enthaltend eine Verbindung gemäß Anspruch 1 und übliche Träger- und Hilfsstoffe.

7. Verbindungen gemäß Anspruch 1 zur Verwendung als Arzneimittel bei der Bekämpfung erhöhter Lipide im Blut.

## Claims

1. Carboxylic acids of the general formula I

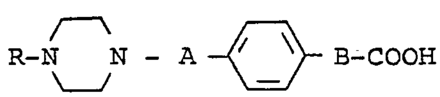

(I)

in which

A signifies a valency bond or a straight-chained or branched alkylene chain with 1 to 3 C-atoms,

B a valency bond or a saturated or unsaturated, straight-chained or branched alkylene chain with 1 to 3 C-atoms and

R a hydrogen atom, a straight-chained or branched $C_1$-$C_{16}$-alkyl chain, which is substituted one or more times by hydroxyl, carboxyl, a sulphonic acid group or a phenoxy group optionally substituted one or more times by $C_1$-$C_6$-alkyl, $C_1$-$C_6$-alkoxy, halogen, nitro, cyano, carboxyl or $C_1$-$C_6$-acylamino ; a phenylalkyl chain, whereby the phenyl group can optionally carry one or more halogen atoms, hydroxyl, $C_1$-$C_6$-alkoxy, cyano, carboxyl, nitro, $C_1$-$C_6$-acylamino, $C_1$-$C_6$-alkyl or trifluoromethyl groups and the alkyl chain is optionally unsaturated and has up to 4 C-atoms ; a phenacyl group, the phenyl radical of which can be substituted one or more times by halogen, hydroxyl or $C_1$-$C_6$-alkyl and the acyl part of which contains 2 to 4 C-atoms ; a 2-methyl-3-phenylpropionyl, 3-phenyl-propionyl, cinnamoyl, phenacetyl or benzoyl radical, whereby the phenyl radicals can be substituted by halogen, hydroxyl or $C_1$-$C_6$-alkyl ; a methanesulphonyl, benzenesulphonyl or phenacylsulphonyl radical, whereby the phenyl radical can be substituted by halogen or $C_1$-$C_6$-alkyl ; or a 4-chlorophenyl, 3-trifluoromethylphenyl, a 4-(4-chlorobenzoyl)-phenyl, a 4-biphenylyl, a 4-phenoxyphenyl or a 4-benzyloxyphenyl radical ;

with the proviso that when A is a valency bond, R cannot be hydrogen, methyl, ethyl, hydroxyethyl or benzyl ; as well as their physiologically acceptable salts, their esters with methanol, ethanol, n-butanol, 4-chlorobenzyl alcohol or 2-diethylamino-ethanol and their amides with ammonia, diethylamine, ethanolamine, p-aminobenzoic acid, β-alanine or morpholine.

2. Compounds according to formula I of claim 1 with B = valency bond and the given meanings for A and R.

3. Compounds according to formula I of claim 1 with B = $CH_2$ group, A a lower akylene chain and the given meaning for R.

4. Compounds according to formula I of claim 1 with A = $CH_2—CH_2$ and B = valency bond and the given meaning for R.

5. Process for the preparation of carboxylic acids of the general formula I

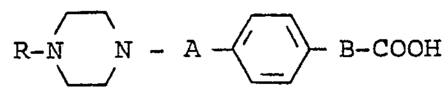

(I)

in which

A signifies a valency bond or a straight-chained or branched alkylene chain with 1 to C-atoms,

B a valency bond or a satured or unsaturated, straight-chained or branched alkylene chain with 1 to 3 C-atoms and

R a hydrogen atom, a straight-chained or branched $C_1$-$C_{16}$ alkyl chain, which is substituted one or more times by hydroxyl, carboxyl, a sulphonic acid group or a phenoxy group optionally substituted one or more times by $C_1$-$C_6$-alkyl, $C_1$-$C_6$-alkoxy, halogen, nitro, cyano, carboxyl or $C_1$-$C_6$-acylamino ; a phenylalkyl chain, whereby the phenyl group can optionally carry one or more halogen atoms, hydroxyl, $C_1$-$C_6$-alkoxy, cyano, carboxyl, nitro, $C_1$-$C_6$-acylamino, $C_1$-$C_6$-alkyl or trifluoromethyl groups and the alkyl chain is optionally unsaturated and has up to 4 C-atoms ; a phenacyl group, the phenyl radical of which can be substituted one or more times by halogen, hydroxyl or $C_1$-$C_6$-alkyl and the acyl part of which contains 2 to 4 C-atoms ; a 2-methyl-3-phenylpropionyl, 3-phenyl-propionyl, cinnamoyl, phenacetyl or benzoyl radical, whereby the phenyl radical can be substituted by halogen, hydroxyl or $C_1$-$C_6$-alkyl ; a methanesulfphonyl, benzenesulphonyl or phenacylsulphonyl radical, whereby the phenyl radical can be substituted by halogen or $C_1$-$C_6$-alkyl ; or a 4-chlorophenyl, 3-trifluoromethylphenyl, a 4-(4-chlorobenzoyl)-phenyl, a 4-biphenylyl, a 4-phenoxyphenyl or 4-benzyloxyphenyl radical ;
with the proviso that when A is a valency bond, R cannot be hydrogen, methyl, ethyl, hydroxyethyl or benzyl ; as well as of their physiologically acceptable salts, of their esters with methanol, ethanol, n-butanol, 4-chlorobenzyl alcohol or 2-diethylaminoethanol and of their amides with ammonia, diethylamine, ethanolamine, p-aminobenzoic acid, β-alanine or morpholine, characterised in that in per se known manner one

a) reacts a compound of the formula II

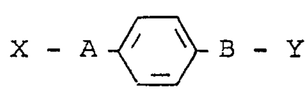

(II)

either with an $N_1$-protected piperazine and, after splitting off the protective group, introduces the substituent R by alkylation or acylation ; or with a piperazine already monosubstituted by R ; or

b) reacts a compound of the formula III

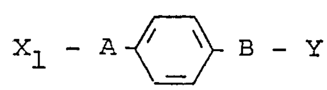

(III)

with a compound of the formula IV

$$R—X_2 \quad (IV)$$

whereby one of the residues $X_1$ and $X_2$ signifies an $NH_2$ group and the other a

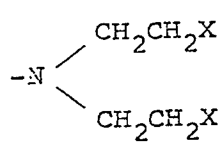

group, while in both the above processes

A and R have the given meanings,

X signifies a reactive residue,

B a valency bond, a saturated or unsaturated lower alkylene chain or a residue convertible into this group and

Y an acid amide or $—COOR_2$ group, in which $R_2$ represents hydrogen or a lower alkyl group or a residue convertible into these groups ; subsequently possibly converts the compounds obtained into the free carboxylic acids, their salts, esters or amides, possibly converts groups B into saturated or unsaturated lower alkyl chains or exchanges the residue R into another residue R.

6. Medicaments containing a compound according to claim 1 and conventional carrier and adjuvant materials.

7. Compounds according to claim 1 for use as medicaments in the combating of increased lipids in the blood.

**Revendications**

1. Acides carboxyliques de formule générale I

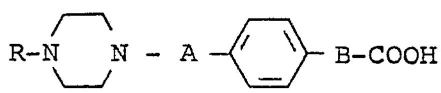

(I)

dans laquelle

A est une liaison de valence ou une chaîne alkylène droite ou ramifiée ayant de 1 à 3 atomes de carbone,

B est une liaison de valence ou une chaîne alkylène saturée ou insaturée, droite ou ramifiée ayant de 1 à 3 atomes de carbone et,

R est un atome d'hydrogène, une chaîne alkyle $C_1$-$C_{16}$ droite ou ramifiée, substituée une ou plusieurs fois par de l'hydroxyle, du carboxyle, un groupe acide sulfonique ou par un groupe phénoxy éventuellement substitué une ou plusieurs fois par de l'alkyle $C_1$-$C_6$, de l'alcoxy $C_1$-$C_6$, de l'halogène, du nitro, du cyano, du carboxyle ou de l'acylamino $C_1$-$C_6$, une chaîne phénylalkyle dans laquelle le groupe phényle peut porter éventuellement un ou plusieurs atomes d'halogène, de l'hydroxyle, de l'alcoxy $C_1$-$C_6$, du cyano, du carboxyle, du nitro, de l'acylamino $C_1$-$C_6$, de l'alkyle $C_1$-$C_6$ ou des groupes trifluorométhyle, et la chaîne alkyle est éventuellement insaturée et possède jusqu'à 4 atomes de carbone, un groupe phénacyle, dont le reste phényle peut être substitué une ou plusieurs fois par de l'halogène, de l'hydroxyle ou de l'alkyle $C_1$-$C_6$ et dont la fraction acyle possède 2 à 4 atomes de carbone, un reste 2-méthyl 3-phénylpropionyle, 3-phényl-propionyle, cinnamoyle, phénacétyle ou benzoyle, où les restes phényle peuvent être substitués par de l'halogène, de l'hydroxyle ou de l'alkyle $C_1$-$C_6$, un reste méthanesulfonyle, benzènesulfonyle ou phénacylsulfonyle, où le reste phényle peut être substitué par de l'halogène ou de l'alkyle $C_1$-$C_6$, ou un reste 4-chlorophényle, 3-trifluorométhylphényle, 4-(4-chlorobenzoyl) phényle, 4-biphénylyle, 4-phénoxyphényle ou 4-benzyloxyphényle,

avec la restriction que si A est une liaison de valence, R ne peut pas être de l'hydrogène, du méthyle, de l'éthyle, de l'hydroxyéthyle ou du benzyle, ainsi que leurs sels physiologiquement insoupçonnables, leurs esters avec le méthanol, l'éthanol, le n-butanol, l'alcool 4-chlorobenzylique ou le 2-diéthylaminoéthanol et leurs amides avec l'ammoniac, la diéthylamine, l'éthanolamine, l'acide p-aminobenzoïque, la β-alanine ou la morpholine.

2. Composé selon la formule I de la revendication 1 dans laquelle B est une liaison de valence et A et R ont les significations indiquées.

3. Composés selon la formule I de la revendication 1 dans laquelle B est le groupe $CH_2$ et A est une chaîne alkylène inférieur et R a les significations indiquées.

4. Composés selon la formule I de la revendication 1 dans laquelle A est le groupe $CH_2$—$CH_2$ et B est une liaison de valence et R a les significations indiquées.

5. Procédé pour la préparation d'acides carboniques de formule générale I

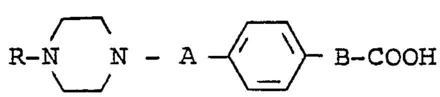

(I)

dans laquelle

A est une liaison de valence ou une chaîne alkylène droite ou ramifiée ayant de 1 à 3 atomes de carbone,

B est une liaison de valence ou une chaîne alkylène saturée ou insaturée, droite ou ramifiée ayant de 1 à 3 atomes de carbone et,

R est un atome d'hydrogène, une chaîne alkyle $C_1$-$C_6$ droite ou ramifiée, substituée une ou plusieurs fois par de l'hydroxyle, du carboxyle, un groupe acide sulfonique ou par un groupe phénoxy éventuellement substitué une ou plusieurs fois par de l'alkyle $C_1$-$C_6$, de l'alcoxy $C_1$-$C_6$, de l'halogène, du nitro, du cyano, du carboxyle ou de l'acylamino $C_1$-$C_6$, une chaîne phénylalkyle dans laquelle le groupe phényle peut porter éventuellement un ou plusieurs atomes d'halogène, de l'hydroxyle, de l'alcoxy $C_1$-$C_6$, du cyano, du carboxyle, du nitro, de l'acylamino $C_1$-$C_6$, de l'alkyle $C_1$-$C_6$ ou des groupes trifluorométhyle, et la chaîne alkyle est éventuellement insaturée et possède jusqu'à 4 atomes de carbone, un groupe phénacyle, dont le reste phényle peut être substitué une ou plusieurs fois par de l'halogène, de l'hydroxyle ou de l'alkyle $C_1$-$C_6$ et dont la fraction acyle possède 2 à 4 atomes de carbone, un reste 2-méthyl 3-phénylpropionyle, 3-phényl-propionyle, cinnamoyle, phénacétyle ou benzoyle, où les restes phényle peuvent être substitués par de l'halogène, de l'hydroxyle ou de l'alkyle $C_1$-$C_6$, un reste méthanesulfonyle, benzènesulfonyle ou phénacylsulfonyle, où le reste phényle peut être substitué par de l'halogène ou de l'alkyle $C_1$-$C_6$, ou un reste 4-chlorophényle, 3-trifluorométhylphényle, 4-(4-chlorobenzoyl) phényle, 4-biphénylyle, 4-phénoxyphényle ou 4-benzyloxyphényle,

avec la restriction que si A est une liaison de valence, R ne peut pas être de l'hydrogène, du méthyle, de l'éthyle, de l'hydroxyéthyle ou du benzyle, ainsi que leurs sels physiologiquement insoupçonnables, leurs

esters avec le méthanol, l'éthanol, le n-butanol, l'alcool 4-chlorobenzylique ou le 2-diéthylaminoéthanol et leurs amides avec l'ammoniac, la diéthylamine, l'éthanolamine, l'acide p-aminobenzoïque, la β-alanine ou la morpholine caractérisé en ce que de façon en soi connue comme on fait réagir :

a) un composé de formule II

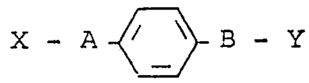

(II)

soit : avec une pipérazine dont l'atome $N_1$ est protégé et après l'élimination du groupe de protection on introduit le substituant R par alkylation ou acylation, soit : avec une pipérazine portant déjà comme substituant unique un groupe R, ou bien

b) un composé de formule III

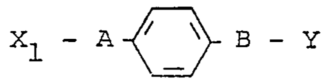

(III)

avec un composé de formule IV

$$R—X_2 \quad \text{(IV)}$$

un des restes $X_1$ ou $X_2$ étant un groupe $NH_2$ et l'autre un groupe

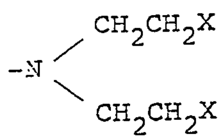

dans ces procédés

A et R ayant la signification indiquée,

X étant un reste réactif

B étant une liaison de valence, une chaîne alkylène inférieur saturée ou insaturée ou un reste pouvant être transformé en ce groupe et

Y étant un groupe amide d'acide ou $—COOR_2$ dans lequel $R_2$ est de l'hydrogène ou un groupe alkyle inférieur ou un reste pouvant être transformé en ces groupes,

et en ce qu'ensuite on transforme éventuellement les composés obtenus en les acides carboxyliques libres, leurs sels, esters ou amides, on transforme éventuellement des groupes B en chaînes alkyle inférieur saturées ou insaturées, ou on échange le reste R contre un autre reste R.

6. Médicaments, comportant un composé selon la revendication 1 et substances de support et auxiliaires habituelles.

7. Composés selon la revendication 1 pour l'utilisation comme médicaments pour le traitement des taux élevés de lipides dans le sang.